# FASCICULE DE BREVET EUROPEEN

(11) **EP 2 235 057 B1**
(45) Date de publication et mention de la délivrance du brevet: **30.08.2017**
(21) Numéro de dépôt: 08856851.4
(22) Date de dépôt: 28.11.2008
(51) Int. Cl.: A61K 39/00, C07K 16/12, C12N 15/13, A61K 39/395, A61P 31/04, A61K 47/42, G01N 33/569, C12N 15/63

(54) **IMMUNOGLOBULINE G CONTRE LES TOXINES DU CHARBON**
GEGEN ANTHRAXTOXINE EINGESETZTES G-IMMUNOGLOBULIN
G IMMUNOGLOBULIN USED AGAINST ANTHRAX TOXINS

(30) Priorité: 29.11.2007 FR 0759419
(43) Date de publication de la demande: 06.10.2010
(73) Titulaire: Laboratoire Français du Fractionnement et des Biotechnologies, 91940 Les Ulis (FR); ETAT FRANCAIS Représenté par le Délégué Général pour l'Armement, 00457 Armées (FR)
(72) Inventeur: THULLIER, Philippe, F-38190 Bernin (FR); BEHRENS, Christian, F-91120 Palaiseau (FR)
(74) Mandataire: Jacobacci Coralis Harle
(86) Numéro de dépôt international: PCT/FR2008/052160
(87) Numéro de publication internationale: WO 2009/071860

(56) Documents cités:
- WO-A-2007/084107
- WO-A-2009/050388
- US-A1- 2006 121 045
- LAFFLY EMMANUELLE ET AL: "Selection of a macaque Fab with framework regions like those in humans, high affinity, and ability to neutralize the protective antigen (PA) of Bacillus anthracis by binding to the segment of PA between residues 686 and 694" ANTIMICROBIAL AGENTS AND CHEMOTHERAPY, AMERICAN SOCIETY FOR MICROBIOLOGY, WASHINGTON, DC, US, vol. 49, no. 8, août 2005 (2005-08), pages 3414-3420, XP002444247 ISSN: 0066-4804 cité dans la demande
- WARK ET AL: "Latest technologies for the enhancement of antibody affinity" ADVANCED DRUG DELIVERY REVIEWS, AMSTERDAM, NL, vol. 58, no. 5-6, 7 août 2006 (2006-08-07), pages 657-670, XP005611420 ISSN: 0169-409X cité dans la demande
- PRESTA ET AL: "Engineering of therapeutic antibodies to minimize immunogenicity and optimize function" ADVANCED DRUG DELIVERY REVIEWS, AMSTERDAM, NL, vol. 58, no. 5-6, 7 août 2006 (2006-08-07), pages 640-656, XP005611419 ISSN: 0169-409X
- LAFFLY E ET AL: "Improvement of an Antibody Neutralizing the Anthrax Toxin by Simultaneous Mutagenesis of Its Six Hypervariable Loops" JOURNAL OF MOLECULAR BIOLOGY, LONDON, GB, vol. 378, no. 5, 16 mai 2008 (2008-05-16), pages 1094-1103, XP022627585 ISSN: 0022-2836 [extrait le 2008-03-28]

## Description

### DOMAINE DE L'INVENTION

La présente invention se rapporte à une immunoglobuline G primatisée dirigée contre la sous-unité PA (antigène protecteur) de la bactérie *Bacillus anthracis.*

### ART ANTERIEUR

Le charbon est une maladie infectieuse causée par une bactérie à gram positif, *Bacillus anthracis.* Cette bactérie est immobile, et forme des spores, hautement résistantes, qui germinent en une forme végétative lorsqu'elles se trouvent dans des environnements tels que le sang ou les tissus des hommes ou des animaux. Malgré leur haute résistance, les spores ne se reproduisent pas, en revanche elles peuvent survivre des dizaines d'années dans le sol.

Une infection par le charbon peut prendre trois formes : cutanée, pulmonaire ou digestive. L'infection pulmonaire est la plus fréquemment mortelle. En cas d'inhalation, les spores de *B. anthracis* passent dans les alvéoles où elles sont phagocytées par les macrophages et les cellules dendritiques, notamment. Les spores germent dans ces cellules et les formes végétatives se multiplient dans les ganglions. Les bactéries passent alors dans le sang, se reproduisent en continu et produisent des toxines, responsables en partie de la létalité de la maladie. Les toxines du charbon sont composées de trois protéines distinctes : l'antigène protecteur (PA, 83 kDa avant clivage enzymatique intracellulaire et 63 kDa après clivage), le facteur létal (LF, 90 kDa) et le facteur oedémateux (EF, 89 kDa). La toxine létale est formée de PA et LF ; et la toxine oedémateuse, dont le rôle dans la physiologie de la maladie est moindre, de PA et EF.

Ces protéines sont sécrétées par la bactérie en tant que monomères non toxiques, et s'assemblent à la surface des cellules cibles pour former des complexes toxiques.

Jusqu'à présent, plusieurs antibiotiques, tels que la pénicilline, la doxycycline et les fluoroquinones (par exemple la ciprofloxacine), ont été utilisés pour le traitement des infections par le charbon.

Cependant, certains de ces antibiotiques peuvent ne pas avoir d'effets sur certaines souches, résistantes aux antibiotiques. En particulier, certains de ces traitements pourraient ne pas être utilisables en cas de terrorisme ou guerre bactériologique, lorsque des souches résistantes aux antibiotiques pourraient être volontairement disséminées.

De plus, comme les antibiotiques ne peuvent pas inhiber l'action de la toxine du charbon, il est nécessaire que ces antibiotiques soient administrés à des stades précoces de l'infection, or les diagnostics précoces sont difficiles à établir car les symptômes initiaux sont non spécifiques.

Des vaccins, dont le composant majeur est l'antigène protecteur PA, ont été développés mais ne sont utilisés que pour des personnes très fortement susceptibles d'être en contact avec *B. anthracis.* De plus, du fait de la nécessité d'une période de plusieurs mois pour acquérir une immunité suffisante, ces vaccins ne peuvent pas être utilisés dans des situations d'urgence. En France actuellement, aucun de ces vaccins n'est agréé pour l'utilisation humaine. Il est donc nécessaire de développer de nouvelles approches thérapeutiques et préventives, autres que les antibiotiques.

L'immunisation passive avec des anticorps représente une stratégie efficace pour neutraliser la toxine. Plusieurs essais ont été réalisés pour neutraliser la toxine létale du charbon à l'aide d'anticorps monoclonaux contre l'antigène protecteur (PA) et le facteur létal (LF). La neutralisation de la toxine létale du charbon à l'aide d'un anticorps peut avoir lieu par inhibition de la liaison de PA et de son récepteur cellulaire, inhibition du clivage de PA, inhibition de la liaison entre PA et LF ou encore inhibition de l'action de LF par exemple.

Le développement de nouveaux anticorps permettant de neutraliser la toxine du charbon est ainsi d'un intérêt général pour une prévention et un traitement efficace du charbon.

Dans un travail récent, un macaque a été immunisé avec l'antigène protecteur PA83 pour obtenir des anticorps destinés à traiter l'infection humaine par le charbon. A partir de la moelle osseuse, les inventeurs ont amplifié les gènes codant des fragments d'anticorps spécifiques de PA83 et les ont clonés pour obtenir une librairie.

Un fragment de forte affinité (Kd=3,4 nM) et fortement neutralisant (concentration d'inhibition à 50% = 5,6 +/- 0,13 nM), désigné sous le nom de 35PA83, a ensuite été isolé (Laffly et al., antimicrobial agents and chemotherapy, 2005, 49(8) : 3414-3420). Laffly et al, 2008 (J Mol Biol, 378, 1094-1103) décrit une méthode permettant d'isoler des anticorps dirigés contre l'antigène protecteur de la toxine du charbon avec une affinité élevée. Ce document décrit plusieurs anticorps, entre autres l'anticorps 6.20 avec une affnité de 0.18 x 10⁻⁹ M. Les auteurs indiquent que les affinités élevées sont exceptionnelles. Le fragment d'immunoglobuline 35PA83 neutralise la toxine du charbon en empêchant l'interaction de PA avec son récepteur cellulaire.

Toutefois, en vue de l'utilisation de ce fragment d'immunoglobuline pour une utilisation médicale (prophylactique ou thérapeutique), une amélioration de son affinité pourrait présenter l'avantage de réduire la quantité à administrer au patient et de réduire le coût du traitement. Par ailleurs, du fait de l'origine simiesque de ce fragment d'immunoglobuline, il pourrait présenter un risque d'immunogénicité et d'altération de sa biodisponibilité chez l'homme.

### RESUME DE L'INVENTION

La présente invention a donc pour objet de fournir une immunoglobuline entière, d'isotype IgG1 ou IgG2, dirigé contre l'antigène PA, obtenue à partir du fragment d'immunoglobuline 35PA83, préalablement modifié en vue d'améliorer son affinité pour l'antigène et de présenter une plus grande ressemblance avec les anticorps humaines

La présente invention a ainsi pour objet une immunoglobuline de classe G (IgG) dirigée contre l'antigène protecteur (PA) de la toxine du charbon, comprenant les régions variables de l'anticorps 35PA83 dont certains résidus sont mutés, et des régions constantes d'origine humaine.

En particulier, l'invention a pour objet une immunogtobutine de classe G (IgG) dirigée contre l'antigène protecteur (PA) de la toxine du charbon, comprenant :
- une région variable de chaîne légère ayant :
   - une séquence d'acides aminés représentée par la séquence SEQ ID N° 1, ou
   - une séquence d'acides aminés représentée par la séquence SEQ ID N° 1 et comprenant en outre au moins une mutation sélectionnée parmi :
      - aucun/A (1) dans laquelle un acide aminé A a été ajouté en position -4 de la séquence SEQ ID N° 1 , soit immédiatement en amont du résidu N°-3 s'il existe, et à défaut immédiatement en amont du résidu N°-2, et à défaut immédiatement en amont du résidu N°-1, et à défaut immédiatement en amont du résidu N°1
      - aucun/l (2) dans laquelle un acide aminé I a été ajouté en position -3 de la séquence SEQ IDN° 1, soit immédiatement en amont du résidu N°-2 s'il existe, et à défaut immédiatement en amont du résidu N°- , et à défaut immédiatement en amont du résidu N°1
      - aucun/Q (3) dans laquelle un acide aminé Q a été ajouté en position -2 de la séquence SEQ ID N° 1 , soit immédiatement en amont du résidu N°-1 s'il existe, et à défaut immédiatement en amont du résidu
      - aucun/L (4) dans laquelle un acide aminé L a été ajouté en position -1 de la séquence SEQ ID N° 1 , soit immédiatement en amont du résidu N°1
      - Y/S, dans laquelle l'acide aminé Y en position 10 de la séquence SEQ ID N° 1 est remplacé par l'acide aminé S,
      - K/R, dans laquelle l'acide aminé K en position 14 de la séquence SEQ ID N° 1 est remplacé par l'acide aminé R,
      - H/R, dans laquelle l'acide aminé H en position 20 de la séquence SEQ ID N° 1 est remplacé par l'acide amminé R,
      - Lu, dans laquelle l'acide aminé L en position 100 de la séquence SEQ ID N° 1 est remplacé par l'acide aminé V,,
   et
- une région variable de chaîne lourde ayant :
   - une séquence d'acides aminés représentée par la séquence SEQ ID N° 2, ou
   - une séquence d'acides aminés représentée par la séquence SEQ ID N° 2 et comprenant en outre au moins une mutation sélectionnée parmi les mutations suivantes:
      - aucun/Q (1) dans laquelle un acide aminé Q a été ajouté en position -6 de la séquence SEQ ID N° 2, soit immédiatement en amont du résidu N°-5 s'il existe, et à défaut immédiatement en amont du résidu N°-4, et à défaut immédiatement en amont du résidu N°-3, et à défaut immédiatement en amont du résidu N°-2, et à défaut immédiatement en amont du résidu NO-1, et à défaut immédiatement en amont du résidu N°1
      - aucun/V (2) dans laquelle un acide aminé V a été ajouté en position -5 de la séquence SEQ ID N° 2, soit immédiatement en amont du résidu N°-4 s'il existe, et à défaut immédiatement en amont du résidu N°-3, et à défaut immédiatement en amont du résidu N°-2, et à défaut immédiatement en amont du résidu N°-1, et à défaut immédiatement en amont du résidu N°1
      - aucun/Q (3) dans laquelle un acide aminé Q a été ajouté en position -4 de la séquence SEQ ID N° 2, soit immédiatement en amont du résidu N°-3 s'il existe, et à défaut immédiatement en amont du résidu N°-2, et à défaut immédiatement en amont du résidu N°-1, et à défaut immédiatement en amont du résidu N°1
      - aucun/L (4) dans laquelle un acide aminé L a été ajouté en position -3 de la séquence SEQ ID N° 2, soit immédiatement en amont du résidu N°-2 s'il existe, et à défaut immédiatement en amont du résidu N°-1, et à défaut immédiatement en amont du résidu N°1
      - aucun/Q (5) dans laquelle un acide aminé Q a été ajouté en position -2 de la séquence SEQ ID N° 2, soit immédiatement en amont du résidu N°-1 s'il existe, et à défaut immédiatement en amont du résidu N°1
      - aucun/E (6) dans laquelle un acide aminé E̅ a été ajouté en position -1 de la séquence SEQ ID N° 2, soit immédiatement en amont du résidu N°1
      - L/V, dans laquelle l'acide aminé L en position 5 de la séquence SEQ ID N° 2 est remplacé par l'acide aminé V,
      - A/T, dans laquelle l'acide aminé A en position 17 de la séquence SEQ ID N° 2. est remplacé par l'acide aminé T,
      - A/T, dans laquelle l'acide aminé A en position 113 de la séquence SEQ ID N°2 est remplacé par l'acide aminé T,
      - V/L, dans laquelle l'acide aminé V en position 114 de la séquence SEQ ID N° 2 est remplacé par l'acide aminé L ;
   ladite immunoglobuline étant une IgG1 ou un IgG2.

La présente invention a également pour objet un acide nucléique codant l'IgG de l'invention, ainsi qu'un vecteur comprenant cet acide nucléique, et une cellule hôte comprenant ce vecteur.

La présente invention a aussi pour objet une composition comprenant l'IgG de l'invention ainsi qu'une composition pharmaceutique comprenant l'IgG.

La présente invention a également pour objet l'utilisation de l'IgG de l'invention pour la préparation d'un médicament destiné au traitement ou à la prévention d'une infection par *Bacillus anthracis.*

La présente invention concerne également un kit pour la détection d'une toxine du charbon, une méthode de détection in vitro d'une toxine du charbon, ainsi qu'un immunoconjugué comprenant l'IgG de l'invention.

### DESCRIPTION DE L'INVENTION

La présente invention décrit ainsi une immunoglobuline de classe G (IgG) dirigée contre l'antigène protecteur (PA) de la toxine du charbon, comprenant :
- une région variable de chaîne légère comprenant une séquence d'acides aminés possédant au moins 90 % d'identité en acides aminés avec la séquence SEQ ID N°1, et
- une région variable de chaîne lourde comprenant une séquence d'acides aminés possédant au moins 90% d'identité en acides aminés avec la séquence SEQ ID N°2, et comprenant les résidus d'acides aminés correspondant aux résidus Serine en position 25, Lysine en position 54 et Arginine en position 60,
caractérisée en ce qu'il s'agit d'une IgG1 ou d'une IgG2.

Un premier acide nucléique ayant au moins 90% d'identité avec un second acide nucléique de référence, possédera au moins 90 %, de préférence au moins 91%, 92%, 93%, 94%, 95%, 96%, 97%, 97,5%, 98%, 98,3% 98,6%, 99%, 99,6% d'identité en nucléotides avec ledit second acide nucléique de référence.

Un premier polypeptide ayant au moins 90% d'identité avec un second polypeptide de référence, possédera au moins 90%, de préférence au moins 91%, 92%, 93%, 94%, 95%, 96%, 97%, 97,5%, 98%, 98,3% 98,6%, 99%, 99,6% d'identité en acides aminés avec ledit second polypeptide de référence.

Le « pourcentage d'identité entre deux séquences d'acide nucléique ou entre deux séquences de polypeptide, au sens de la présente invention, est déterminé en comparant les deux séquences alignées de manière optimale, à travers une fenêtre de comparaison.

La partie de la séquence nucléotidique ou d'acides aminés dans la fenêtre de comparaison peut ainsi comprendre des additions ou des délétions (par exemple des « gaps ») par rapport à la séquence de référence (qui ne comprend pas ces additions ou ces délétions) de manière à obtenir un alignement optimal entre les deux séquences.

Le pourcentage d'identité est calculé en déterminant le nombre de positions auxquelles une base nucléique identique, ou un résidu d'acide aminé identique, est observé pour les deux séquences comparées, puis en divisant le nombre de positions auxquelles il y a identité entre les deux bases nucléiques, ou entre les deux résidus d'acides aminés, par le nombre total de positions dans la fenêtre de comparaison, puis en multipliant le résultat par cent afin d'obtenir le pourcentage d'identité en nucléotides des deux séquences entre elles, ou le pourcentage d'identité en acides aminés des deux séquences entre elles.

L'alignement optimal des séquences pour la comparaison peut être réalisé de manière informatique à l'aide d'algorithmes connus.

De manière tout à fait préférée, le pourcentage d'identité de séquence est déterminé à l'aide du logiciel CLUSTAL W (version 1.82) les paramètres étant fixés comme suit : (1) CPU MODE = ClustalW mp ; (2) ALIGNMENT = « full » ; (3) OUTPUT FORMAT = « aln w/numbers » ; (4) OUTPUT ORDER = « aligned » ; (5) COLOR ALIGNMENT = « no » ; (6) KTUP (word size) = « default » ; (7) WINDOW LENGTH = « default » ; (8) SCORE TYPE = « percent » ; (9) TOPDIAG = « default » ; (10) PAIRGAP = « default » ; (11) PHYLOGENETIC TREE/TREE TYPE = « none » ; (12) MATRIX = « default » ; (13) GAP OPEN = « default » ; (14) END GAPS = « default » ; (15) GAP EXTENSION = « default » ; (16) GAP DISTANCES = « default » ; (17) TREE TYPE = « cladogram » et (18) TREE GRAP DISTANCES = « hide ». L'annotation des mutations sur la région variable de la chaîne légère et sur la région variable de la chaîne lourde d'un anticorps selon l'invention, et plus généralement tout complément relatif à la description de certains modes de réalisation de l'anticorps de l'invention, l'homme du métier peut se référer à la description de la demande de brevet français n° FR 07/06744 déposée le 26 septembre 2007 pour un « anticorps contre les toxines du charbon », dont le contenu est intégré en Annexe, à la fin de la présente description, de la page 52 à la page 80.

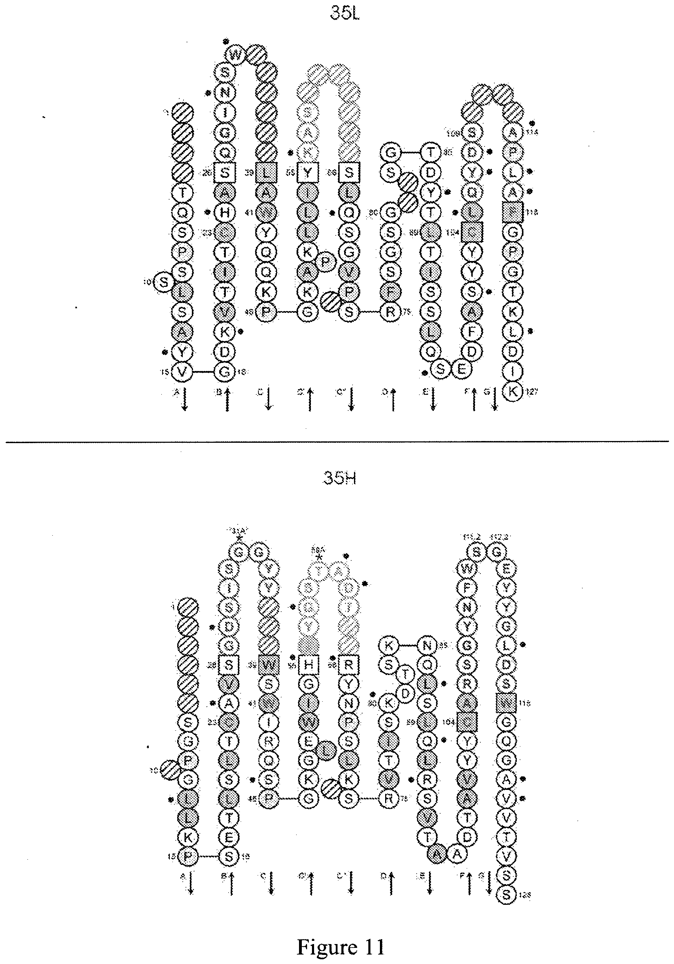

La présence d'un résidu Serine en position 25 de la séquence SEQ ID N° 2 correspond à la mutation désignée « 31A » sur la partie « 35H » de la Figure 11, cette mutation pouvant être désignée également « G/S (31A) ».

La présence d'un résidu Lysine en position 54 de la séquence SEQ ID N° 2 correspond à la mutation « 66 » sur la partie « 35H » de la Figure 11, cette mutation pouvant être désignée également « R/K (66) ».

La présence d'un résidu Arginine en position 60 de la séquence SEQ ID N° 2 correspond à une mutation pouvant être désignée « K/R (73) » et correspond au résidu Arginine en position 73 de la partie « 35H » de la Figure 11.

Selon l'invention, les résidus « correspondant » aux résidus Serine en position 25, Lysine en position 54 et Arginine en position 60 consistent en les résidus ci-dessus, et :
(i) qui sont à la même position dans la séquence ayant au moins 90% d'identité avec la séquence SEQ ID N°2, ou
(ii) qui sont à une position distincte, par exemple du fait que la séquence ayant 90% d'identité avec la séquence SEQ ID N°2 comprend une ou plusieurs délétions ou additions d'un acide(s) aminé(s), par rapport à la séquence SEQ ID N° 2 de référence.

L'invention a aussi pour objet une immunoglobuline de classe G (IgG) telle que définie ci-dessus, caractérisée en ce qu'elle comprend :
- une région variable de chaîne légère ayant une séquence d'acides aminés représentée par la séquence SEQ ID N° 1, et
- une région variable de chaîne lourde ayant une séquence d'acides aminés représentée par la séquence SEQ ID N°2

Le fragment d'immunoglobuline 35PA83, composé d'une chaîne légère et d'un fragment Fd, a été obtenu par immunisation d'un macaque avec l'antigène protecteur PA83 du charbon, comme décrit dans Laffly et al. (2005).

L'affinité K_{D} d'un anticorps peut être mesurée par les techniques conventionnelles connues de l'homme du métier.

L'anticorps non modifié (i.e. non muté) parental 35PA83 présente une affinité K_{D} égale à 3,4 10⁻⁹ M. Cette constante d'affinité a été calculée à partir des constantes d'association et de dissociation mesurées en temps réel par résonance plasmonique de surface tel qu'expliqué dans les exemples.

La région variable de la chaîne légère de l'IgG de l'invention (SEQ ID N°1) est dérivée de la région variable de la chaîne légère du fragment d'immunoglobuline 35PA83, dont la séquence est accessible dans les banques informatisées, comme Genbank, sous les numéros CAH17921 et AJ810487.

Avantageusement, la région variable de la chaîne légère de l'IgG de l'invention, ayant une séquence d'acides aminés représentée par la séquence SEQ ID N°1, comprend en plus au moins une mutation choisie parmi :
- aucun/A (1)
- aucun/I (2)
- aucun/Q (3)
- aucun/L (4)
- Y/S (14)
- K/R (18)
- H/R (24)
- L/V (124).

La mutation aucun/A (1) signifie qu'un acide aminé A a été ajouté en position 1 de la séquence de la partie « 35L » de la figure 11 et en position -4 de la séquence SEQ ID N° 1, soit immédiatement en amont du résidu N°-3 s'il existe, et à défaut immédiatement en amont du résidu N°-2, et à défaut immédiatement en amont du résidu N°-1, et à défaut immédiatement en amont du résidu N°1.

La mutation aucun/l (2) signifie qu'un acide aminé l a été ajouté en position 2 de la séquence de la partie « 35L » de la figure 11 et en position -3 de la séquence SEQ IDN° 1, soit immédiatement en amont du résidu N°-2 s'il existe, et à défaut immédiatement en amont du résidu N°-1, et à défaut immédiatement en amont du résidus N°1.

La mutation aucun/Q (3) signifie qu'un acide aminé a été ajouté en position 3 de la séquence de la partie « 35L » de la figure 11 et en position -2 de la séquence SEQ ID N ° 1, soit immédiatement en amont du résidu N °-1 s'il existe, et à défaut immédiatement en amont du résidus N°1.

La mutation aucun/L (4) signifie qu'un acide aminé a été ajouté en position 4 de la séquence de la partie « 35L » de la figure 11 et en position -1 de la séquence SEQ ID N° 1, soit immédiatement en amont du résidu N °1.

La mutation Y/S (14) signifie que l'acide aminé Y en position 14 de la séquence de la partie « 35L » de la figure 11 et en position 10 de la séquence SEQ ID N° 1 est remplacé par l'acide aminé S.

La mutation K/R (18) signifie que l'acide aminé K en position 18 de la séquence de la partie « 35L » de la figure 11 et en position 14 de la séquence SEQ ID N° 1 est remplacé par l'acide aminé R.

La mutation H/R (24) signifie que l'acide aminé H en position 24 de la séquence de la partie « 35L » de la figure 11 et en position 20 de la séquence SEQ ID N°1 est remplacé par l'acide amminé R.

La mutation L/V (124) signifie que l'acide aminé L en position 124 de la séquence de la partie « 35L » de la figure 11 et en position 100 de la séquence SEQ ID N°1 est remplacé par l'acide aminé V.

De préférence, l'addition d'au moins une de ces mutations permet de réduire l'immunogénicité de la région variable de la chaîne légère de l'IgG de l'invention par rapport à celle du fragment 35PA83 dont elle est dérivée.

De manière particulièrement avantageuse, la région variable de la chaîne légère de l'IgG de l'invention, ayant une séquence d'acides aminés représentée par la séquence SEQ ID N°1, comprend les mutations suivantes :
- aucun/A (1)
- aucun/I (2)
- aucun/Q (3)
- aucun/L (4).

De manière particulièrement avantageuse, la région variable de la chaîne légère de l'IgG de l'invention, ayant une séquence d'acides aminés représentée par la séquence SEQ ID N° 1, comprend les mutations suivantes :
- aucun/A (1)
- aucun/I (2)
- aucun/Q (3)
- aucun/L (4)
- Y/S (14)
- K/R (18)
- H/R (24)
- L/V (124).

De préférence, l'addition de ces résidus permet de réduire l'immunogénicité de la région variable de la chaîne légère de l'IgG de l'invention par rapport à celle du fragment 35PA83 dont elle est dérivée.

La région variable de la chaîne lourde de l'IgG de l'invention (SEQ ID N°2) est dérivée de la région variable du fragment Fd du fragment d'immunoglobuline 35PA83, dont la séquence est accessible dans les banques informatisées, comme Genbank, sous les numéros CAH17920 et AJ810486.

Avantageusement, la région variable de la chaîne lourde ayant une séquence d'acides aminés représentée par la séquence SEQ ID N°2 comprend en plus au moins une mutation sélectionnée parmi :
- aucun/Q (1)
- aucun/V (2)
- aucun/Q (3)
- aucun/L (4)
- aucun/Q (5)
- aucun/E (6)
- L/V (12)
- A/T (24)
- A/T (122)
- V/L (123).

La mutation aucun/Q (1) signifie qu'un acide aminé Q a été ajouté en position 1 de la séquence de la partie « 35H » de la figure 11 et en position -6 de la séquence SEQ ID N° 2, soit immédiatement en amont du résidu N°-5 s'il existe, et à défaut immédiatement en amont du résidu N°-4, et à défaut immédiatement en amont du résidu N°-3, et à défaut immédiatement en amont du résidu N°-2, et à défaut immédiatement en amont du résidu N°-1, et à défaut immédiatement en amont du résidus N°1.

La mutation aucun/V (2) signifie qu'un acide aminé V a été ajouté en position 2 de la séquence de la partie « 35H » de la figure 11 et en position -5 de la séquence SEQ ID N° 2, soit immédiatement en amont du résidu N°-4 s'il existe, et à défaut immédiatement en amont du résidu N°-3, et à défaut immédiatement en amont du résidu N°-2, et à défaut immédiatement en amont du résidu N°-1, et à défaut immédiatement en amont du résidus N°1.

La mutation aucun/Q (3) signifie qu'un acide aminé Q a été ajouté en position 3 de la séquence de la partie « 35H » de la figure 11 et en position **-4** de la séquence SEQ ID N°2, soit immédiatement en amont du résidu N°-3 s'il existe, et à défaut immédiatement en amont du résidu N°-2, et à défaut immédiatement en amont du résidu N°-1, et à défaut immédiatement en amont du résidus N°1.

La mutation aucun/L (4) signifie qu'un acide aminé L a été ajouté en position 4 de la séquence de la partie « 35H » de la figure 11 est en position -3 de la séquence SEQ ID N° 2, soit immédiatement en amont du résidu N°-2 s'il existe, et à défaut immédiatement en amont du résidu N°-1, et à défaut immédiatement en amont du résidus N°1.

La mutation aucun/Q (5) signifie qu'un acide aminé Q a été ajouté en position 5 de la séquence de la partie « 35H » de la figure 11 et en position -2 de la séquence SEQ ID N° 2, soit immédiatement en amont du résidu N°-1 s'il existe, et à défaut immédiatement en amont du résidus N°1.

La mutation aucun/E (6) signifie qu'un acide aminé E a été ajouté en position 6 de la séquence de la partie « 35H » de la figure 11 et en position -1 de la séquence SEQ ID N° 2, soit immédiatement en amont du résidu N°1.

La mutation L/V (12) signifie que l'acide aminé L en position 12 de la séquence de la partie « 35H » de la figure 11 et en position 5 de la séquence SEQ ID N° 2 est remplacé par l'acide aminé V.

La mutation A/T (24) signifie que l'acide aminé A en position 14 de la séquence de la partie « 35H » de la figure 11 et en position 17 de la séquence SEQ ID N° 2 est remplacé par l'acide aminé T.

La mutation A/T (122) signifie que l'acide aminé A en position 122 de la séquence de la partie « 35H » de la figure 11 et en position 113 de la séquence SEQ ID N° 2 est remplacé par l'acide aminé T.

La mutation V/L (123) signifie que l'acide aminé V en position 123 de la séquence de la partie « 35H » de la figure 11 et en position 114 de la séquence SEQ ID N° 2 est remplacé par l'acide aminé L.

De préférence, l'addition d'au moins une de ces mutations permet de réduire l'immunogénicité de la région variable de la chaîne lourde de l'IgG de l'invention par rapport à celle du fragment 35PA83 dont elle est dérivée.

La région variable de la chaîne lourde de l'IgG de l'invention (SEQ ID N°2) est dérivée de la région variable du fragment Fd du fragment d'immunoglobuline 35PA83, dont la séquence est accessible dans les banques informatisées, comme Genbank, sous les numéros CAH17920 et AJ810486. La région variable de la chaîne lourde de l'invention (SEQ ID NO :2) est modifiée, par rapport à la région variable du fragment d'immunoglobuline 35PA83, en ce qu'elle comprend les trois mutations suivantes : G/S (31A), R/K (66) et K/R (73). De manière avantageuse, ces trois mutations permettent d'améliorer l'affinité de la région variable de la chaîne lourde de l'IgG de l'invention par rapport à la région variable du fragment d'immunoglobuline 35PA83.

De manière particulièrement avantageuse, la région variable de chaîne lourde ayant une séquence d'acides aminés représentée par la séquence SEQ ID N°2 comporte en plus les mutations suivantes :
- aucun/Q (1)
- aucun/V (2)
- aucun/Q (3)
- aucun/L (4)
- aucun/Q (5)
- aucun/E (6)

De manière particulièrement avantageuse, la région variable de chaîne lourde ayant une séquence d'acides aminés représentée par la séquence SEQ ID N°2 comporte en plus les mutations suivantes :
- aucun/Q (1)
- aucun/V (2)
- aucun/Q (3)
- aucun/L (4)
- aucun/Q (5)
- aucun/E (6)
- L/V (12)
- A/T (24)
- A/T (122)
- V/L (123).

De préférence, l'addition d'au moins une de ces mutations permet de réduire l'immunogénicité de la région variable de la chaîne légère de l'IgG de l'invention par rapport à celle du fragment 35PA83 dont elle est dérivée.

Dans un mode de réalisation particulier de l'invention, la région variable de la chaîne légère de l'anticorps (SEQ ID NO : 1) comprend les mutations suivantes :
- aucun/A (1)
- aucun/I (2)
- aucun/Q (3)
- aucun/L (4),
et la région variable de la chaîne lourde de l'anticorps (SEQ ID NO : 2) comprend les mutations suivantes :
- aucun/Q (1)
- aucun/V (2)
- aucun/Q (3)
- aucun/L (4)
- aucun/Q (5)
- aucun/E (6).

De manière avantageuse, la région constante de la chaîne légère de l'IgG de l'invention possède une séquence d'acides aminés comprenant la séquence SEQ ID N°3, et la région constante de la chaîne lourde possède une séquence d'acides aminés comprenant la séquence SEQ ID N°4.

Aux fins de la description de la présence invention, le terme « immunoglobuline » se réfère à une molécule d'immunoglobuline ou un fragment d'une molécule d'immunoglobuline ayant la capacité de se lier spécifiquement à un antigène particulier. Des fragments d'immunoglobuline bien connus sont par exemple les fragments F(ab')2, Fab, Fv, scFv et Fd.

Les immunoglobulines de type G (IgG), sont des hétérodimères constitués de 2 chaînes lourdes et de 2 chaînes légères, liées entre elles par des ponts disulfures. Chaque chaîne est constituée, en position N-terminale, d'une région ou domaine variable (codée par les gènes réarrangés V-J pour la chaîne légère et V-D-J pour la chaîne lourde) spécifique de l'antigène contre lequel l'immunoglobuline est dirigée, et en position C-terminale, d'une région constante, constituée d'un seul domaine CL pour la chaîne légère ou de 3 domaines (CH1, CH2 et CH3) pour la chaîne lourde. L'association des domaines variables et des domaines CH₁ et CL des chaînes lourdes et légères forme les parties Fab, qui sont connectées à la région Fc par une région charnière très flexible permettant à chaque Fab de se fixer à sa cible antigénique tandis que la région Fc, médiatrice des propriétés effectrices de l'anticorps, reste accessible aux effecteurs immunitaires, phagocytes ou cellules tueuses, et le complément ; ces régions constantes ne sont pas impliquées dans la liaison à l'antigène. La région Fc, constituée des 2 domaines globulaires CH₂ et CH₃, est glycosylée au niveau du domaine CH₂ avec la présence, sur chacune des 2 chaînes, d'un *N*-glycanne biantenné de type lactosaminique, lié à l'Asn 297.

La région variable est quant à elle impliquée dans la liaison de l'anticorps à son épitope.

Un anticorps dont la région constante (Fc) a été enzymatiquement clivée de façon à en préserver la région charnière est désigné comme un fragment F(ab')2 et conserve les deux sites de liaison à l'antigène.

De même, un anticorps dont la région constante, y compris la région charnière a été enzymatiquement clivée, ou qui a été produit sans cette région, est désigné comme un fragment Fab et conserve un des deux sites de liaison à l'antigène.

Le fragment Fd est formé des régions VH et CH1.

Dans la région variable, on trouve les régions déterminant la complémentarité (CDRs, complementary determining régions), aussi appelées régions hypervariables, qui interagissent directement avec l'antigène. Modifier les CDRs peut donc permettre de modifier l'affinité d'un anticorps. Dans la région variable, on trouve un second type de régions, appelées régions charpentes (FRs, frameworks), qui maintiennent la structure tertiaire des CDRs. Ces régions charpentes sont assez spécifiques de l'espèce où a été produit l'anticorps. Dans le fragment Fd de la chaîne lourde et dans la chaîne légère, on trouve quatre régions charpentes (FR1 à 4) séparées respectivement par trois CDR (CDR1 à 3).

D'après la description ci-dessus des acides aminés de la région variable de la chaîne lourde, et de la région variable de la chaîne légère de l'IgG anti-PA de l'invention, l'homme du métier est capable de synthétiser ou de faire synthétiser, des acides nucléiques qui codent ces séquences d'acides aminés.

Avantageusement, les régions constantes de chacune des chaînes légères et des chaînes lourdes de l'IgG de l'invention sont des régions constantes humaines.

De manière préférée, les régions constantes de chacune de chaînes légères de l'IgG de l'invention est de type K. Tout allotype convient à la réalisation de l'invention, par exemple Km(1), Km(1,2), Km(1, 2, 3) ou Km(3), mais l'allotype préféré est Km(3).

La région constante de chacune des chaînes lourdes de l'anticorps peut être de type γ1, de type γ2, de type γ3, ces trois types de régions constantes présentant la particularité de fixer le complément humain, ou encore de type γ4.

De manière préférée, la région constante de chacune des chaînes lourdes de l'anticorps est de type γ1, car un tel anticorps montre une capacité à engendrer une activité ADCC chez le plus grand nombre d'individus (humains). A cet égard, tout allotype convient à la réalisation de l'invention, par exemple G1 m(3), G1 m (1, 2, 17), G1 m(1, 17) ou G1 m(1,3). De manière préférentielle, l'allotype est G1 m(1,17).

De manière avantageuse, la région constante de chacune des chaînes lourdes de l'IgG de l'invention est de type y1 et comprend la séquence en acides aminés SEQ ID N°4 et la région constante de chacune des chaînes légères de l'IgG de l'invention comprend la séquence en acides aminés SEQ ID N°3.

De manière avantageuse, chacune des chaînes légères de l'IgG de l'invention comprend la séquence en acides aminés SEQ ID N°5, et chacune des chaînes lourdes de l'IgG de l'invention comprend la séquences en acides aminés SEQ ID N°6.

Un autre objet de l'invention est un acide nucléique codant l'IgG de l'invention.

La région variable de chacune des chaînes légères de l'IgG de l'invention est codée par la séquence d'acide nucléique SEQ ID NO : 7, et la région variable de chacune des chaînes lourdes de l'anticorps selon l'invention est codée par la séquence d'acide nucléique murine SEQ ID NO : 8.

Dans un aspect particulier de l'invention, la région constante de chacune des chaînes lourdes de l'IgG de l'invention est codée par la séquence d'acide nucléique humaine SEQ ID NO : 9, et la région constante de chacune de ses chaînes légères étant codée par la séquence d'acide nucléique humaine SEQ ID NO : 10.

Plus particulièrement, chacune des chaînes légères de l'anticorps selon l'invention est codée par la séquence d'acide nucléique SEQ ID NO : 11, et chacune des chaînes lourdes est codée par la séquence d'acide nucléique SEQ ID NO : 12.

Un autre objet de l'invention est un vecteur comprenant un acide nucléique codant l'IgG de l'invention.

Le terme « vecteur » se réfère à un acide nucléique dans lequel la séquence d'intérêt peut être insérée par restriction puis ligation pour le transport entre différents environnements génétiques ou pour l'expression dans une cellule hôte. Les vecteurs sont par exemples les plasmides, les cosmides, les chromosomes artificiels de levures (YAC), les chromosomes artificiels de bactéries (BAC) et les chromosomes artificiels dérivés du bactériophage P1 (PAC), les vecteurs dérivés de virus. Un vecteur de clonage est un vecteur capable de se répliquer dans une cellule hôte et qui est de plus caractérisé par la présence de un ou plusieurs sites de restrictions par les endonucléases. Un vecteur d'expression est un vecteur dans lequel la séquence d'ADN d'intérêt peut être insérée par restriction ou ligation de telle façon qu'elle puisse être répliquée et/ou transcrite en ARN. Les vecteurs peuvent contenir en outre un ou plusieurs marqueurs de sélection ou d'identification des cellules ayant été transformées ou transfectées avec le vecteur.

Ces acides nucléiques pourront être compris dans un vecteur recombinant pour le clonage ou pour l'expression des anticorps de l'invention.

La présente invention inclut tous les vecteurs recombinants contenant des séquences codantes pour la transformation, transfection ou thérapie génique eucaryote ou procaryote. De tels vecteurs pourront être préparés selon les techniques conventionnelles de biologie moléculaire et comprendront en outre un promoteur approprié, optionnellement une séquence signal pour l'export ou la sécrétion, et des séquences régulatrices nécessaires pour la transcription de la séquence nucléotidique.

Un polypeptide de fusion peut être utile pour la purification des anticorps de la présente invention. Le domaine de fusion peut par exemple inclure une queue poly-histidine qui permet la purification sur des colonnes Ni²⁺, ou une ancre membranaire de phage filamenteux qui est particulièrement utile pour le screening de banque, selon la technologie du « phage display ».

Un des vecteurs approprié dans le cadre de l'invention est une molécule d'ADN recombinante adaptée pour recevoir et exprimer une première et une seconde séquence d'ADN, de façon à permettre l'expression d'anticorps hétérodimérique tel qu'un anticorps de longueur complète ou des fragments F(ab')2 ou Fab selon l'invention. Un tel vecteur fournit un système pour indépendamment cloner les deux séquences d'ADN dans deux cassettes séparées présentes dans le vecteur, de façon à former deux cistrons séparés pour l'expression d'un premier et d'un second polypeptide de l'anticorps hétérodimérique. Un tel vecteur d'expression est appelé vecteur dicistronique.

Les anticorps modifiés de la présente invention peuvent être produits dans des cellules eucaryotes telles que des cellules CHO ou des hybridomes humain ou murin par exemple, ainsi que dans des cellules végétales et des animaux transgéniques.

La présente invention a également pour objet des cellules hôtes, procaryotes ou eucaryotes, comprenant un vecteur selon l'invention.

Avantageusement, le vecteur d'expression de l'invention permet l'expression de la chaîne légère de l'IgG de l'invention. Dans ce mode de réalisation particulier, le vecteur est une molécule d'acide nucléique dans laquelle la séquence d'acide nucléique SEQ ID NO : 7 codant pour la région variable de chacune des chaînes légères de l'IgG, et la séquence d'acide nucléique SEQ ID NO :10 codant pour la région constante de chacune des chaînes légères de l'anticorps ont été insérées, afin de les introduire et de les maintenir dans une cellule hôte. Il permet l'expression de ces fragments d'acide nucléique étrangers dans la cellule hôte car il possède des séquences indispensables (promoteur, séquence de polyadénylation, gène de sélection) à cette expression. De tels vecteurs sont bien connus de l'homme du métier, et peuvent être un adénovirus, un rétrovirus, un plasmide ou un bactériophage, cette liste n'étant pas limitative. De plus, toute cellule de mammifère peut être utilisée comme cellule hôte, c'est-à-dire comme cellule exprimant les fragments d'acides nucléique portés par le vecteur d'expression de la chaîne légère de l'IgG de l'invention, par exemple YB2/0, CHO, CHO dhfr- (par exemple CHO DX B11, CHO DG44), CHO Lec13, SP2/0, NS0, 293, BHK ou COS.

Avantageusement, le vecteur d'expression de l'invention permet l'expression de la chaîne lourde de l'IgG de l'invention. Dans ce mode de réalisation particulier, le vecteur est une molécule permettant l'expression de l'IgG de l'invention dont la chaîne lourde est codée par la séquence d'acide nucléique SED ID NO : 12. Ce vecteur est une molécule d'acide nucléique dans laquelle la séquence d'acide nucléique SEQ ID NO : 8 codant pour la région variable de chacune des chaînes lourdes de l'IgG, et la séquence d'acide nucléique SEQ ID NO : 9 codant pour la région constante de chacune des chaînes lourdes de l'anticorps ont été insérées, afin de les introduire et de les maintenir dans une cellule hôte. Il permet l'expression de ces fragments d'acide nucléique étrangers dans la cellule hôte car il possède des séquences indispensables (promoteur, séquence de polyadénylation, gène de sélection) à cette expression. Tout comme indiqué précédemment, le vecteur peut être par exemple un plasmide, un adénovirus, un rétrovirus ou un bactériophage, et la cellule hôte peut être toute cellule de mammifère, par exemple YB2/0, CHO, CHO dhfr- (CHO DX B11, CHO DG44), CHO Lec13, SP2/0, NS0, 293, BHK ou COS.

Le vecteur de l'invention peut comporter les séquences codant la chaîne lourde et/ou les chaînes légères de l'immunoglobuline de l'invention.

Un exemple de vecteur permettant l'expression des chaînes lourdes et légères de l'IgG de l'invention est donné à l'exemple 2. Ce vecteur est un vecteur unique contenant les deux unités de transcription pour la chaîne lourde et pour la chaîne légère de l'IgG de l'invention.

Un autre objet de l'invention est une cellule hôte comprenant le vecteur tel que défini ci-dessus.

Avantageusement, la cellule hôte de l'invention est choisie parmi SP2/0, YB2/0, IR983F, le myélome humain Namalwa, PERC6, les lignées CHO, notamment CHO-K-1, CHO-Lec10, CHO-Lec1, CHO-Lec13, CHO Pro-5, CHO dhfr-, Wil-2, Jurkat, Vero, Molt-4, COS-7, 293-HEK, BHK, K6H6, NS0, SP2/0-Ag 14 et P3X63Ag8.653.

Dans un mode de réalisation préféré, l'anticorps est produit dans l'hybridome de rat YB2/0 (cellule YB2/3HL.P2.G11.16Ag.20, déposée à l'American Type Culture Collection sous le numéro ATCC CRL-1662). Cette lignée a été choisie en raison de sa capacité à produire des anticorps présentant une activité ADCC (antibody-dependent cell-mediated cytotoxicity) améliorée par rapport à des anticorps de même structure primaire produits par exemple dans CHO.

Dans un aspect particulier de l'invention, la lignée cellulaire stable exprimant un anticorps selon l'invention, et plus particulièrement choisie dans le groupe précédemment décrit, a intégré le ou les deux vecteurs d'expression de la chaîne lourde et de la chaîne légère tels que précédemment décrits.

Un autre objet de l'invention est une composition comprenant au moins une IgG de l'invention.

Un autre objet de l'invention est une composition pharmaceutique comprenant au moins une IgG de l'invention.

Ladite composition pharmaceutique comprend de façon préférée un véhicule pharmaceutiquement acceptable. Un tel véhicule correspond au sens de l'invention à un matériel non toxique qui n'interfère pas avec l'efficacité de l'activité biologique des ingrédients actifs de la composition. Le terme « pharmaceutiquement acceptable » réfère à un matériel non toxique qui est compatible avec un système biologique tel qu'une cellule, une culture cellulaire, un tissu ou un organisme. Les caractéristiques du véhicule dépendront du mode d'administration.

La présente invention concerne l'utilisation d'au moins une IgG de l'invention pour la préparation d'une composition pharmaceutique ou d'un médicament destiné au traitement ou à la prévention d'une infection par *Bacillus anthracis.*

Le terme « prévention » correspond à la prévention de l'apparition de la maladie chez un sujet, en particulier un humain, chez qui la maladie ne s'est pas encore déclarée.

Le terme « traitement » correspond à l'inhibition de cette maladie, i.e. l'arrêt de son développement, sa régression, ou à la disparition des symptômes et conséquences de la maladie, ou encore à la disparition des causes de la maladie.

L'IgG de l'invention peut être marquée. Des exemples de marqueurs comprennent les enzymes, les radio-isotopes, les composés fluorescents, les métaux colloïdes, les composés chimioluminescents, et les composés bioluminiscents.

Les méthodes de liaison d'un marqueur à un anticorps sont bien connues de l'homme du métier.

Une autre technique de marquage consiste à coupler l'anticorps à des haptènes de faibles poids moléculaire, ces haptènes pouvant être spécifiquement modifiés au moyen d'une seconde réaction. Des exemples d'haptènes sont la biotine, qui réagit avec l'avidine, ou le dinitrophenol, le pyridoxal ou la fluorescéine, qui peuvent réagir avec des anticorps spécifiques anti-haptènes.

Un objet de la présente invention est de fournir un kit pour la détection d'une toxine du charbon comprenant PA. Ce kit comprend :
- un container comprenant au moins une IgG anti-PA de l'invention et qui peut être ou non marquée,
- optionnellement, un container comprenant des solutions tampons
- et optionnellement un container comprenant des moyens de détection de l'IgG marquée, tel qu'une protéine de liaison à la biotine, par exemple l'avidine ou la streptavidine, liée à une molécule rapporteur, telle qu'un marqueur fluorescent ou enzymatique. Ce container peut aussi comprendre des moyens de détection de l'IgG non marquée, soit essentiellement des anticorps ou fragments d'anticorps.

L'IgG de l'invention peut être utilisée *in vitro,* par exemple dans des tests immunologiques dans lesquels ils sont utilisés en phase liquide ou liés à un véhicule de phase solide. Des exemples de véhicules bien connus sont le verre, le polystyrène, le polypropylène, le polyéthylène, le dextran, le nylon, l'amylase, la cellulose naturelle ou modifiée, le polyacrylamide, l'agarose ou la magnétite. Des exemples de tests immunologiques utilisant l'IgG anti-PA de l'invention sont des radioimmunoessais, des marquages histoimmunologiques, des ELISA, des westernblots, des essais d'immuno-précipitation, des essais d'immuno-diffusion, des essais de fixation du complément, des analyses au FACS ou encore des analyses par puces à protéines.

La présente invention a également pour objet de fournir une méthode de détection *in vitro* d'une toxine du charbon, comprenant PA, dans un échantillon biologique, comprenant :
- la mise en contact de l'échantillon avec au moins une IgG anti PA de l'invention, et
- la détection de la liaison dudit anticorps comme indicateur de la présence de ladite toxine du charbon.

L'échantillon biologique peut être liquide : par exemple de la salive, de l'urine, du fluide cérébrospinal, du sérum ou du sang, ou solide ou semi-solide, par exemple des tissus ou des matières fécales ou un tissu solide tel qu'utilisé couramment en diagnostic histologique.

La présente invention a également pour objet de fournir une méthode de détection *in vivo* d'une toxine du charbon, comprenant PA, dans laquelle une IgG selon la présente invention et marquée est administré à un sujet. La quantité d'IgG marquée administrée doit être suffisante pour permettre la détection de la liaison de l'anticorps à la toxine. La quantité d'IgG marquée administrée dépendra des facteurs tels que l'âge et le sexe du sujet, ainsi que du stade de la maladie. La quantité administrée peut varier entre 0.01 mg/kg et 50 mg/kg, préférablement entre 0.1 mg/kg et 20 mg/kg, et plus préférablement entre 0.1 mg/kg et 2 mg/kg.

Pour effectuer le diagnostic *in vivo,* l'IgG anti PA modifié de l'invention doit être liée à un radioisotope directement ou indirectement par l'intermédiaire de groupes fonctionnels. Des groupes fonctionnels couramment utilisés sont par exemple l'acide diéthylène-triamine-pentacétique (DTPA) et l'acide éthylène-diamine-tétraacétique (EDTA). Des exemples d'ions métalliques radioisotopes sont 111In, 97Ru, 67Ga, 68Ga, 15 72As, 89Zr et 201TI.

Les IgG anti PA modifiées de l'invention peuvent aussi être marquées avec un isotope paramagnétique pour le diagnostic par imagerie de résonance magnétique (IRM) ou par résonance de spin électronique (ESR). Des radioisotopes gamma émetteurs de positrons peuvent également être utilisés, tels que 157Gd, 55Mn, 162Dy, 68Ga, 52Cr, et 20 56Fe.

Les IgG de l'invention peuvent également être utilisées *in vitro* ou *in vivo* pour suivre l'évolution du traitement de la maladie, par exemple en déterminant l'augmentation ou la diminution du nombre de cellules ciblées par les toxines du charbon ou les changements dans la concentration de la toxine PA dans un échantillon biologique.

La présente description décrit une méthode de traitement d'un sujet, de préférence un humain, susceptible d'être infecté par *Bacillus anthracis,* dans laquelle une quantité thérapeutiquement efficace d'un anticorps anti PA modifié selon l'invention est administré audit sujet.

Le terme « quantité thérapeutiquement efficace » se réfère à la quantité qui est suffisante pour effectuer le traitement lorsqu'elle est administrée à un sujet qui nécessite un tel traitement. La quantité thérapeutique effective dépend du sujet, du stade de la maladie à traiter et du mode d'administration, et peut être déterminée par des opérations de routine par l'homme du métier.

Le terme « charbon » se réfère à toute maladie causée, directement ou indirectement, par une infection par Bacillus anthracis. Les symptômes initiaux d'une infection par inhalation ressemblent à ceux d'un rhume (fièvre, douleurs musculaires..). Après plusieurs jours, ces symptômes progressent vers des problèmes sévères de détresse respiratoire et de choc septique. L'inhalation du charbon est en générale fatale.

L'infection cutanée par le charbon a lieu lorsque la bactérie entre dans la peau au niveau d'une brèche cutanée préexistante. Cette infection donne lieu dans un premier temps à une papule, qui se développe en deux-trois jours en une vésicule puis en un ulcère de 1-3 centimètres de diamètre présentant une aire nécrotique au centre. L'infection gastrointestinale par le charbon se développe suite à la consommation de viande contaminée et est caractérisée par une inflammation aigue du tractus intestinal.

Une quantité thérapeutiquement efficace correspond à une quantité suffisante pour diminuer les symptômes de la maladie et l'évolution de l'infection. Cette quantité peut varier avec l'âge, le sexe du sujet et le stade de la maladie et sera déterminée par l'homme du métier. Une quantité thérapeutiquement efficace peut varier entre 0.01 mg/kg et 50 mg/kg, préférablement entre 0.1 mg/kg 5 et 20 mg/kg, et plus préférablement entre 0.1 mg/kg et 2 mg/kg, en une ou plusieurs administrations quotidiennes, pendant un ou plusieurs jours.

Le mode d'administration peut être par injection ou par perfusion graduelle. L'injection peut être intraveineuse, intra-péritonéale, intramusculaire, sous-cutanée ou transdermale.

Les préparations pour une administration parentérale peuvent inclure des solutions aqueuses ou non-aqueuses stériles, des suspensions ou des émulsions. Des exemples de solvents non-aqueux sont le propylène glycol, le polyéthylène glycol, des huiles végétales, telle que l'huile d'olive, ou des esters organiques injectables tels que l'éthyl oléate. Des véhicules aqueux comprennent l'eau, des solutions alcool/eau, des émulsions ou des suspensions.

La présente invention a également pour objet un immunoconjugué comprenant une IgG de l'invention lié, de façon directe ou indirecte, à un agent thérapeutique.

De tels agents thérapeutiques comprennent des agents chimiques, des radionucléides, des agents immunothérapeutiques, des cytokines, des chimiokines, des toxines ou des inhibiteurs d'enzyme. Des exemples de toxines sont la chaîne A de la diphtérie, la chaîne A de l'exotoxine, la chaîne A de la ricin, la chaîne A de l'abrine, la chaîne A de la modeccin, l'alpha-sarcin, les protéines Aleurites fordii, les protéines dianthines, les protéines Phytolaca americana, l'inhibiteur momordica charantia, la curcine, la crotine, l'inhibiteur sapaonaria officinalis, la gélonine, la mitogélline, la restrictocine, la phénomycine, l'énomycine et le tricothecenes. Des exemples de radionucléide sont ²¹²Bi, ¹³¹I, ¹³¹In, ⁹⁰Y, et ¹⁸⁶Re.

Avantageusement, l'IgG de l'invention est associée à un traitement prophylactique avec la tétracycline. L'IgG de l'invention permet de raccourcir la prophylaxie basée sur la tétracycline, en arrêtant, de manière sécurisée, après un risque d'exposition, par un traitement rapide (« court traitement ») de tétracycline suivi d'une injection de l'IgG de l'invention.

Avantageusement, l'IgG de l'invention est associée à un traitement curatif avec la ciprofloxacine.

La présente invention sera mieux comprise à l'aide du complément de description qui va suivre, qui se réfère à des exemples d'obtention d'IgG anti-PA.

### DESCRIPTION DES FIGURES :

Dans les exemples qui suivent, donnés à titre illustratif, il sera fait référence aux figures en annexe:
**Figure 1** : schéma de l'amplification de la région VKV2 (région variable de la chaîne légère de l'IgG de l'invention comportant les mutations décrites dans le tableau 1).
**Figure 2** : schéma de l'amplification de la région VHV2 (région variable de la chaîne lourde de l'IgG de l'invention comportant les mutations décrites dans le tableau 2).
**Figure 3** : carte du Vecteur CHK463-23.
**Figure 4** : carte du Vecteur HK558-12.
**Figure 5** : prophylaxie par l'IgG 35PA83 v2 (IgG de l'invention comportant les mutations décrites dans les tableaux 1 et 2). 2mg/kg d'IgG 35PA83 v2 permettent un taux de survie de 60% et 5 mg/kg permettent une survie complète. Aucun nouvel événement n'est observé jusqu'au 30^{ème} jour.
**Figure 6** **:** prophylaxie par la tétracycline. L'administration de la tétracycline a été arrêtée après le 7^{ème} jour et toutes les souris meurent entre le 4^{ème} et le 7^{ème} jour après cet arrêt du traitement.
**Figure 7** **:** Prophylaxie par la doxycycline, complémentée ou non par l'IgG 35PA83. Un traitement à la tétracycline (dose quotidienne de 5 mg/kg) a été initié chez des souris A/J. Douze après, les animaux ont été infectés avec 10 000 LD₅₀ de spores Sterne. Les injections de tétracycline ont été stoppées au Jour 7 du traitement, en présence ou en absence d'un injection de 35PA83 (1 ou 2 mg/kg). Aucun évènement n'a été observé au-delà de la période de temps représentée sur la figure (500^{ème} heure). Les effets hautement significatifs sont repérés sur la figure avec un signe «***».
**Figure 8** **:** traitement par la ciprofloxacine, l'IgG 35PA83 v2 ou les deux. La ciprofloxacine ou l'IgG 35PA83 v2 utilisée seule ne permettent presque aucune survie, alors qu'une combinaison de ces deux molécules permettent un taux de survie de 80%. Aucun nouvel événement n'est observé jusqu'au 30^{ème} jour.

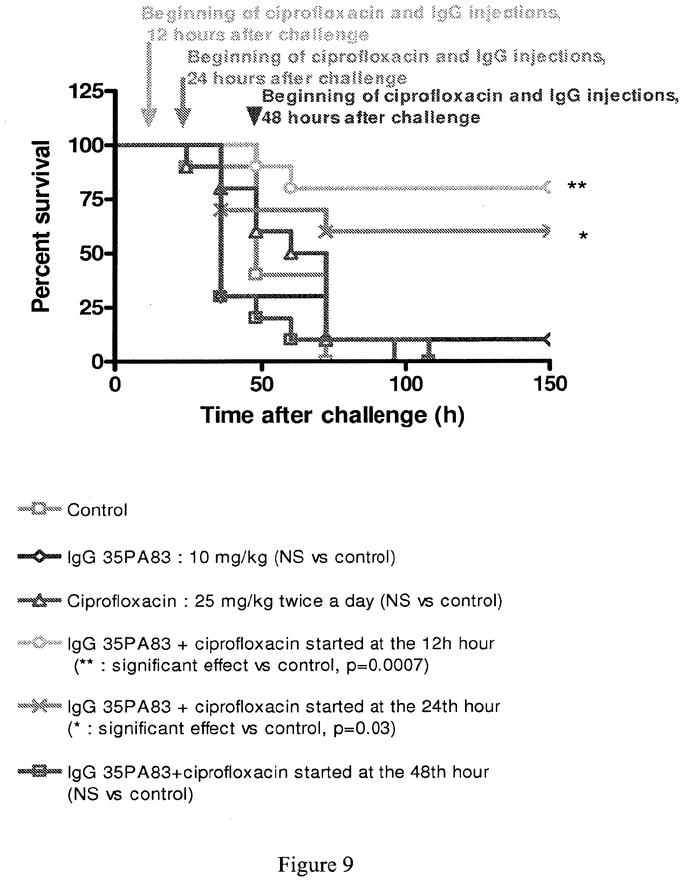

**Figure 9** **:** Traitement avec la ciprofloxacine, complémentée ou non avec l'IgG 35PA83. Des souris A/J ont ét éinfectées avec 1000 LD₅₀ spores Sterne. Douze heures après l'infection, dans deux groupes distincts (10 animaux dans chaque groupe), on a initié un traitement de ciprofloxacine seule (25 mg/kg deux fois par jour) pendant cinq jours, ou une dose unique d'IgG 35PA83 (10 mg/kg) a été injectée. A trois temps distincts suivant l'infection (12, 24 ou 48 heures), chaque temps étant représenté par un groupe différent d'animaux, les souris ont reçu un traitement combiné de ciprofloxacine (25 mg/kg deux fois par jour pendant cinq jours) plus une injection d'IgG 35PA83 (10 mg/kg). Aucun évènement n'a été observé au-delà de la période de temps représentée sur la figure (150^{ème} heure). Les effets significatifs sont repérés sur la figure avec un signe « * » (p=0,03) ou « ** » (p=0,0007).
**Figure 10** **:** Prophylaxie passive par l'IgG 35PA83. Une injection de 35PA83 (2 mg/kg ou 5 mg/kg) a été administrée douze heures avant l'infection (10 000 LD₅₀). Les souris survivant au-delà du 30^{ème} jour ont ét éré-infectées (10 000 LD₅₀) au Jour 30. Aucun évènement n'a été observé au-delà de la période de temps représentée sur la figure (40^{ème} jour). Les effets hautement significatifs sont repérés sur la figure avec un signe « *** » (p=0,0001).
**Figure 11** **:** schéma en collier de perle de la région variable de la chaîne lourde et de la région variable de la chaîne légère de l'anticorps 35PA83 non muté.

La représentation IMGT en collier de perles est réalisée en accord avec la numérotation IMGT. Les points indiquent les différences entre les gènes humains les plus similaires à 35PA83, et 35PA83. Les cercles hachurés correspondent aux positions manquantes selon la numérotation IMGT.

### EXEMPLES

### Exemple 1 : Construction de la bibliothèque mutante de Fab (35PA83)

### Matériels et méthodes

### Souches E. coli

Les souches E. coli suivantes ont été utilisées :
- XL1 (Stratagène, La jolla, CA) : recA1, endA1, gyrA96 thi-1 hsdR17 sup E44 relA1 lac [F'proAB laclqZΔM15 Tn10(Tetr)].
- SURE (Stratagène): e14(McrA) Δ(mcrCB-hsdSMR-mrr)171 endA1 supE44 thi-1 gyrA96 relA1 lac recB recJ sbcC umuC::Tn5 (Kanr) uvrC (F' proAB laclqZΔM15 Tn10 (Tetr)]
- HB2151 (Carte et al., 1985), utilisées pour l'expression de Fabs solubles.

### Toxins

Les toxines du charbon (PA83, LF et EF) ont été achetées chez List laboratories.

### Construction de la bibliothèque de mutants 35PA83

Un variant du fragment d'immunoglobuline 35PA83 a été construit afin de l'humaniser. Ce variant a été obtenu en effectuant les mutations décrites dans les tableaux 3 et 4 :

**Tableau 1 :Mutations pour l'humanisation de la chaîne légère de 35PA83**

| Residue number | 35PA83 | Hu35PA83 |
|---|---|---|
| 1 | Aucun | A |
| 2 | Aucun | I |
| 3 | Aucun | Q |
| 4 | Aucun | L |

**Tableau 2 : Mutations pour l'humanisation du Fd de 35PA83**

| Residue number | 35PA83 | Hu35PA83 |
|---|---|---|
| 1 | Aucun | Q |
| 2 | Aucun | V |
| 3 | Aucun | Q |
| 4 | Aucun | L |
| 5 | Aucun | Q |
| 6 | Aucun | E |

A partir de ce variant humanisé, une bibliothèque d'anticorps mutants dérivés du gène 35PA83 a été générée par Massive mutagenesis® (Biomethodes, Evry, France). Les mutations ont été introduites dans les CDRs des chaînes lourde et légère en utilisant des codons NNS (N code pour A, T, G, ou C et S code pour G ou C). Les régions CDRs ont été définies selon Kabat et al. (Wu and Kabat 1970) et IMGT (Lefranc, Pommie et al. 2003).

La bibliothèque ADN a été utilisée pour transformer les cellules SURE par électroporation. Après ajout de milieu SB supplémenté en carbénicilline à la culture et incubation pendant 1h à 37°C, 1 ml de phage helper VCSM13 (Andris-Widhopf et al., 2001) (environ 1012 pfu) a été ajouté à la culture. Après incubation pendant 2h, 70 µg/ml de kanamycine ont été ajoutés et la culture a été mise sous agitation pendant la nuit à 37°C.

### Sélection d'anticorps par phage

Les particules de phages-Fab ont été purifiées et concentrées à partir de 50 ml de culture par précipitation au PEG, puis re-suspendues dans 3 ml de PBS-BSA 1%-azide 0,02% et filtrées sur un filtre de 0,45 µm. Le titre de cette préparation de phage était d'environ 1010 pfu/ml. Les phages-Fab ont été soumis à trois cycles d'infection-sélection récupération, correspondant à 5, 10 et 15 lavages respectivement, tel que précédemment décrits (Andris-Widhopf, Rader et al. 2000).

### Expression de Fab soluble, extraction périplasmique et purification

Chaque variant ADN a été transformé dans des bactéries de la souche d'E. coli appelée HB2151, rendues chimiquement compétentes. Les cellules ont été cultivées à 30°C, agitées à 250 rpm dans 1L de milieu SB contenant 50 µg/ml de carbénicilline et 0,1% de glucose. Lorsque la culture a atteint une absorbance à λ = 600 nm de 1,5, une induction avec 1 mM d'IPTG (Isopropyl β-D-1-thiogalactopyranoside) a été effectuée pendant 18h à 22°C.

Les Fabs ont été extraits avec du sulfate de polymixine B (Sigma) et purifiés sur colonne de nickel (Ni-NTA spin column, QIAGEN, Valencia, CA) selon les instructions du fabricant, puis dialysés avec du PBS 1X à 4°C pendant 3h.

### Quantification du Fab soluble

La pureté du Fab a été testée par SDS-PAGE et sa concentration a été déterminée à l'aide du logiciel Quantity One® (Biorad).

### Mesure en temps réel de la résonance plasmonique de surface (SPR)

Les constantes de cinétique de l'interaction entre PA83 et les variants 35PA83 ont été déterminées en utilisant le système Biacore X SPR (BIAcore, Uppsala, Sweden). Le PA83 a été immobilisé sur une puce sensible CM5 (Biacore) en utilisant une procédure de couplage des amines par injection de 30 µl de 2 µg/ml de PA83 dans 10 mM de sodium acétate pH 4,5. Pour minimiser la probabilité de reliaison, le K_{D} a été mesuré en utilisant un débit élevé (30 µl/min) et une quantité minimale d'antigène couplé (environ 500 RU, unités de résonance). Le taux de liaison de différentes concentrations de Fab allant de 5 à 400 nM dans du PBS a été déterminé à un débit de 30 µl/min. Les données de liaison ont été introduites dans un modèle langmuir 1 :1 du logiciel d'évaluation BIA (Biacore). Les constantes d'association et de dissociation (kₒₙ et k_{off} respectivement) pour la liaison du Fab à PA83 ont été déterminées à 35°C.

### Analyse de séquences

Les séquences des chaînes lourde et légère des clones sélectionnés ont été déterminées par Genome Express (Meylan, France) en utilisant les amorces ompseq et newpelseq (Andris-Widhopf, Rader et al., 2000). Les séquences ont été analysées en ligne, en utilisant le système IMGT (http:/imgt.cines.fr).

### Résultats

Une maturation d'affinité in vitro a été réalisée pour générer des nouveaux variants présentant une meilleure affinité. Dans ce but, une bibliothèque de variants a été générée par mutation exclusive des résidus de 6 CDRs, c'est-à-dire au niveau de 73 positions. La taille de la bibliothèque est de 5,4.10⁸ transformants. 45 clones plasmidiques indépendants ont été séquencés pour déterminer la diversité et le taux de mutation. Le taux de mutation expérimental est de 3 mutations par fragment (VH+VL), ce qui permet une sélection directe des combinaisons de mutations qui augmentent l'affinité à PA.

L'examen de la fréquence mutationnelle à chaque position CDR ciblée dans la bibliothèque non sélectionnée comparé à la bibliothèque sélectionnée (tableau 1) a montré que certaines positions ne sont pas tolérantes à la variation. Ainsi, les résidus se trouvant à ces positions semblent être des résidus clés pour la liaison à l'antigène, préservant l'intégrité du site de liaison à l'antigène. En particulier, les résidus (H31-H40) du CDR1 sont définis comme des résidus de contact à l'antigène.

Il semble qu'une pression de sélection substantielle ait eu lieu pendant le processus de sélection car la bibliothèque non sélectionnée présente une plus grande diversité en comparaison avec les séquences sélectionnées, en particulier dans L-CDR1 et H-CDR1.

Parmi toutes les positions mutées, une combinaison de mutations a montré une affinité nettement améliorée : le clone v2, dont les affinité et cinétique de liaison sont montrées dans le tableau 3.

**Tableau 3 : Affinité et cinétiques de liaison pour le Fab 35PA83 et du clone v2.**

| Clone parental | Séquence H | Séquence L | KD (M) | Kon (M-1.s-1) | Koff (s-1) |
|---|---|---|---|---|---|
| 35PA83 | parentale | parentale | 3,4.10⁻⁹ | 9,3.10⁴ | 3,2.10⁻⁴ |
| Clone v2 | G>S (31A) | parentale | 6,6.10⁻¹⁰ | 1,22.10⁵ | 8,1.10⁻⁵ |
| | R>K (66) | | | | |
| | K>R (73) | | | | |

Les constantes d'association (kₒₙ) et de dissociation (k_{off}) ont été déterminées par résonance plasmonique de surface (BIAcore) et K_{D} a été calculé comme égal au rapport K_{off}/Kₒₙ.

Le triple mutant v2 a montré une constante de dissociation plus faible (K_{off} = 8,1 105 s-1) et une constante d'association un peu plus rapide (Kₒₙ = 1,22 105 M-1.s-1) que 35PA83, résultant en l'augmentation de l'affinité de 5,15 fois. Ce mutant présente 3 mutations dans le domaine variable de la chaîne lourde : une mutation (G31AS) dans H-CDR1 (le CDR1 de la région variable de la chaîne lourde) et deux mutations (R66K, K73R) dans H-CDR2 (le CDR2 de la région variable de la chaîne lourde).

Après le troisième cycle, les phages ont été criblés selon deux processus supplémentaires de sélection : panning dans des puits recouverts de l'antigène avec incubation longue (« sélection par incubation longue ») ou bien en utilisant de l'antigène biotinylé soluble à très petite concentration (« sélection par élution à très faible concentration d'antigène soluble »).

### Exemple 2: construction du vecteur d'expression HK558-12 pour l'expression de l'IgG chimérique cynomolgus-homme 35PA83 v2

La région variable de la chaîne lourde (VH) du variant sélectionné (v2) présente trois mutations comparée à celle du variant humanisé du Fab 35PA₈₃ : G→>S (6^{eme} résidu de HI, selon la nomenclature d'IMGT), R→>K (2^{ème} résidu d'ancrage de H2) et K→>R (6^{ème} résidu du FR3 selon la nomenclature d'IMGT.

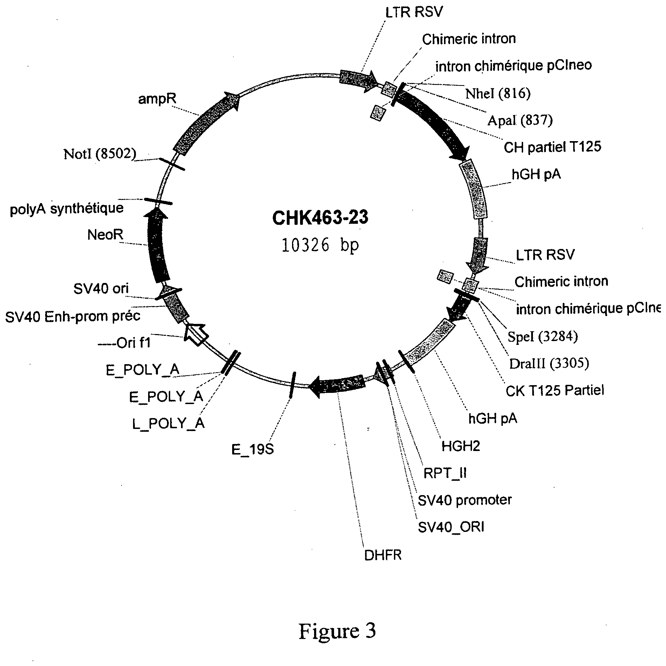

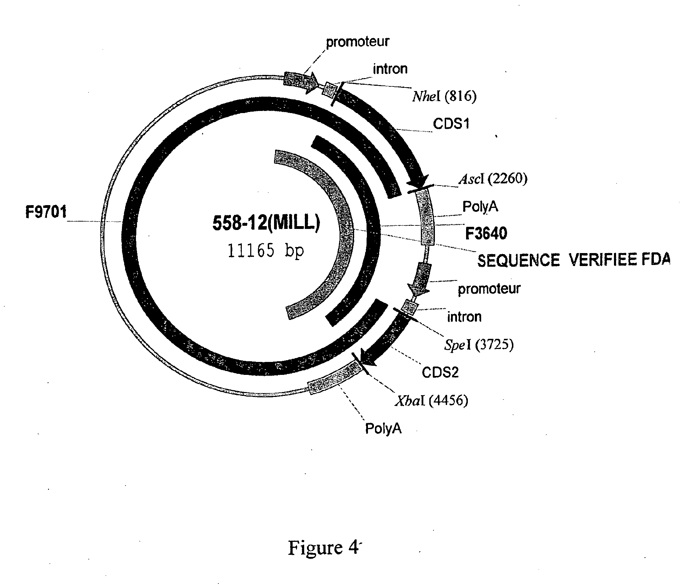

Le vecteur HK558-12 (voir figure 4) a été construit à partir du vecteur générique unique optimisé CHK463-23-1 (voir figure 3) par « double » chimérisation, c'est-à-dire ajout à la séquence de cynomolgus v2 des régions leaders humaines en 5' (les séquences de v2 clonées dans le plasmide pCOMB (Andris-Widhopf et al., 2000) ne comportent pas les séquences leaders), et des régions constantes CK et G1 humaines en 3'.

### 1. Principe des méthodes

Des techniques classiques de biologie moléculaire ont été mises en oeuvre pour la construction du vecteur HK558-12. Les séquences d'ADN d'intérêt ont été amplifiées par PCR d'assemblage (10 cycles avec enzyme "Proofstart DNA polymerase" Qiagen ref 202 203) et clonées (digestion par une enzyme de restriction puis ligation) dans un plasmide ou vecteur d'expression. Les plasmides recombinants ainsi obtenus ont été ensuite introduits dans des bactéries (transformation des bactéries) pour amplification (culture des bactéries) afin d'obtenir des quantités de vecteur suffisantes pour l'étape de transfection. Les vecteurs produits au cours de la culture bactérienne ont été ensuite purifiés puis linéarisés en prévision de la transfection dans les lignées YB2/0 et CHO.

### 1.1 Synthèse par PCR d'assemblage de la région variable de la chaîne légère du variant v2 (VKv2)

La région VKv2 correspond à la chimérisation des régions :
- leader de la région variable de la chaîne légère (VK) - début VK : séquence humaine Z0006 (Accession Number) (sous groupe VK1 ,VK1 -13), choix CRSSA-IMGT :
- VK cynomolgus (plasmide pComb3X, décrit dans l'article Laffly et al., 2005, qui contient les sequences VH et VL du v2).

La séquence leader humaine est ajoutée en 5' du VK cynomolgus pour donner la séquence suivante : (en italiques : début VK humain)

Cette séquence ne contient aucun des sites de restriction utilisé pour les clonages.

Amplification de la région VKv2 :
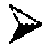 Couple d'amorces VK1_CA et VK2_CA

Ce couple d'amorces permet d'introduire le site Spe I et le début de la séquence leader correspondant au leader VK humain (VK1-13 Z00006, Accession Number) le plus proche de la séquence du v2.

L'amplicon obtenu correspond à l'amplicon 1 (78 pb). 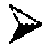

### Couple d'amorces VK3_CA et VK4_CA

Ce couple d'amorces permet introduire le reste de la séquence leader la région 5' du VK humain (VK1-13 Z00006) et le début du VK du V2. L'amplicon obtenu correspond à l'amplicon 2 (75 pb).

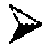 Couple d'amorces VK1_CA et VK4_CA

Ce couple d'amorces permet d'obtenir l'amplicon 3 (136 pb) par PCR d'assemblage des amplicons 1 et 2.

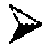 Couple d'amorces VK_CA_Nde et VK_CA_Dra

*VK_CA_Nde : 5'-TCGTCCCTGTCTGCATATGTGGGAG-3' (SEQ* ID N°20)

Ce couple d'amorces permet d'obtenir l'amplicon 4 (327 pb) en utilisant comme matrice le plasmide pCOMB v2. De plus, l'amplicon 4 contient en amont la région VK humaine permettant la dernière PCR d'assemblage avec l'amplicon 3.

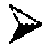 Couple d'amorces VK1_CA et VK_CA_Dra

Ce couple d'amorces permet d'obtenir l'amplicon 5 (438 pb) par PCR d'assemblage des amplicons 3 et 4. Il permet la concaténation des séquences leader VK humaine et du VK cynomolgus.

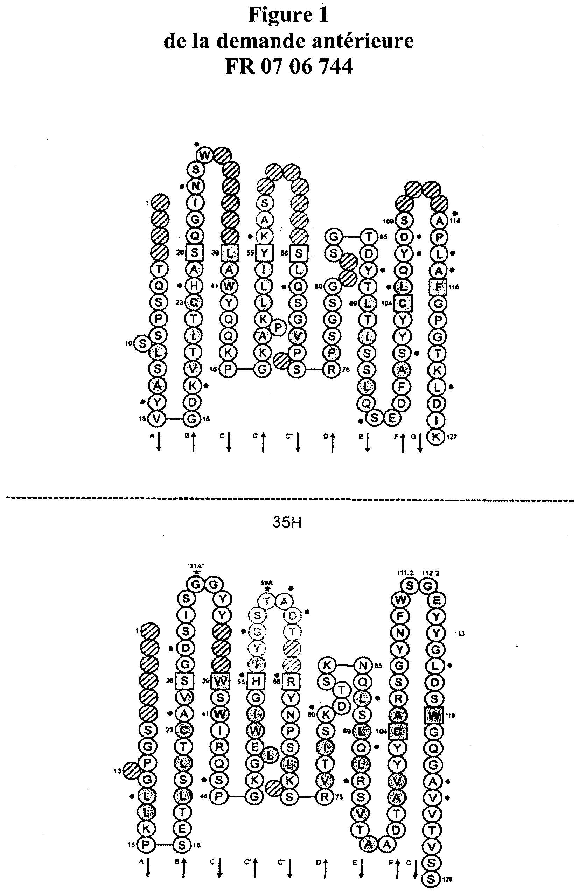

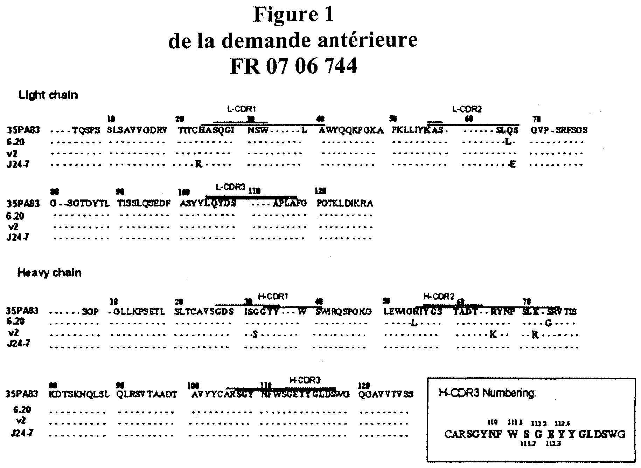

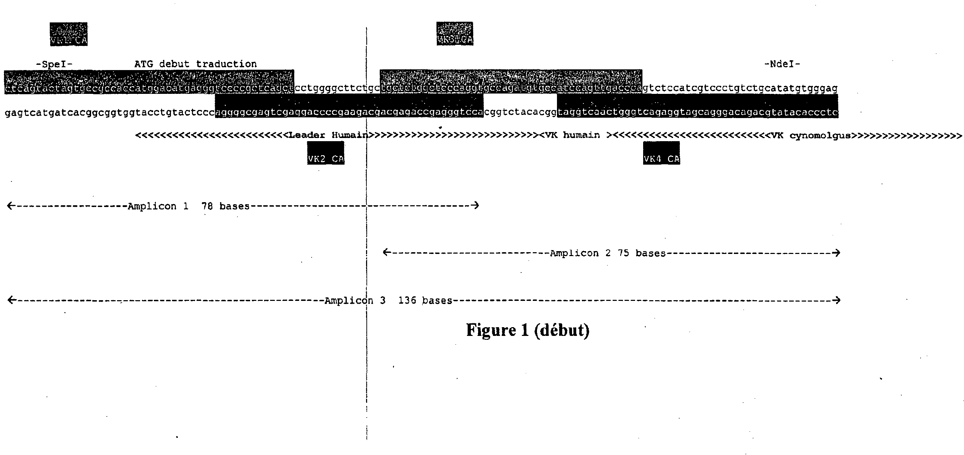

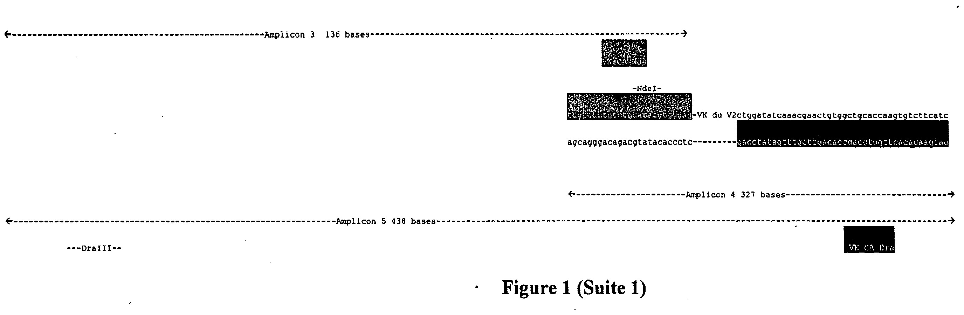

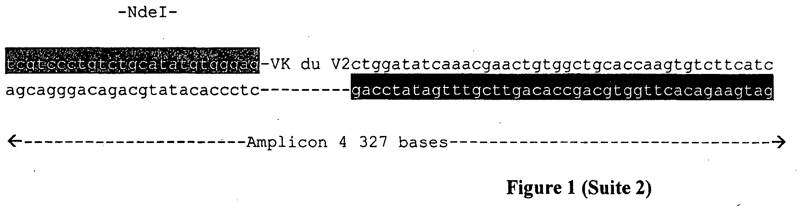

Les séquences des amorces et amplicons obtenus sont présentés à la figure 1.

L'amplicon 3 est obtenu par PCR d'assemblage des amplicons 1 et 2 introduisant le site Spe I, la séquence leader VK humaine et le début de la séquence VK du v2. L'amplicon 4 correspond à la région codante VK de v2.

L'amplicon 5 final est obtenu par PCR d'assemblage des amplicons 3 et 4 pour permettre la concaténation des séquences leader VK humaine et VK cynomolgus.

### 1.2 Synthèse par PCR d'assemblage de la région variable de la chaîne lourde avec le variant v2 (VHV2)

La région VHv2 correspond à la chimérisation des régions :
- leader VH - début VH : séquence humaine M29812 (Accession Number) (sous groupe VH4 ,VH4-59). Les gènes V humains codant les séquences les plus proches de 35PA83 : IGHV4-59*01 pour Fd et IGKV1-13*02 pour la chaine legere (nomenclature IMGT), mutées comme décrit ci-dessus
- VH cynomolgus (plasmide pComb3X)

La séquence humaine est ajoutée en 5' du VH cynomolgus pour donner la séquence suivante :
ATGAAACATCTGTGGTTCTTCCTTCTCCTGGTGGCAGCTCCCAGATGGGTC *CTGTCCCAGGTGCAGCTGCAGGAGT* (*SEQ* ID N° 24)
(en italiques : début VH humain)

Cette séquence ne contient aucun des sites de restriction utilisé pour les clonages.

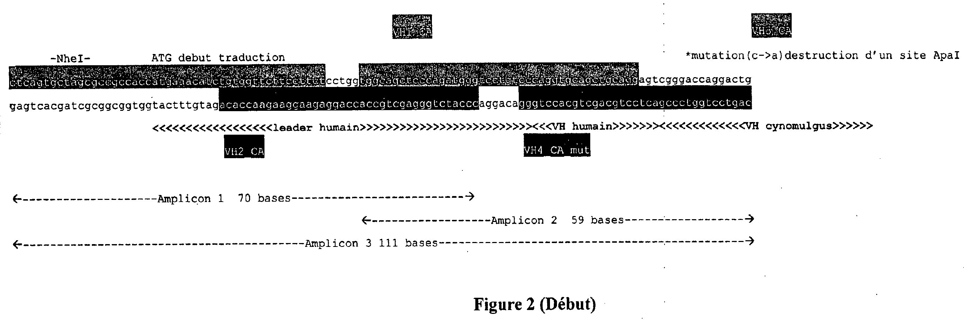

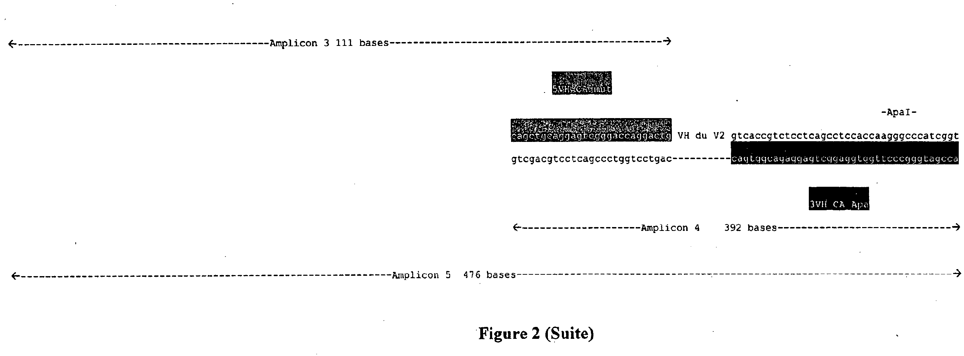

La figure 2 montre la synthèse de la région VHV2 avec les différents couples d'amorces et amplicons obtenus.

### 1.2.1 Amplification de la région VHV2

L'amplification de la région VHV2 est schématisée à la figure 2.

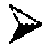 Couple d'amorces VH1_CA et VH2_CA

Ce couple d'amorces permet d'introduire le site Nhe I et le début de la séquence leader correspondant au leader VH humain (VH4-59 M29812, les gènes V humains codant les séquences les plus proches de 35PA83 : IGHV4-59*01 pour Fd et IGKV1-13*02 pour la chaine legere (nomenclature IMGT), mutées comme décrit ci-dessus) le plus proche de la séquence du v2. L'amplicon obtenu correspond à l'amplicon 1 (70 pb).

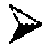 Couple d'amorces VH3_CA et VH4_CA_mut

Ce couple d'amorces permet d'introduire le reste de la séquence leader soit la région 5' du VH humain (VH4 M29812) et le début de la séquence VH de v2. L'amorce VH4_CA supprime par mutagenèse un site Apa I du début du VH. L'amplicon obtenu correspond à l'amplicon 2 (59 pb).

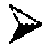 Couple d'amorces VH1 _CA et VH4_CA_mut

Ce couple d'amorces permet d'obtenir l'amplicon 3 (111 pb) par PCR d'assemblage des amplicons 1 et 2.

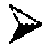 Couple d'amorces 5VH_CA_mut et 3VH_CA_Apa

*5VH_CA_mut : 5'-CAGCTGCAGGAGTCGGGACCAGGACTG-3' (SEQ* ID N°31)

Ce couple d'amorces permet d'obtenir l'amplicon 4 (392 bases) en utilisant comme matrice le plasmide pCOMB V2. De plus, l'amplicon 4 contient en amont la région VH humaine permettant la dernière PCR d'assemblage.

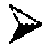 Couple d'amorces VH1_CA et 3VH_CA_Apa

Ce couple d'amorces permet d'obtenir l'amplicon 5 (476 pb) par PCR d'assemblage des amplicons 3 et 4. Il permet la concaténation des séquences leader VH humaine et VH cynomolgus.

Les séquences des amorces et amplicons obtenus sont présentés à la figure 2.

L'amplicon 3 est obtenu par PCR d'assemblage des amplicons 1 et 2 introduisant le site Nhe I, la séquence leader VH humaine et le début de la séquence VH du v2. L'amplicon 4 correspond à la région codante VH de v2.

L'amplicon 5 final est obtenu par PCR d'assemblage des amplicons 3 et 4 pour permettre la concaténation des séquences leader VH humaine et VH cynomolgus.

### 1.3 Séquençage et vérification FDA (Food and Drug Administration) du vecteur final

Le séquençage est réalisé selon la technique de Sanger (ou méthode des terminateurs de chaîne, réf. : Sanger F. et al, 1977, PNAS 74 : 5463).

Cette technique utilise l'incorporation aléatoire de didéoxynucléotides (ddNTP), ou « terminateurs », pour générer, à partir d'une extrémité fixe (zone de fixation de l'amorce de séquençage), tous les fragments se terminant par une base donnée (A, C, G ou T). L'analyse de ces fragments sur séquenceur automatique (séparation selon leur taille et détection) permet de définir l'ordre des différentes bases et donc la séquence d'un ADN donné.

Les séquençages ont été réalisés selon le niveau de qualité « FDA ». Il s'agit du niveau de qualité maximal, avec double couverture double brin de l'ADN séquencé, un minimum de redondance de 4 fois, une précision de 100%, des instruments dédiés, l'édition d'un rapport de qualité et l'archivage des documents générés.

Après séquençage, les séquences fournies par le prestataire sont comparées par alignement (logiciel AlignX, Vector NTI, Invitrogen) avec la séquence théorique attendue.

1.4 Amorces de criblage par PCR

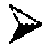 Amorce 5prsvbis

*5'-GCTCGATACAATAAACGCCA-3* (SEQ ID N°35)'

Amorce localisée dans l'intron de l'UT (unité de transcription) K ou H, utilisée avec CK4 ou GSP2ANP, permet la détection des inserts VK (amplicon de 781 pb) et VH (amplicon de 821 pb) après clonage.

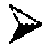 Amorce CK4

*5'-TCTGGGATAGAAGTTATTCAG-3 (SEQ* ID N°36)'

Amorce localisée en 5' de la région constante CK humaine, utilisée avec 5prsvbis, permet la détection des inserts VK après clonage.

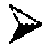 Amorce GSP2ANP

*5'-GGAAGTAGTCCTTGACCAGGCAG-3'* (SEQ ID N° 37)

Amorce localisée en 5' de la région constante G1 humaine, utilisée avec 5prsvbis, permet la détection des inserts VH après clonage.

### 1.5 Vecteur intermédiaire K558-12

### 1.5.1 Clonage dans le vecteur CHK463-23

Cette étape réalise la chimérisation cynomolgus-homme de la chaîne kappa de l'IgG 35PA₈₃ v2.

Après amplification par PCR d'assemblage et digestion par Spe I et Dra III, la séquence VKv2 a été clonée dans le vecteur CHK463-23 au niveau des sites unique Spe I et Dra III prévus à cet effet.

Après ligation, les colonies recombinantes ont été criblées pour la présence de l'insert par PCR à l'aide des amorces 5prsvbis et CK4 (amplicon de 781 pb).

Sur les 23 clones bactériens criblés par PCR, 20 étaient recombinants, porteurs de l'insert VKv2. Après purification des plasmides des clones 1 à 8 ont été contrôlés par restriction Nde I (8536, 1246, 943 bases), Dra III (linéarisation) et Spe I (linéarisation).

### 1.5.2 Séquencage de la région VKV2 du vecteur intermédiaire K558-12

Les 8 clones recombinants identifiés ont été contrôlés par séquençage avec le primer CK4.

Les clones 2, 3, 4, 5 et 8 ont une séquence correcte entre les sites de clonage Spe I et Dra III. Cependant, les clones 1 et 6 présentent des mutations et le clone 7 n'a pas donné de résultats exploitables.

Le clone 55806231-2 a été retenu pour poursuivre la construction du vecteur d'expression HK558-12.

### 1.6 Vecteur final HK558-12

### 1.6.1 Clonage dans le vecteur K558-12

Cette étape réalise la chimérisation cynomolgus-homme de la chaîne lourde de l'anticorps (IgG 35PA₈₃ v2).

Après amplification par PCR d'assemblage et digestion par Nhe I et Apa I, la séquence VHv2 a été clonée dans le vecteur intermédiaire K558-12 au niveau des sites unique Nhe I et Apa I prévus à cet effet. Après ligation, les colonies recombinantes ont été criblées pour la présence de l'insert par PCR à l'aide des amorces 5prsvbis et GSP2ANP (amplicon de 821 pb). Sur les 22 clones bactériens criblés par PCR, 18 étaient recombinants, porteurs de l'insert VHv2.

### 1.6.2 Séquencage de la région VHv2 du vecteur final et contrôle par restriction

Après purification, des plasmides des clones 1 à 5, 7, 9 et 11 ont été vérifiés par séquençage avec le primer GSP2ANP.

Les clones 2, 4, 7 et 9 ont une séquence correcte entre les sites Nhe I et Spe I tandis que les quatre autres clones présentent des mutations.

Les contrôles de restriction ont été réalisés sur les clones 2, 4, 7 et 9. L'analyse par restriction Nhe I (linéarisation), Apa I (linéarisation) et Bgl II + Nde I (2900, 2222, 1975, 1879, 1246, 934, 9 pb) a permis de confirmer que les quatre clones présentaient le profil de restriction attendu.

Le clone 55806298-9 a été choisi pour l'expression de l'anticorps chimérique cynomolgus-homme (IgG 35PA₈₃). La carte de ce vecteur est présentée en figure 4.

### 1.6.3 Contrôle du vecteur final par digestion

Une digestion Not I (linéarisation) et une double digestion Bgl II + Ndel (2900, 2222, 1975, 1880, 1246, 934, 9 pb) ont été réalisées afin de contrôler le plasmide purifié issu du clone 55806298-9 sélectionné.

Le profil de restriction obtenu correspondant à l'attendu, le clone 55806298-9 a été séquencé en qualité FDA. La séquence est conforme à l'attendu.

### 1.6.4 Préparation du vecteur HK558-12 pour la transfection

La préparation du vecteur HK558-12, linéarisé par Not I (cf. paragraphe 1.6.3), en tampon TE (10 mM Tris pH 8 et 1 mM EDTA) a été conservée à -20°C avant ajustement à la concentration de 1 µg/µl et cession au secteur Ingénierie Cellulaire pour transfection dans les lignées YB2/0 et CHO.

### Exemple 3: obtention de transformants producteurs de l'anticorps monoclonal chimérique cynomolctus-homme 35PA83 v2 dirigé contre l'antigène protecteur du charbon.

L'anticorps 35PA83 v2 a été produit dans les lignées YB2/0 (anticorps EMABIing®) et CHO (anticorps non EMABling®) afin d'étudier l'impact de la glycosylation sur son activité neutralisante des toxines *in vitro* et *in vivo.*

Pour les expérimentations ci-dessous, la technique ELISA mise en oeuvre se fait selon les conditions suivantes :

Des plaques de 96 puits microtitrés (maxisorp, Nunc, Danemark) sont coatés avec du PA dilué dans du PBS (5 µg/ml, 100 µl par puits), overnight à 4°C. Les plaques sont bloquées par ajout de 200 µl de PBS-BSA 5% à 37°C pendant 1 heure, et des sérum dilués en série dans du PBS-Tween 20 0.1%-BSA1% sont incubés (100 µl par puits) à 37°C pendant 2 heures. Une phosphatase alcaline anti-IgG de souris conjuguée ou une »anti-human IgG alkaline phosphatase conjugate » (Sigma) sont incubées (1/10 000) à 37°C pendant 1 heure. Un substrat P-Nitrophenyl Phosphate est ensuite incubé pendant 30 minutes à température ambiante. Les résultats sont déterminés par mesure de l'absorbance à 405 nm avec un automated microplate reader. (iEMS reader MF, Labsystems, Helsinky, Finland). Le dernier point de dilution dont la réversion détermine le titre du sérum, est déterminé comme donnant un signal inférieur ou égal à 2 fois le sérum naïf utilisé pour le contrôle négatif.

Le schéma d'obtention des transformants dans la lignée YB2/0 est illustré dans le Tableau 4, à la fin de la présente description.

### 1.1 Contrôle de la qualité de la transformation

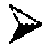 Taux de transformationLe taux de transformation a été évalué sur les taux de pousses des cellules en P96 cinq semaines après culture à J+3 en milieu sélectif.

En sélection simple avec l'agent sélectif G418, le taux de transformation est de l'ordre de 1/500 à 1/900. En sélection double avec les agents sélectifs G418 et MTX (méthotrexate), il est supérieur à 1/2200.

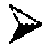 Taux de production moyen

3 pools de 8 puits P24 ont été effectués lorsque les puits sont remplis au % de cellules afin de réaliser une production maximale (J+7) pour évaluer la production moyenne.

Ces pools permettent d'évaluer les caractéristiques moyennes de la population considérée et permettent de s'assurer qu'un niveau minimum de ces caractéristiques est atteint alors même que les données sur les transformants ne sont pas encore disponibles.

La production moyenne avec le vecteur HK558-12 et le mélange témoin de vecteurs est respectivement de 1,2 µg/ml et de 3,3 µg/ml.

1.2 Sélection des cloïdes

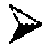 Taux de production : premier dépistage des cloïdes plus forts producteurs

La production d'IgG humaines a été déterminée par la technique ELISA sur les surnageants des puits de P96 en double sélection contenant ¾ de cellules afin de réaliser une première hiérarchisation des cloïdes sur leurs capacités de production.

Trois dépistages successifs (tous les 2-3 jours) ont été réalisés et les 10 meilleurs producteurs de chaque dépistage ont été sélectionnés. Sur 528 transformants, 27 ont été poursuivis et entretenus en P24 et une étude de leur productivité à J+3 et de leur production maximale (J+7) a été effectuée en parallèle.

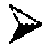 Productivité à J+3 et production maximale (J+7)

Les 15 cloïdes meilleurs producteurs sélectionnés avec une productivité en majorité supérieure à 5 pcd et une production maximale supérieure à 10 µg/ml ont été amplifiés cellulairement en milieu sélectif (double sélection) pour sauvegarde en azote liquide.

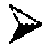 Taux de fucose

Un dosage du taux de fucose sur les surnageant des 15 cloïdes sélectionnés à J+3 et J+7 a été effectué par la technique ELISA.

### 1.3 Sélection du cloïde DD12 et production d'IgG en roller

Le cloïde DD12 a été retenu pour la production d'IgG en rollers (19L) car il est le meilleur cloïde concernant les critères de sélection pour la productivité (11,6 pcd), la production maximale (20,17 µg/ml) et le taux de fucose (26,9% à J+3 et 26,7% à J+7).

461 mg d'anticorps ont été produits pour purification . Après concentration (15 fois) et purification, 351 mg d'anticorps ont été obtenus soit une quantité suffisante pour les essais préliminaires *in vivo* à réaliser.

### 1.4 Colonage de 3 cloïdes

Les 3 cloïdes meilleurs producteurs DD12, FH2 et GA11 (productivité supérieure à 10 pcd, production maximale supérieure à 20 µg/ml et taux de fucose inférieur à 33%) ont été clonés par dilution limite afin d'anticiper une éventuelle instabilité des transformants.

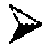 Production en IgG : premier dépistage des clones plus forts producteurs

La production d'IgG humaines a été déterminée par la technique ELISA (Enzyme Linked Immunosorbent Assay) sur les surnageants des puits de P96 contenant ¾ de cellules afin de réaliser une première hiérarchisation des clones sur leurs capacités de production.

Deux dépistages successifs (à 7 jours d'écart) ont été réalisés et les 8 meilleurs clones producteurs de chaque cloïde ont été sélectionnés. La productivité est supérieure à 6 µg/ml.

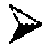 Productivité à J+3

La productivité à J+3 de ces 24 clones a été réalisée afin de sélectionner les 15 clones meilleurs producteurs soit 5 clones/cloïde. Elle est en majorité supérieure à 4 pcd.

Les 15 clones sélectionnés ont été amplifiés cellulairement pour sauvegarde en azote liquide.

### 1.5 Sélection du clone DD12-8F2

Le clone DD12-8F2 a été retenu comme meilleur clone sur les critères de sélection pour la productivité (6,6 pcd) et le taux de fucose à J+3 (27,8%).

Les caractéristiques du clone DD12-8F2 sont proches de celles de son cloïde parental DD12 excepté pour la productivité (J+3) qui est plus faible due à l'emploi de milieux différents. La productivité des clones étant homogène, elle valide la stabilité du cloïde.

### 1.6 Obtention de transformants dans la lignée CHO

Le flow-chart correspondant à l'obtention des transformants producteurs d'anticorps 35PA83 v2 dans la lignée CHO est représenté dans le Tableau 5 à la fin de la présente description.

### 2.1 Contrôle de la qualité de la transformation

### Taux de transformation

Le taux de transformation a été évalué sur les taux de pousses des cellules en P96 cinq semaines après culture à J+3 en milieu sélectif.

2.2Sélection des cloïdes

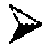 Taux de production : premier dépistage des cloïdes plus forts producteurs

La production d'IgG humaines a été déterminée par la technique ELISA sur les surnageants des puits de P96 en simple et double sélection contenant ¾ de cellules afin de réaliser une première hiérarchisation des cloïdes sur leurs capacités de production.

Trois dépistages successifs (tous les 2-4 jours) ont été réalisés et les 10 meilleurs producteurs de chaque dépistage ont été sélectionnés. Sur 953 transformants, 30 ont été poursuivis et entretenus en P24 et une étude de leur productivité à J+4 et de leur production maximale (J+7) a été effectuée en parallèle.

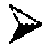 Productivité à J+4 et production maximale (J+7)

Les 15 cloïdes meilleurs producteurs sélectionnés avec une productivité en majorité supérieure à 1 pcd et une production maximale supérieure à 1 µg/ml ont été amplifiés cellulairement en milieu sélectif (simple ou double sélection selon les conditions d'obtention) pour sauvegarde en azote liquide.

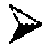 Taux de fucose

Un dosage du taux de fucose sur les surnageant des 15 cloïdes sélectionnés à J+7 a été effectué par la technique ELISA.

Le taux de fucose des IgG produites dans la lignée CHO est généralement supérieur à 75%.

### 2.3Amplification génique

Les transformants présentant des taux de production faibles et ne permettant pas de produire les quantités souhaitées d'anticorps, une amplification génique a été réalisée afin d'augmenter le nombre de copies de vecteurs intégrés et donc la productivité des cloïdes amplifiés.

L'amplification génique a été effectuée sur 3 cloïdes (13G8, 9D4 et 8F11) et 2 groupes de cloïdes (1 pool de 4 cloïdes (PA1) et 1 pool de 8 cloïdes (PA2)). Ce choix a été réalisé en prenant en compte l'ensemble des résultats obtenus pour la productivité J+4, la production maximale et le taux de fucose..

L'amplification génique a été réalisée par repiquage des cellules en milieu de culture sélectif avec G418 et MTX. La première étape d'amplification a été réalisée avec une concentration de MTX de 5 nM pour les cloïdes obtenus sans MTX et de 40 nM pour les cloïdes obtenus avec 10 nM de MTX. La production en IgG à J+4 est ensuite effectuée.

D'autres étapes d'amplification ont suivi en augmentant la concentration de MTX de 4 fois à la deuxième étape d'amplification et de 16 fois à la troisième.

L'étude des productions J+4 montre une augmentation de la production d'IgG au cours du processus d'amplification. En effet la productivité est environ quatre fois supérieure à celle obtenue avant amplification génique.

### 2.4Sélection de deux cloïdes et production en roller du cloïde 13G8

Les cloïdes 13G8 et 9D4 présentent le meilleur taux de production après amplification avec un maximum de productivité atteint à la deuxième étape d'amplification.

Les cloïdes 13G8 (20 nM MTX) et 9D4 (160 nM MTX) ont donc été amplifiés cellulairement en milieu sélectif pour sauvegarde en azote liquide.

Suite au dosage du taux de fucose réalisé sur les IgG purifiées issues des deux cloïdes 13G8 et 9D4, le cloïde 13G8 (20nM MTX) a été retenu pour la production d'IgG en rollers (5,5 L). Le taux de fucose sur IgG purifiées est de 76,6%.

65,5 mg d'IgG ont été produits pour purification. Après purification, 46.2 mg d'anticorps ont été obtenus.

### 2.5 Conclusion

Pour la production dans la lignée YB2/0, le cloïde DD12 a été retenu comme étant le meilleur sur l'ensemble des critères de sélection avec une productivité de 11,6 pcd, une production maximale de 20,17 µg/ml et un taux de fucose d'environ 27%.

La production en roller (19 L) de ce cloïde a permis d'obtenir 351 mg d'anticorps purifiés ayant un taux de fucose de 26%.

Pour la production dans la lignée CHO, le cloïde 13G8 (20 nM MTX) a été retenu comme étant le meilleur sur l'ensemble des critères de sélection avec une productivité de 8,2 pcd et un taux de fucose sur IgG purifiées de 76,6%.

La production en roller (5,5 L) de ce cloïde a permis d'obtenir 46,2 mg d'anticorps purifiés ayant un taux de fucose de 84%.

Les quantités d'anticorps purifiés produites dans ces deux lignées sont donc suffisantes pour permettre de réaliser les premiers essais *in vivo.*

### Exemple 4 : Essais de neutralisation in vitro

Après la production de DD12 dans des cellules YB2/0, le surnageant de culture cellulaire est récupéré, concentré 15 fois puis soumis à une chromatographie d'affinité au moyen d'une protéine recombinante de A-Sépharose. Une seconde étape de purification est mise en oeuvre au moyen de la colonne échangeuse de cation HiPrep 16/10 SP FF. L'intégrité de l'IgG purifiée et l'absence de contaminant sont contrôlés par SDS-PAGE et par ELISA pour la liaison au PA83 recombinant.

Les affinités sont mesurées par résonance de surface plasmonique (SPR) au moyen du BIAcore™ (Biacore Uppsala, Suède). PA83 (List biological laboratories, Campbell, CA) est immobilisé à un maximum de 210 RU sur une puce CM5 (Biacore) au moyen d'une liaison aminé, selon les instructions du fournisseur. Un flux de 30 µl/min est maintenu durant la mesure. Pour chaque mesure, un minimum de 6 dilutions d'IgG dans du tampon HBS-EP (Bioacore), avec des concentrations comprises entre 10 à 0.1 µg/ml, sont testées durant 1900 secondes. Après chaque dilution d'IgG, la puce est régénérée avec de la glycine pH 1.5 (Biacore), avec un flux de 10 µl/min pendant 30 secondes. Les constantes sont calculées au moyen d'une méthode d'analyte bivalent (Karlsson et al. 1991), et vérifié avec des tests de consistence interne (Schuck et al. 1996).

Le test de neutralisation in vitro est réalisé selon le protocole décrit par Little et al. (Little et al., 1990). La lignée cellulaire de macrophage de souris J774A.1 (ATCC-LGC, Molsheim, France) est incubée pendant 16 h à une concentration de 14,000 cellules/puits sur une plaque de 96 puits. Les composants de la toxine léthale, 400ng/ml de PA (List laboratories) et 40 ng/ml de LF, chacun étant dilué dans du PBS à 1 mg/ml et conservé congelé jusqu'à utilisation, sont ajoutés simultanément à l'IgG ou au milieu seul et incubés pendant 1 heure à 37°C. Le produit de l'incubation est ensuite ajouté aux macrophages et incubé à 37°C pendant 4 heures. L'essai Cytotox® (Promega) est utilisé selon les indications du fournisseur pour évaluer la viabilité des cellules. Chaque essai est corrigé pour une viabilité cellulaire de 100% (les puits de contrôle sont ceux qui ne contiennent pas de toxine ni d'IgG) et une viabilité de 0% (les puits de contrôle étant ceux contenant de la toxine mais pas d'IgG).

Résultats : L'affinité apparente de l'IgG 35PA83 est mesurée comme étant de 80pM et la valeur de 50% de neutralisation (IC₅₀) mesurée est de 0.75 ± 0.02nM (moyenne ± SD), représentant : (IgG 35PA83 / PA) de ¼ ou un ratio (IgG 35PA83 sites de liaison / PA) de ½.

### Exemple 5 : Etudes pharmacocinétiques

Pour évaluer le temps de demi-vie de l'IgG 35PA83, six souris A/J âgées de six semaines (Harlan, Gannat, France) ont été réparties en deux sous-groupes de taille égale. Toutes les souris ont reçu l'IgG 35PA83, administré par une injection unique sous-cutanée à la dose de 10 mg/kg. Le sang a été collecté par ponction rétro-orbitale quotidienne, à partir du Jour 1 et jusqu'au Jour 6 après l'injection, puis du Jour 8 jusqu'au Jour 10 après l'injection, en utilisant chaque jour distinct les souris en alternance. Le temps de demi-vie de l'IgG 35PA83 a été établi à partir des résultats des tests ELISA réalisés sur les pools d'échantillons de sérum, après extrapolation linéaire des valeurs obtenues.

Pour réaliser les Tests ELISA, les puits de plaques de microtitration de 96 puits ont été recouverts par incubation avec l'antigène PA83 ou l'antigène LF (List Laboratories) dilués dans du tampon PBS (5µg/ml, 100 µl par puits) pendant une nuit à 4°C. Les sites libres des puits de microplaque ont ensuite été bloqués par incubation avec un volume de 200 µl d'une solution d'albumine sérique bovine (BSA) à 5% dans du tampon PBS, pendant 1 heure à 37°C. Les serums ont été dilués en série dans un tampon de PBS à 0,1% de Tween® 20, 1% de BSA, puis incubés dans les plaques (100 µl/puits), pendant 2 heures à 37°C. Les puits des plaques ont été ensuite incubés avec un conjugué IgG anti-souris/phosphatase alcaline ou un conjugué IgG anti-humain/ phosphatase alcaline dilué au 1/10 000 (Sigma, Saint Louis, Missouri, Etats-Unis), pendant 1 heure à 37°C. Le substrat phosphate de P-nitrophényle (Sigma) a ensuite été ajouté et les plaques ont été incubées pendant 30 minutes à température du laboratoire. On a déterminé l'absorbance à 405 nm en utilisant un lecteur automatisé de microplaques (iEMS reader MF, Labsystems, Helsinki, Finlande). On a défini le point de dilution limite, dont la valeur réciproque correspond au titre anticorps du sérum, comme le point pour lequel la valeur du signal était égale au double de la valeur du signal mesurée pour le sérum de souris naïves. Le sérum de souris naïves est utilisé comme témoin négatif.

Résultats : Le temps de demi-vie de l'IgG 35PA83 chez les souris A/J a été déterminé comme étant de 7,78 ± 1,46 jours.

### Exemple 6 : Etudes de protection passive des rats

Pour les essais *in vivo,* ont injecte à des rats Fischer (250 à 300g) (C. River, L'Abresle, France) 40 µg de PA (List biological laboratories, Campbell, CA) et 8 µg de LF par 250g de rat, de la manière décrite dans Ezzel et al. (Ezzell et al., 1984), excepté le fait que la veine de la queue est utilisée. 4 animaux sont utilisés par groupe et pour l'évaluation de l'IgG 35PA83, l'IgG est ajoutée au PA et au LF avant l'injection. On observe les rats 2 fois par jour pendant 10 jours. Tous les essais in vivo présentés dans cette étude sont approuvés par le comité d'éthique local pour l'expérimentation et le soin animal.

### Préparation et utilisation des spores Sterne :

Des spores de B. anthracis Sterne (collection Pasteur) sont préparées comme exposé dans Albrecht et al. (Albrecht et al., 2007), et gardées congelées (-20°C). Les spores sont numérées par comptage de plaque viable après congélation/décongélation et le compte est vérifié quand chaque tube est utilisé dans cette étude. La LD50 de ces spores administrées par voie intraveineuse dans des souris males A/J (Harlan, Gannat, France), âgées de 9 semaines, pesant 20-25 g, sont établies à 1.10⁴, menant à la mort en 48 à 72 heures, proche d'une valeur de 2.10⁴ utilisée dans une autre étude (Albrecht et al., 2007).

Résultats : Les rats injectés avec des toxines meurent en seulement 2 heures. Lorsqu'ils sont protégés par 0.15 nmol d'IgG 35PA83, 2 rats seulement meurent à 4.5 heure et 5 heure (effet considéré significatif par analyse statistique, p = 0.045). Les 4 rats survivent quand 0.2 nmol d'IgG 35PA83 est utilisé (effet significatif, p = 0.03), correspondant à un ratio molaire (sites de liaison à l'antigène de l'IgG / PA83) de 0.8.

### Exemple 7 : Prophylaxie par l'IgG 35PA83 v2, court traitement avec la tétracycline, ou les 2

Pour les études d'un schéma prophylactique de l'IgG 35PA83 v2 ou par tétracycline seulement, on injecte les IgG à des groupes de 10 souris A/J, par voie subcutanée, 12 heures avant l'infection (une injection de 5 mg/kg ou de 2 mg/kg). Le challenge est administré comme 10 000 LD50 ou 1.10⁸ spores et les souris sont observées 2 fois par jour pendant 2 semaines, puis 5 fois par semaine pendant 2 semaines additionnelles. Les souris survivantes sont re-testées par infection un mois plus tard, avec la même quantité de spores, et observées pendant un mois additionnel. Pour les études d'un schéma prophylactique impliquant à la fois la tétracycline et l'IgG 35PA83 des groupes de 10 souris sont traités avec la tétracycline comme dans le schéma impliquant la tétracycline seule mais, en plus, l'IgG 35PA83v2 est injecté 12 heures avant le challenge. Pour les études de protection active, 10 souris sont injectées par voie subcutanée avec 5 µg de PA par souris, dans de l'adjuvant complet de Freund. Un second groupe reçoit la même injection puis, 1 mois plus tard, la réponse immunitaire de ce groupe est stimulée avec la même dose de PA dans de l'adjuvant de Freund incomplet.

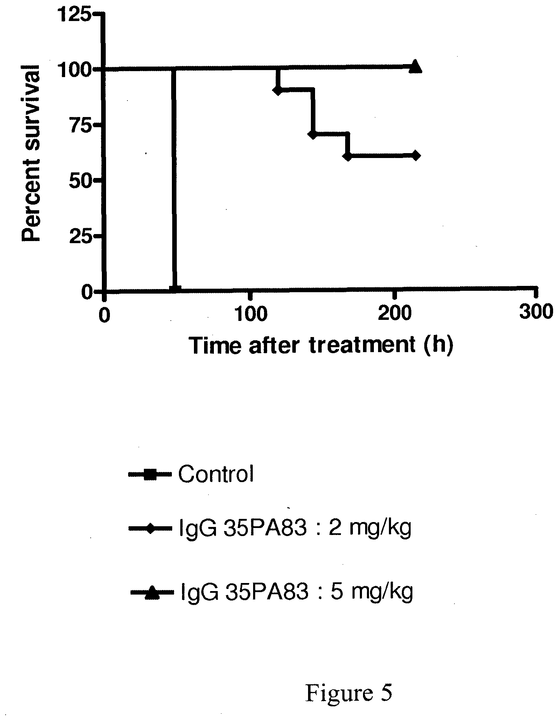

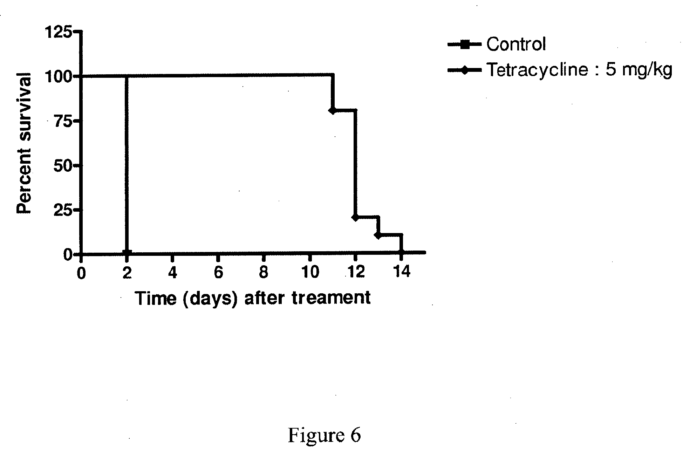

Résultats : Les traitements prophylactiques commencent 12 heures avant un challenge de 10 000 LD50 et les courbes de survie sont montrées en figures 5 et 6 (tétracycline, 7 jours). Toutes les souris ayant reçu 5 mg/kg d'IgG 35PA83 survivent et 60% des souris ayant reçu 2 mg/kg d'IgG 35PA83 survivent (significatif, p<0.0001). Dans ces 16 souris survivantes, un mois après l'injection du 35PA83 et le challenge, aucun anticorps n'est détectable au moyen du conjugué anti-IgG humaine, et il est ainsi considéré qu'aucune IgG 35PA83 n'est encore présente à ce stade. Les titres de ces souris en IgG murin dirigé contre le PA est dans la plage de 64 000-128 000, et leurs titres d'IgG murine dirigées contre le LF est dans la plage de 32 000 à 64 000, indépendamment de la dose reçue. Quand ces souris sont re-challengées avec 10 000 LD50 de spores Sterne, un mois après l'infection initiale, toutes survivent.

Toutes les souris ayant reçu un traitement prophylactique avec la tétracycline survivent durant le traitement (7 jours). Toutefois, ces souris meurent entre 4 et 7 jours après l'arrêt de l'administration de l'antibiotique. Quand la dernière injection est complémentée avec une injection de l'IgG 35PA83 à une dose de 1 mg/kg, 20% des souris survivent (non significatif) mais toutes survivent quand une dose de 2 mg/kg est utilisée (significatif, p=0.008). Les souris qui sont activement protégées par une injection de PA ont un titre d'anticorps anti-PA compris entre 25 000 et 50 000 un mois plus tard. Puis elles sont challengées, et 6 souris sur 10 survivent (considéré comme significatif, p=0.01). Les souris qui sont activement protégées par 2 injections de PA ont un titre d'anticorps anti-PA compris entre 160 000 et 640 000 1 mois après la seconde injection, et toutes survivent

Ainsi, les tests effectués montrent qu'une injection de IgG 35PA83 v2 permet de raccourcir la prophylaxie basée sur la tétracycline et, quand l'anticorps a été injecté une fois à une dose de 2 mg/kg à la fin de la prophylaxie, une survie complète des souris est observée. Alors que des souris protégées avec un court traitement de tétracycline (7 jours) sont.injectées avec 1000 LD50 de *B. anthracis* sterne strain, 12 heures après avoir commencé le traitement, 60% des souris survivent. Après avoir challengé par 10 000 LD50, toutes les souris ayant reçu la tétracycline meurent en moins de 7 jours après l'arrêt de l'administration de l'antibiotique, probablement à cause d'une germination des spores tardive. Ainsi, ceci démontre qu'une prophylaxie au moyen de tétracycline peut être arrêtée, de manière sécurisée, après un risque d'exposition, par un short traitement de tétracycline suivi d'une injection d'IgG 35PA83. Cette solution a l'avantage d'envisager un meilleur respect de la prise d'antibiotique par les personnes, puisque cette prise est plus courte, ainsi que des coûts réduits dus à la réduction des doses d'antibiotiques administrées.

### Exemple 8 : Prophylaxie par l'IgG 35PA83 v2, court traitement avec la doxycycline, ou les 2

L'étude de la prophylaxie avec la doxycycline, avec ou sans l'IgG 35PA83, a été réalisée avec des groupes de dix souris A/J âgées de 10 semaines (Harlan, Gannat, France), auxquelles on a injecté de manière prophylactique l'antibiotique par voie intra péritonéale, à la dose unique quotidienne de 5 mg/kg. La chimioprophylaxie a débuté 12 heures avant l'infection et a été réalisée pendant 7 jours, ce qui représente une réduction de 9/10 de la durée standard qui est de 60 jours.

On a choisi un dosage de doxycycline qui est approximativement le double du dosage humain standard (dosage quotidien de 3 mg/kg pour un humain adulte), et on a montré que des doses plus petites étaient efficaces contre *B. anthracis* (Friedlander et al., 1993, J Infect Dis, Vol. 167 : 1239-1243 ; Kalns et al., 2002, Biochem Biophys Res Commun, Vol. 297 : 506-509). Des doses plus grandes ont été utilisées (Heine et al., 2007, Antimicrob Agents Chemother, Vol. 51 : 1373-1379) ; on a toutefois observé qu'une dose de 50 mg/kg semblait mal tolérée chez les souris A/J, qui présentaient alors un gonflement de l'abdomen et un poil hérissé. Pour complémenter le traitement par la doxycycline avec l'IgG 35PA83, une dose unique de cet anticorps (1 ou 2 mg/kg) a été injectée ou non de manière concomitante à la dernière dose de doxycycline. L'infection a utilisé 1 x 10⁸ spores injectées par voie intra péritonéale, ce qui représente 10 000 LD₅₀. Les souris ont été observées deux fois par jour pendant les deux premières semaines, puis cinq fois par semaine pendant les deux semaines additionnelles.

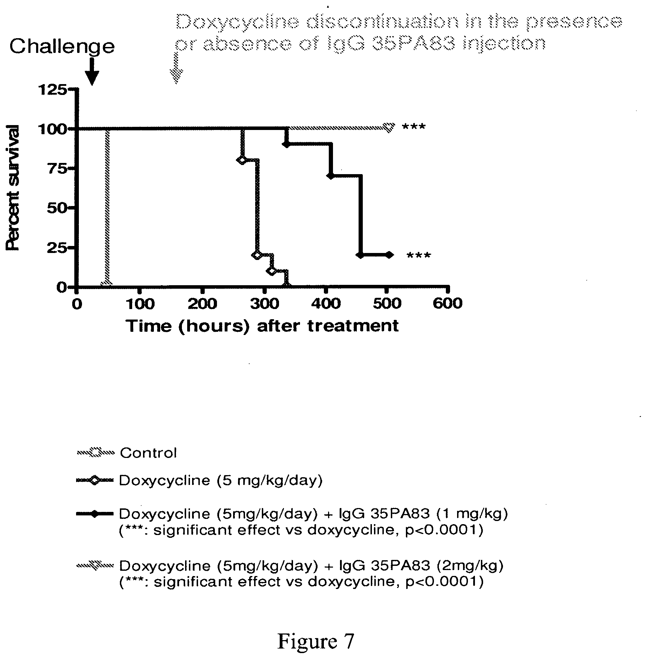

Résultats : Après la dissémination de l'anthrax aux Etats-Unis en 2001, il a été montré une observance incomplète du traitement prophylactique de longue durée (60 jours) aux antibiotiques. Afin de déterminer si l'IgG 35PA83 pouvait raccourcir le temps de traitement, on a débuté un traitement prophylactique avec une dose quotidienne de 5 mg/kg de doxycycline pendant seulement 7 jours, 12 heures avant 'infection avec 10 000 LD₅₀. Le dernier jour du traitement, le traitement prophylactique aux antibiotiques a été complémenté ou non avec une injection unique de l'IgG 35PA83 (**Figure 7**). Cependant, si la dernière injection de doxycycline était complémentée avec 1 mg/kg d'IgG 35PA83, la durée moyenne jusqu'à la mrt était augmentée de 288 à 456 heures et 20% des souris ont survécu, ce qui représente un effet protecteur significatif (comparaison *versus* doxycycline, p<0,001). Avec une dose de 2 mg/kg d'IgG 35PA83, la totalité des dix animaux a survécu. Un mois après l'infection, les sera des douze souris survivantes ont été prélevés, réunis en pools, et stockés à -20°C, de manière systématique. Lorsque les sera ont été testés par ELISA, la moyenne du titre anticorps IgG anti-PA était de 64 000 et la moyenne du titre anticorps IgG anti-LF était de 32000.

### Exemple 9 : Thérapie avec l'IgG 35PA83 v2, ciprofloxacine court traitement ou les 2

Pour les études de schéma thérapeutique, des groupes de 10 souris A/J sont challengées avec une dose de 1000 LD50 ou 1.10⁷ spores. Après 12 heures, l'IgG 35PA83 v2 (voie subcutanée, 1 injection de 10 mg/kg) ou de ciprofloxacine (voie subcutanée, 50 mg/kg 2 fois par jours pendant 5 jours) sont injectés séparément ou la ciprofloxacine et l'IgG 35PA83 sont toutes deux injectées le premier jour puis la ciprofloxacine seule est encore injectée pendant 4 jours additionnels.

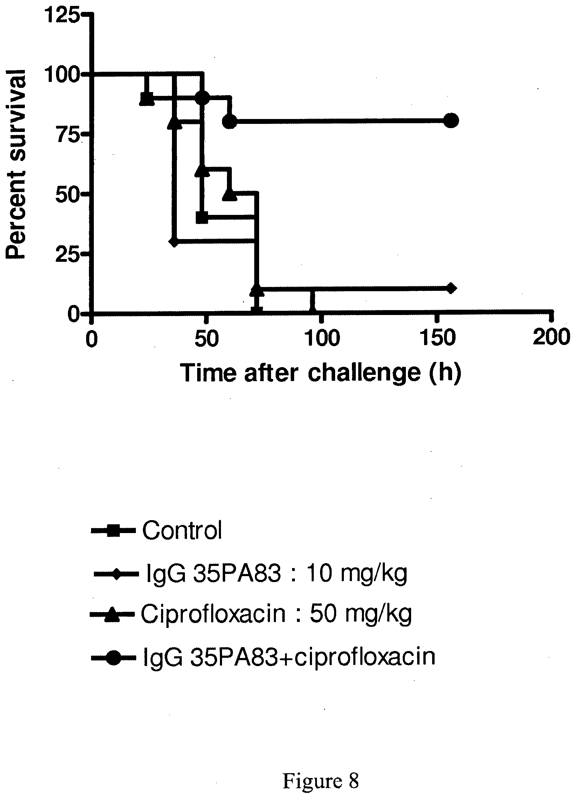

Résultats : Les traitements thérapeutiques commencent 12 heures après un challenge de 1000 LD50 et les courbes de survie (ciprofloxacine, 5 jours de traitement ou IgG 35PA83 ou les 2) sont montrées en figure 8. Aucune souris ayant reçu 5 jours de ciprofloxacine ne survit, seulement 10% de souris ayant reçu l'IgG 35PA83 survivent (non significatif). Toutefois, 80% des souris ayant reçu à la fois la ciprofloxacine et l'IgG 35PA83 survivent (significatif, p=0.0007).

L'utilisation simultanée de la ciprofloxacine et de l'IgG 35PA83 permet une survie de 80%. Un fort effet synergique de IgG PA83 et de ciprofloxacine a été démontré pour la thérapie, et, de manière intéressante, aucun vaccin ne permet un traitement par antibiotique aussi court (5 jours), étant donné que ce délai ne permet pas l'élaboration d'une réponse immunitaire. De plus, IgG 35PA83 prévient certainement les rechutes après ce court traitement d'antibiotique, et il pourrait être encore plus efficace pour cette raison chez l'homme, où son temps de survie est attendu pour être au moins 3 fois plus long que chez la souris (3 à 7 jours vs 21 jours).

### Exemple 10: Thérapie avec l'IgG 35PA83 v2, ciprofloxacine court traitement ou les 2 (autre essai)

Pour les études de traitement curatif, des groupes de 10 souris A/J sont infectées avec une dose de 1000 LD₅₀ ou 1.10⁷ spores. Après 12 heures, les souris ont été traitées avec la ciprofloxacine (par voie sous-cutanée, avec une injection initiale de 25 mg/kg) ou avec l'IgG 35PA83 v2 (voie subcutanée, 1 injection de 10 mg/kg) séparément ; ou la ciprofloxacine et l'IgG 35PA83 sont toutes deux injectées simultanément à deux sites différents. On a aussi testé des délais additionnels de 24 heures et 48 heures avant de débuter le traitement combiné (ciprofloxacine et IgG 35PA83). Après la première administration du traitement, la ciprofloxacine seule (25 mg/kg, deux fois par jour) a été injectée durant les 4,5 jours suivants. La dose de ciprofloxacine a été choisie comme étant approximativement le double de la dose standard humaine (dose quotidienne de 20 mg/kg chez l'homme adulte), cette dose ayant déjà été utilisée efficacement contre *B. anthracis* (Kalns et al., 2002, Biochem Biophys Res Commun, Vol. 297 : 506-509). La tolérance à cette dose sélectionnée a été favorablement testée chez les souris A/J avant de débuter cette étude. Cette partie de l'étude vise essentiellement à résoudre le problème de la survie à court-terme suivant un traitement différé, et la surveillance a été limitée à la période de 18 jours suivant l'infection.

Résultats : l'anthrax est rarement rencontré dans la pratique courante et son diagnostic et son traitement ont une forte probabilité d'être différés. Dans cette étude, des traitements uniques (traitement de cinq jours de ciprofloxacine à la dose de 50 mg/kg/jour ou une dose unique de 10 mg/kg d'IgG 35PA83) ont été différés de 12 heures après une infection avec 1000 LD₅₀ de spores de Sterne, et les traitements combinés (ciprofloxacine combinée à IgG 35PA83) ont été différés de 12, 24 et 48 heures après la même infection (**Figure 9**) et testés pour leur efficacité. Les souris traitées avec la ciprofloxacine seule n'ont pas survécu et le traitement avec l'IgG 35PA83 seul a permis la survie d'une seule des dix souris testées (non significatif). Cependant, 80% des souris traitées avec à la fois la ciprofloxacine et l'IgG 35PA83 ont survécu lorsque le traitement a été différé de 12 heures (significatif par rapport au témoin non traité, p=0,0007), et 60% des souris ont survécu lorsque le traitement a été différé de 24 heures (significatif par rapport au témoin non traité, p=0,003). A la 48^{ème} heure suivant l'infection sans traitement, seules deux des dix souris avaient survécu, mais sont mortes peu de temps après, malgré l'administration du traitement combiné. Dix-huit jours après l'infection, les sera des quatorze souris survivantes ont été prélevés, réunis en pools, stockés puis testés par ELISA. Le titre d'anticorps IgG anti-PA était de 32000 et le titre d'anticorps IgG anti-LF était de 8000.

### Exemple 11 : Comparaison entre les traitements anti-anthrax prophylactiques passifs et actifs

Un traitement anti-anthrax prophylactique passif consiste en un traitement avec l'IgG 35PA83. Un traitement anti-anthrax prophylactique actif consiste en un traitement par immunisation avec l'antigène PA.

Pour comparer l'immuno-protection active et passive, un groupe de dix souris ont été immunisées par voie sous-cutanée avec 5 µg de PA83 dans de l'adjuvant complet de Freund et infectées par voie intra péritonéale avec 10 000 LD₅₀, un mois après. Un autre groupe de dix souris ont été immunisées de manière identique, mais ont reçu une immunisation de rapel quatre semaines après avec 5 µg de PA83 dans de l'adjuvant incomplet de Freund et infectées un mois après la seconde injection. En parallèle, on a évalué la protection passive par l'IgG 35PA83 contre la même infection. Tous les animaux infectés ont été observés pendant un mois, et les résultats des deux types de prohylaxie ont été comparés.

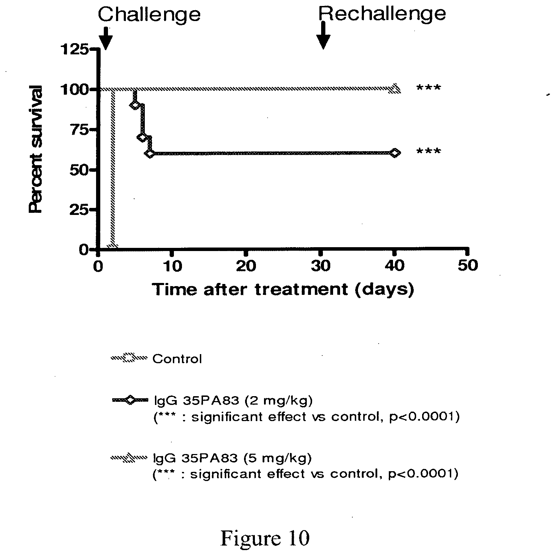

Résultats : La vaccination basée sur l'injection de PA est couramment le moyen prophylactique le plus utilisé contre l'anthrax, et son efficacité est corrélée avec les titres anticorps anti-anthrax (Grunow et al., 2007, Vaccine, Vol. 25 : 3679-3683). On a immunisé les souris A/J vace le PA, afin d'obtenir des titres anti-PA d'une valeur équivalente à celle qui est observées chez les humains vaccinés, de manière à comparer l'efficacité d'une telle vaccination avec la protection apportée par l'IgG 35PA83. Les souris immunisées avec une injection unique de PA83 avaient des titres d'anticorps anti-PA de 25 000 à 50 000, un mois après l'immunisation. Les souris ont été infectées avec 10 000 LD₅₀ de spores Sterne, et six des dix souris ont survécu (comparaison par rapport aux souris naïves témoin significative, p=0,01). Dix souris immunisées avec deux injections de PA83 avaient des titres anticorps anti-PA83 de 160 000 et 640 000 et ces dix animaux ont survécu à une infection similaire, un mois après la fin du traitement d'immunisation, ce qui démontre un niveau accru de protection significatif (p=0,02) avec un traitement avec deux injections, par rapport à un traitement avec une injection. En parallèle, on a évalué la protection passive avec l'IgG 35PA83, à des doses de 2 ou 5 mg/kg. Six des dix souris protégées avec 2 mg/kg d'IgG 35PA83 ont survécu (protection significative en comparaison avec les souris non traitées, p<0,0001, **Figure 10**), et toutes les souris ayant reçu des injections prophylactiques de 5 mg/kg d'IgG 35PA83 ont survécu. Dans cette étude, la protection complète qui a été permise par l'injection de 5 mg/kg d'IgG 35PA83 a donc été équivalente à la protection complète permise par deux immunisations avec PA83, qui était elle-même meilleure que la protection apportée par une injection unique de PA83.

Les souris ayant survécu après le traitement prophylactique passif par l'IgG 35PA83 seul ont été de plus étudiées un mois après l'infection initiale. Dans un test ELISA utilisant le PA83 comme antigène et le conjugué IgG anti-humain, on n'a détecté aucun signal dans les sera correspondants, et il a été conclu à l'absence d'IgG 35PA83 chez les souris un mois après l'injection, ce qui est en accord avec la valeur du temps de demi-vie de l'IgG 35PA83. Cependant, avec le conjugué IgG anti-souris, on a détecté des IgG murines dirigées contre le PA à un titre de 64 000 à 128 000, et des IgG murines dirigés contre le LF à un titre de 32 000 à 64 000, indépendamment de la dose d'IgG 35PA83 administrée. Tous les animaux ont survécu à une ré-infection avec 10 000 LD₅₀ de spores Sterne, un mois après l'infection initiale.

Le tableau 7 ci-dessous résume les résultats des titres anticorps IgG anti-PA et anti-LF observés, dans les quatre conditions expérimentales.

**Tableau 7**

| Conditions expérimentales | Titre IgG anti-PA | Titre IgG anti-LF |
|---|---|---|
| Prophylaxies par doxycycline et IgG 35PA83 (2 mg/kg) : pool de 10 sera, collectés un mois après l'infection | 64 000 | 32 000 |
| Traitement curatif avec la ciprofloxacine et l'IgG 35PA83 : pool de 14 sera, collectés 18 jours après l'infection | 64 000 à 12 000 | 32 000 à 64 000 |
| Immunisation avec PA83 : une injection, sera collectés un mois après l'injection | 25 000 à 50 000 | NP* |
| Immunisation avec PA83 : deux injections, sera collectés un mois après la seconde injection (injection de rappel) | 160 000 à 640 000 | NP |

| | | |
|---|---|---|
| * NP = Non Pertinent | | |

### Statistiques des études in vivo :

Un test log-rank de coparaison d'une analyse de survie de Kaplan-Meier est effectuée sur un logiciel Graph Prism 4.0 (GraphPad software, San Diego, CA).

### Références bibliographiques

Albrecht, M. T., H. Li, E. D. Williamson, C. S. Lebutt, H. C. Flick-Smith, C. P. Quinn, H. Westra, D. Galloway, A. Mateczun, S. Goldman, H. Groen, and L. W. Baillie. 2007. Human monoclonal antibodies against anthrax lethal factor and protective antigen act independently to protect against Bacillus anthracis infection and enhance endogenous immunity to anthrax. Infect Immun.
Andris-Widhopf, J., C. Rader, P. Steinberger, R. Fuller, and C. F. Barbas, 3rd. 2000. Methods for the génération of chicken monoclonal antibody fragments by phage display. J Immunol Methods 242:159-181.
Andris-Widhopf, J., P. Steinberger, R. Fuller, C. Rader, and C. F. Barbas, 3rd. 2001. Generation of antibody libraries: PCR amplification and assembly of light - and heavy-chain coding sequences. In C. F. Barbas, 3rd, D. R. Burton, J. K. Scott, and G. J. Silverman (ed.), Phage Display: A Laboratory Manual. Cold Spring Harbor Laboratory Press, New York.
Carter, P., H. Bedouelle, and G. Winter. 1985. Improved oligonucleotide site-directed mutagenesis using M13 vectors. Nucleic Acids Res 13:4431-43.
Ezzell, J. W., B. E. Ivins, and S. H. Leppla. 1984. Immunoelectrophoretic analysis, toxicity, and kinetics of in vitro production of the protective antigen and lethal factor components of Bacillus anthracis toxin. Infect Immun 45:761-7.
Karlsson, R., A. Michaelsson, and L. Mattsson. 1991. Kinetic analysis of monoclonal antibody-antigen interactions with a new biosensor based analytical system. J Immunol Methods 145:229-40).
Laffly, « Selection of a macaque Fab with framework régions like those in humans, high affinity, and ability to neutralyse the protective antigen (PA) of bacillus anthracis by binding to segment of PA between residues 686 and 694 », Antimicrobial agents and chemotherapy, Aug. 2005, p. 3414-3420.
Little, S. F., S. H. Leppla, and A. M. Friedlander. 1990. Production and characterization of monoclonal antibodies against the lethal factor component of Bacillus anthracis lethal toxin. Infect Immun 58:1606-13.
Schuck, P., and A. P. Minton. 1996. Analysis of mass transport-limited binding kinetics in evanescent wave biosensors. Anal Biochem 240:262-72.

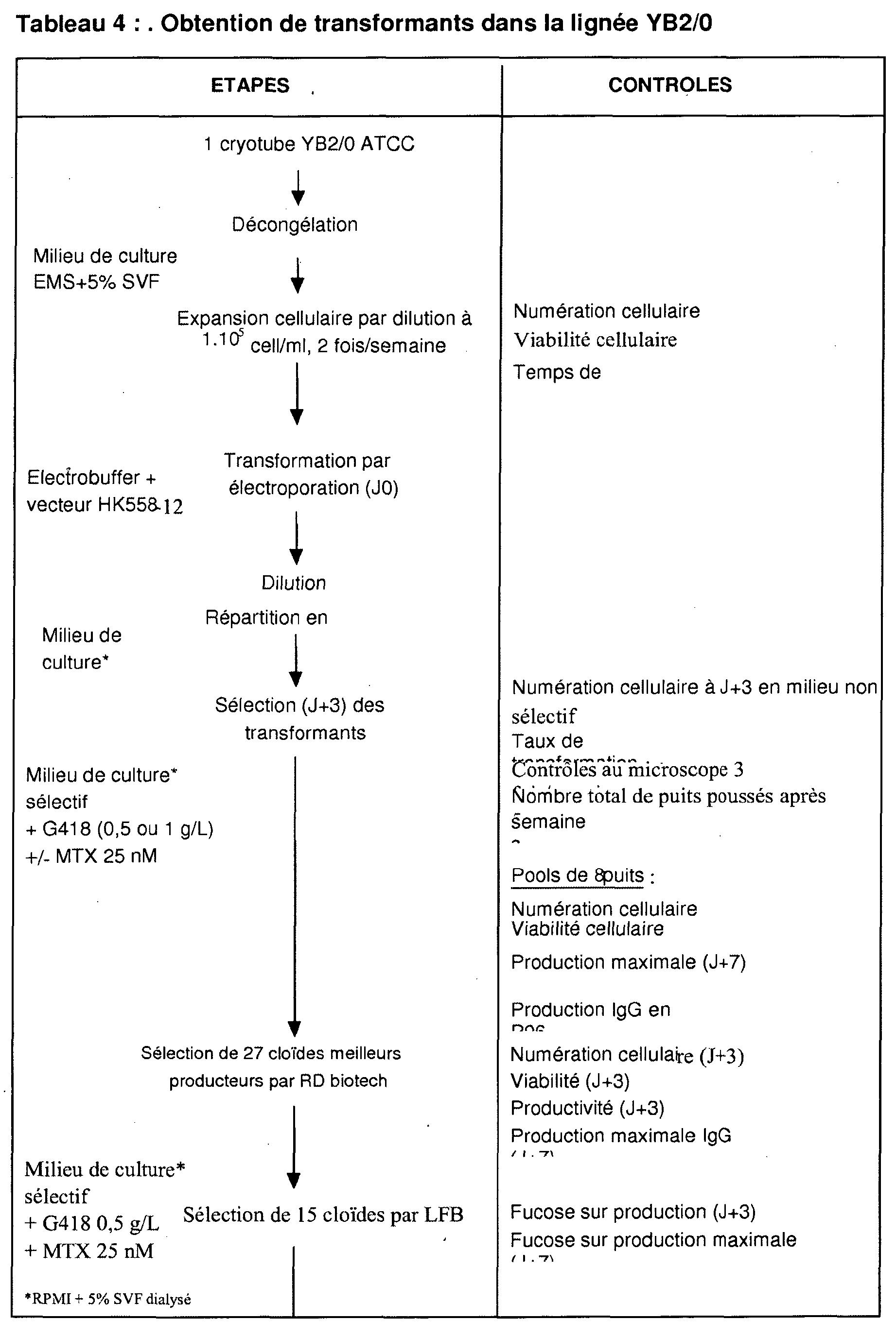

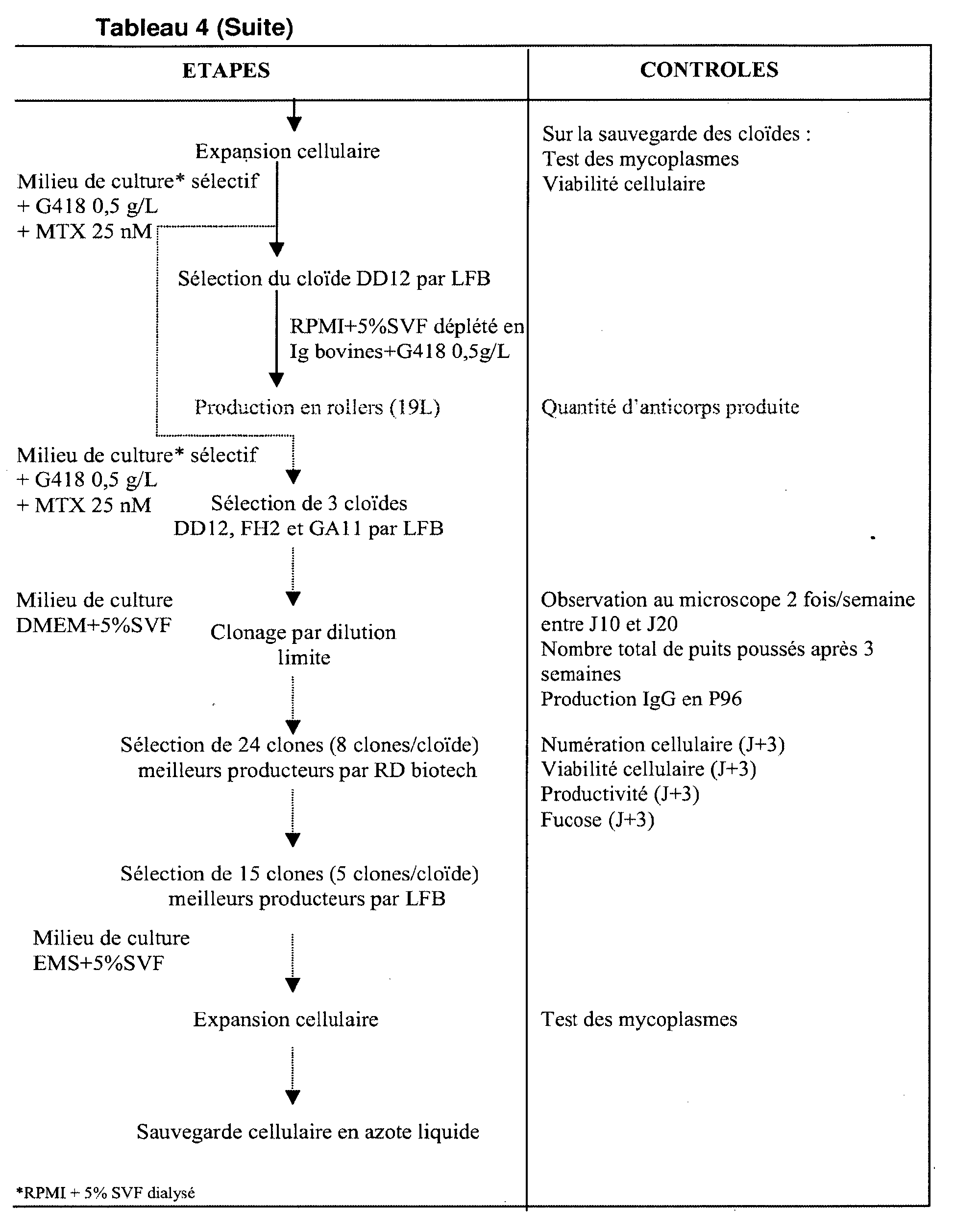

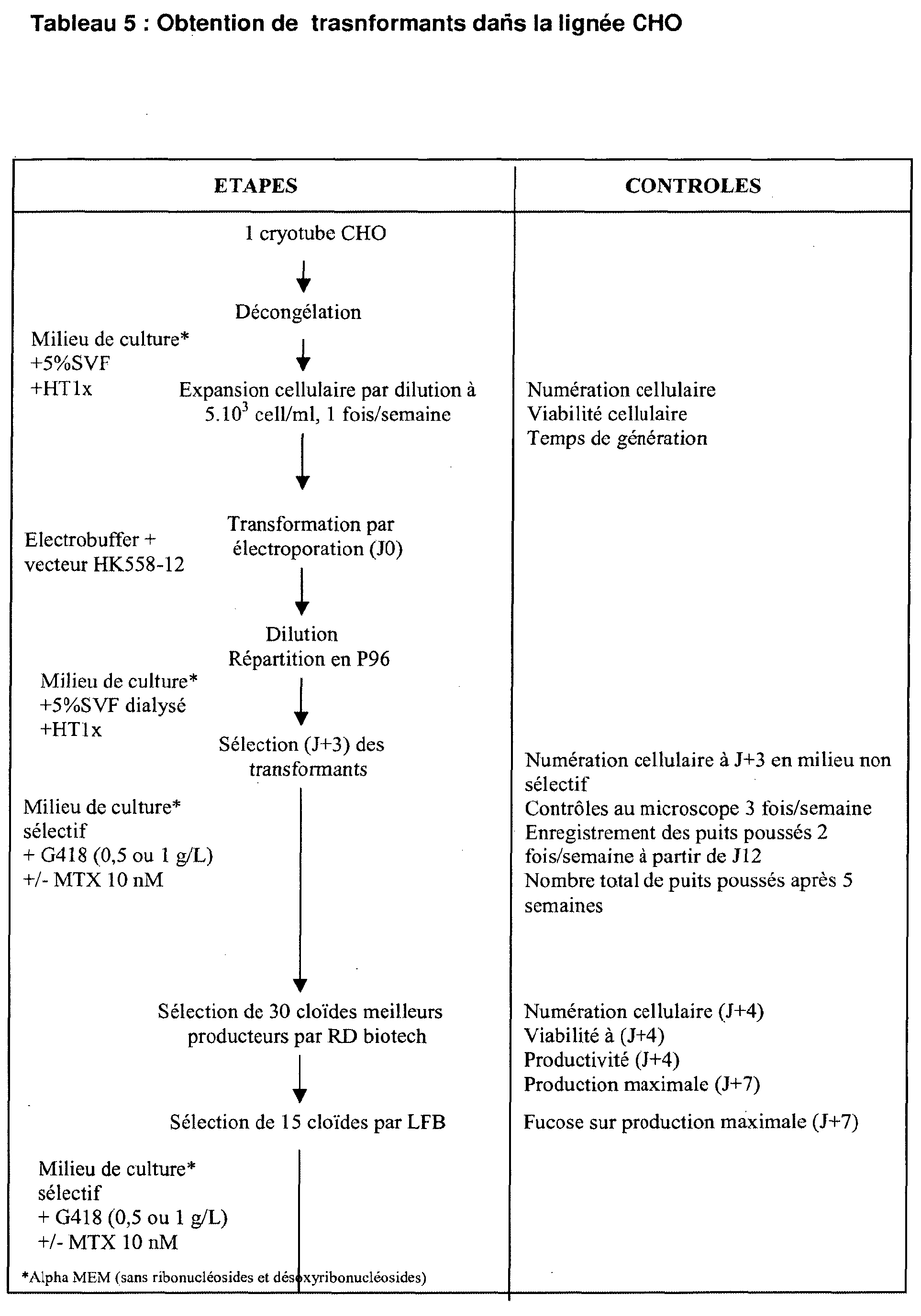

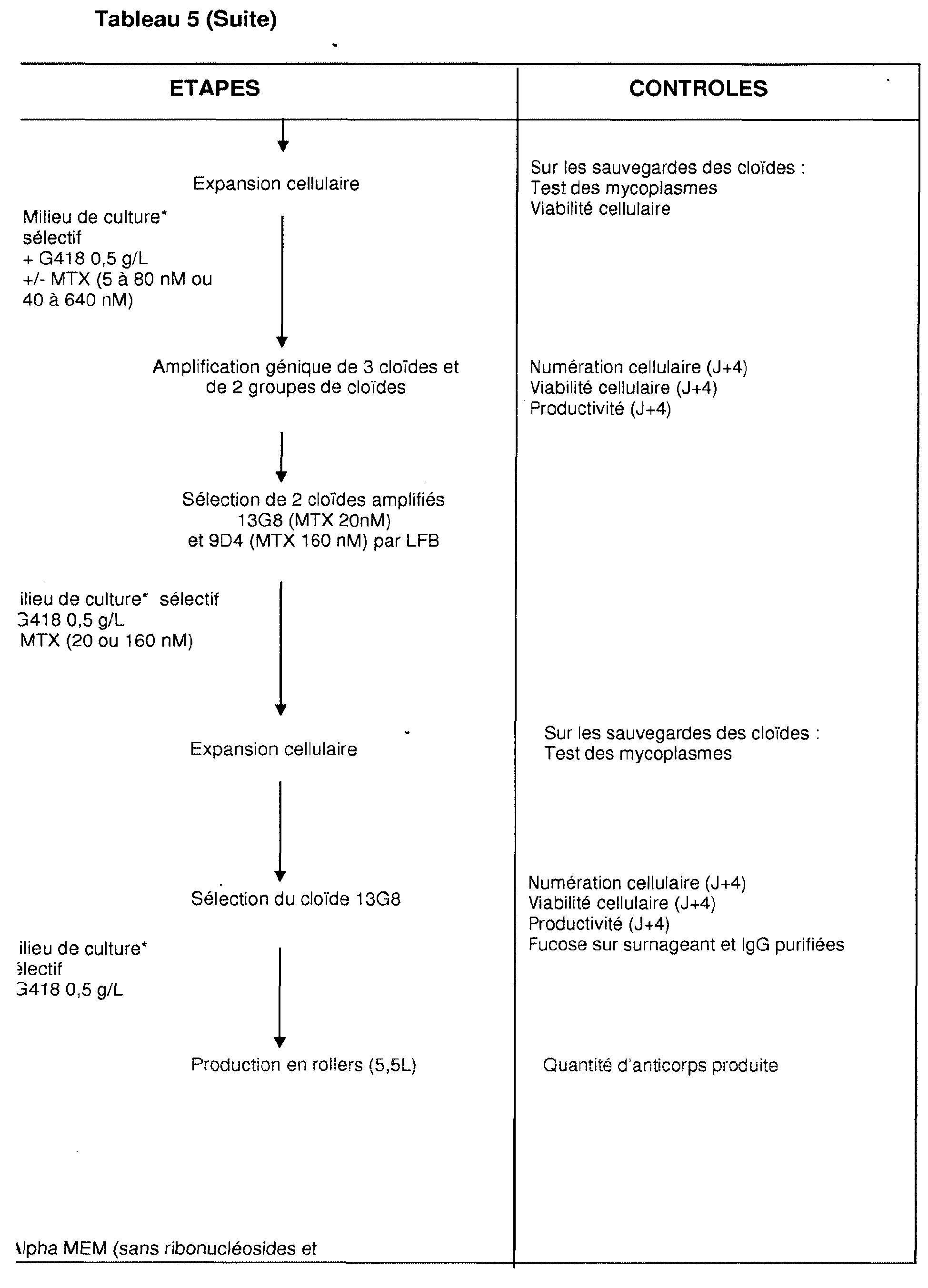

**Tableau 6 : Liste des séquences**

| Séquence | Région | Type |
|---|---|---|
| 1 | région variable de chaîne légère | peptide |
| 2 | région variable de chaîne lourde MUTE AFFINITE (31A, 66, 73) | peptide |
| 3 | région constante de la chaîne légère | peptide |
| 4 | région constante de la chaîne lourde | peptide |
| 5 | chaînes légères de l'IqG | peptide |
| 6 | chaînes lourdes de l'IgG MUTE AFFINITE (31A, 66, 73) | peptide |
| 7 | région variable de chacune des chaînes légères de l'IqG | Acide nucléique |
| 8 | région variable de chacune des chaînes lourdes MUTE AFFINITE (31A, 66, 73) | Acide nucléique |
| 9 | région constante de chacune des chaînes lourdes de l'IgG | Acide nucléique |
| 10 | région constante de chacune de ses chaînes légères | Acide nucléique |
| 11 | chaînes légères de l'anticorps | Acide nucléique |
| 12 | chaînes lourdes MUTE AFFINITE (31A, 66, 73) | A nucléique |
| 13-37 | amorces | A nucléique |

### ANNEXE allant des pages 52 à 80

### Contenu de la demande de brevet français n° FR 07/06744 déposée le 26 septembre 2007 pour « Anticorps contre les toxines du charbon »

### (Avec listage de séquences et sans les figures)

### Annexe

### ANTICORPS CONTRE LES TOXINES DU CHARBON

La présente invention concerne des anticorps contre les toxines du charbon. En particulier, la présente invention a pour objet un anticorps anti PA, dirigé contre la sous-unité PA des toxines létale et oedémateuse du charbon, modifié de façon à présenter une affinité et une tolérance améliorées.

Le charbon est une maladie causée par un bacille Gram-positif, Bacillus anthracis. Cette bactérie est non mobile et forme des spores lorsqu'elle est placée dans un environnement défavorable à sa survie. Les spores peuvent survivre 24 heures dans l'air et environ 100 ans dans le sol, et possèdent des propriétés de résistance à la chaleur ou aux agents désinfectants.

Une infection par le charbon peut prendre trois formes : cutanée, pulmonaire ou digestive. L'infection pulmonaire est la plus fréquemment mortelle. En cas d'inhalation, les spores de *B. anthracis* passent dans les alvéoles où elles sont phagocytées par les macrophages et les cellules dendritiques, notamment. Les spores germent dans ces cellules et les formes végétatives se multiplient dans les ganglions. Les bactéries passent alors dans le sang, se reproduisent en continu et produisent des toxines, responsables en partie de la létalité de la maladie. Les toxines du charbon sont composées de trois protéines distinctes : l'antigène protecteur (PA, 83 kDa avant clivage enzymatique intracellulaire et 63 kDa après clivage), le facteur létal (LF, 90 kDa) et le facteur oedémateux (EF, 89 kDa). La toxine létale est formée de pA et LF; et la toxine oedèmateuse, dont le rôle dans la physiologie de la maladie est moindre, de PA et EF. Ces protéines sont sécrétées par la bactérie en tant que monomères non toxiques, et s'assemblent à la surface des cellules cibles pour former des complexes toxiques.

Jusqu'à présent, plusieurs antibiotiques, tels que la pénicilline, la doxycycline et les fluoroquinones, ont été utilisés pour le traitement des infections par le charbon. Cependant, certains de ces antibiotiques peuvent ne pas avoir d'effets sur certaines souches, résistantes aux antibiotiques. En particulier, certains de ces traitements pourraient ne pas être utilisables en cas de terrorisme ou guerre bactériologique, lorsque des souches résistantes aux antibiotiques pourraient être volontairement disséminées.

De plus, comme les antibiotiques ne peuvent pas inhiber l'action de la toxine du charbon, il est nécessaire que ces antibiotiques soient adminishés à des stades précoces de l'infection, or les diagnostics précoces sont difficiles à établir car les symptômes initiaux sont non spécifiques.

Des vaccins, dont le composant majeur est l'antigène protecteur PA, ont été développés mais ne sont utilisés que pour des personnes très fortement susceptibles d'être en contact avec *B. anthracis.* De plus, du fait de la nécessité d'une période de plusieurs mois pour acquérir une immunité suffisante, ces vaccins ne peuvent pas être utilisés dans des situations d'urgence. En France actuellement, aucun des ces vaccins n'est actuellement agréé pour l'utilisation humaine. Il est donc nécessaire de développer de nouvelles approches thérapeutiques et préventives, autres que les antibiotiques.

L'immunisation passive avec des anticorps représente une stratégie efficace pour neutraliser la toxine. Plusieurs essais ont été réalisés pour neutraliser la toxine létale du charbon à l'aide d'anticorps monoclonaux contre l'antigène protecteur (PA) et le facteur létal (LF). La neutralisation de la toxine létale du charbon à l'aide d'un anticorps peut avoir lieu par inhibition de la liaison de PA et de-son récepteur cellulaire, inhibition du clivage de PA, inhibition de la liaison entre PA et LF ou encore inhibition de l'action de LF par exemple.

Le développement de nouveaux anticorps permettant de neutraliser la toxine du charbon est ainsi d'un intérêt général pour une prévention et un traitement efficace du charbon. Dans un travail récent, les inventeurs ont immunisé un macaque avec l'antigène protecteur PA83 pour obtenir des anticorps destinés à traiter l'infection humaine par le charbon. A partir de la moelle osseuse, les inventeurs ont amplifié les gènes codant des fragments d'anticorps spécifiques de PA83 et les ont clonés pour obtenir une librairie.

Un fragment de forte affinité (Kd:3,4 nM) et fortement neutralisant (concentration d'inhibition à 50%: 5,6 +I- 0,13 nM), désigné sous le nom de 35pAg3, a ensuite été isolé (Laffly et al., antimicrobial agents and chemotherapy, zoo5, 4g(g): 3414-3420). Les inventeurs ont démontré que le fragment d'immunoglobuline 35P483 neutralise la toxine du charbon en empêchant l'interaction de PA avec son récepteur cellulaire.

Les inventeurs ont ensuite recherché à modifier ce fragment d'immunoglobuline pour en améliorer les qualités dans le but d'une utilisation médicale prophylactique ou thérapeutique), en améliorant son affinité et sa tolérance par l'homme. Améliorer l'affinité de ce fragment d'immunoglobuline présente l'avantage d'utiliser une quantité plus faible d'immunoglobuline pour obtenir une activité biologique suffisante et permet aussi de réduire le coût du traitement. Améliorer la tolérance chez l'homme présente l'avantage d'éviter des réponses immunitaires dirigées contre ce fragment d'anticorps. La présente invention a donc pour objet de fournir un anticorps anti PA modifié à partir du fragment d'immunoglobuline 35P483.

La présente invention a aussi pour objet une composition comprenant ledit anticorps modifié ainsi qu'une composition pharmaceutique comprenant ledit anticorps modifié. La présente invention a également pour objet l'utilisation dudit anticorps modifié pour la préparation d'une composition pharmaceutique destinée au traitement ou à la prévention d'une infection par le charbon.

La présente invention concerne également un kit pour la détection d'une toxine du charbon, comprenant ledit anticorps modifié ainsi qu'une méthode de détection d'une toxine du charbon.

La présente invention sera mieux comprise à l'aide des définitions suivantes.

Le terme "anticorps" se réfère à une molécule d'immunoglobuline ou un fragment d'une molécule d'immunoglobuline ayant la capacité de se lier spécifiquement à un antigène particulier. Des fragments d'immunoglobuline bien connus sont par exemple les fragments F(ab')2, Fab, Fv, scFv et Fd.

Le terme "charbon" se réfère à toute maladie causée, directement ou indirectement, par une infection par *Bacillus anthracis.* Les symptômes initiaux d'une infection par inhalation ressemblent à ceux d'un rhume (fièvre, douleurs musculaires..). Après plusieurs jours, ces symptômes progressent vers des problèmes sévères de détresse respiratoire et de choc septique. L'inhalation du charbon est en générale fatale.

L'infection cutanée par le charbon a lieu lorsque la bactérie entre dans la peau au niveau d'une brèche cutanée préexistante. Cette infection donne lieu dans un premier temps à une papule, qui se développe en deux-trois jours en une vésicule puis en un ulcère de 1- 3 centimètres de diamètre présentant une aire nécrotique au centre. L'infection gastrointestinale par le charbon se développe suite à la consommation de viande contaminée et est caractérisée par une inflammation aigue du tractus intestinal.

Le terme "isolé" signifie "amplifié in vitro par PCR", "produit de façon recombinante par clonage", "purifié par séparation sur gel ou par clivage", ou encore "synthétisé par exemple par synthèse chimique".

Le terme "vecteur" se réfère à un acide nucléique dans lequel la séquence d'intérêt peut être insérée par restriction puis ligation pour le transport entre différents environnements génétiques ou pour l'expression dans une cellule hôte. Les vecteurs sont par exemples les plasmides, les cosmides, les chromosomes artificiels de levures (YAC), les chromosomes artificiels de bactéries (BAC) et les chromosomes artificiels dérivés du bactériophage P1 (PAC), les vecteurs dérivés de virus. Un vecteur de clonage est un vecteur capable de se répliquer dans une cellule hôte et qui est de plus caractérisé par la présence de un ou plusieurs sites de restrictions par les endonucléases. Un vecteur d'expression est un vecteur dans lequel la séquence d'ADN d'intérêt peut être insérée par restriction ou ligation de telle façon qu'elle puisse être répliquée et/ou transcrite en ARN. Les vecteurs peuvent contenir en outre un ou plusieurs marqueurs de sélection ou d'identification des cellules ayant été transformées ou transfectées avec le vecteur.

Le terme "anticorps humanisé" se réfère à des anticorps d'origine animale dans lesquels des composants humains ont été substitués à certains composants originels.

Le terme "prévention d'une maladie" correspond à la prévention de l'apparition de cette maladie chez un sujet, en particulier un humain, chez qui la maladie ne s'est pas encore déclarée.

Le terme "traitement d'une maladie" correspond à l'inhibition de cette maladie, i.e. l'arrêt de son développement, sa régression, ou à la disparition des symptômes et conséquences de la maladie, ou encore à la disparition des causes de la maladie.

Le terme "quantité thérapeutique effective" se réfere à la quantité qui est suffisante pour effectuer le traitement lorsqu'elle est administrée à un sujet qui nécessite un tel traitement. La quantité thérapeutique effective dépend du sujet, du stade de la maladie à traiter et du mode d'administration, et peut être déterminée par des opérations de routine par l'homme du métier.

Comme il est bien connu, seule une partie de l'anticorps, la région variable, est impliquée dans la liaison de l'anticorps à son épitope. Les régions constantes de l'anticorps activent les effecteurs immunitaires, phagocytes ou cellules tueuses, et le complément ; ces régions constantes ne sont pas impliquées dans la liaison à l'antigène. Un anticorps dont la région constante (Fc) a été enzpatiquement clivée de façon à en préserver la région charnière est désigné comme un fragment F(ab')2 et conserve les deux sites de liaison à l'antigène.

De même, un anticorps dont la région constante, y compris la région charnière a été enzymatiquement clivée, ou qui a été produit sans cette région, est désigné comme un fragment Fab et conserve un des deux sites de liaison à l'antigène. Les fragments Fab consistent en une chaîne légère qui est liée de façon covalente à une portion de la chaîne lourde appelée Fd.

Dans la région variable, on trouve les régions déterminant la complémentarité (CDRs, complementary determining régions), aussi appelées régions hypervariables, qui interagissent directement avec l'antigène. Modifier les CDRs peut donc permettre de modifier l'affinité d'un anticorps. Dans la région variable, on trouve un second type de régions, appelées régions charpentes (FRs, frameworlc), qui maintiennent la structure tertiaire des CDRs. Ces régions charpentes sont assez spécifiques de l'espèce où a été produit l'anticorps. Dans le fragment Fd de la chaîne lourde et dans la chaîne légère, on trouve quatre régions charpentes (FRI à 4) séparées respectivement par trois CDR (CDRI à 3).

La présente invention a pour objet un anticorps anti PA dont la région variable de la chaîne lourde a une séquence d'acides aminés représentée par SEQ ID N°1 et la région variable de la chaîne légère a une séquence d'acides aminés représentée par SEQ ID N°2, caractérisé en ce que ledit anticorps est modifié par au moins une mutation dans la région variable de la chaîne lourde ou dans la région variable de la chaîne légère pour présenter une affinité supéneure à celle de l'anticorps non modifié.

La séquence peptidique SEQ ID N°3, correspondant au fragment Fd de l'anticorps 35PA83, décrit dans Laffly et al., est accessible dans les banques informatisées, comme Genbank, sous le numéro CAH17920 et la séquence peptidique SEQ ID N°4, correspondant à la chaîne légère de l'anticorps 35PA83 est accessible de la même manière sous le numéro CAH17921.

Les régions variables de ces séquences sont présentées sous forme de schéma bidimensionnel sur la figure 1.

Les séquences d'ADN codant le fragment Fd et la chaîne légère de l'anticorps 35PA83, appelées ici SEQ ID N°5 et SEQ ID N°6, sont également accessibles dans les banques informatisées, sous les numéros AJ810486 et AJ810487, respectivement.

L'affinité K_{D} d'un anticorps peut être mesurée par les techniques conventionnelles corifiues de l'homme du métier.

L'anticorps non modifié (i.e. non muté) parental 35P483 présente une affinité K_{D} égale à 3,4 10⁻⁹. Cette constante d'affinitê a été calculé à partir des constantes d'association et de dissociation mesurées en temps réel par résonance plasmonique de surface tel qu'expliqué dans les exemples.

Dans un mode de réalisation de la présente invention, l'anticorps anti PA modifié selon l'invention est modifié par au moins une mutation choisie parmi G/S (31A), R/K (66), K/R (73), D/G (28), G/E (31A), H/L (55), S/G (74),Y/T (113), S/L (117) dans la région variable de la chaîne lourde, et Q/R (68), Q/R (27), AP (114), Q/L (68), P/S (115), H/R (24), S/E (69), S/R (58) dans la région variable de la chaîne légère.

La mutation G/S (31A) dans la région variable de la chaîne lourde signifie que l'acide aminé G en position 3IA (voir figure 1 pour les positions des acides aminés) a été substitué par l'acide aminé S.

Cet anticorps anti PA modifié par au moins une des mutations citées ci-dessus dans la région variable de la chaîne lourde ou dans la région variable de la chaîne légère présente une affinité pour PA améliorée par rapport à celle de l'anticorps 35PA83.

La présente invention fournit donc des fragment F(ab')2, Fab, Fv, scFv et Fd d'un anticorps anti PA modifié selon l'invention, ainsi que des anticorps chimériques dans lesquels la partie Fc de l'anticorps provient de séquences homologues humaines ou non humaines.

Selon un mode de l'invention, la partie Fc de l'anticorps peut être choisie de façon à produire des IgA, des IgM ou des IgG.

Selon un autre mode de l'invention, la partie Fc de l'anticorps peut être une partie Fc provenant de souris, chevaux, ovins, bovins ou autres mammifères.

Selon un mode préféré de réalisation de l'invention, l'anticorps anti PA modifié selon l'invention possède une partie Fc d'origine humaine. De tels anticorps entiers sont préférés pour l'administration chez l'homme car ils présentent une demi-vie plus longue que des fragments d'anticorps tels que les Fab, et sont plus adaptés à une administration intraveineuse, intra-péritonéale, intramusculaire, sous-cutanée ou transdermale.

Dans certains modes de réalisation de l'invention, les fragments Fab sont préférés pour les raisons suivantes : a) comme les fragments Fab ne possèdent qu'un seul site de liaison à l'antigène, les complexes immuns de grande taille ne peuvent pas se former, b) l'absence de région Fc empêche l'apparition d'une réaction inflammatoire activée par le Fc, telle que l'activation de la cascade du complément, c) la pénétration dans les tissus d'une petite molécule Fab est plus facile, et d) la production de Fabs est facilement réalisée et à faible coût dans des bactéries telles que E. coli.

Ainsi, un objet de la présente invention est de fournir des Fabs de l'anticorps anti PA modifié selon l'invention, des fragments de cet anticorps plus petits ou plus grands que des fragments Fabs ou encore des peptides de liaison à l'épitope, et en particulier des peptides dérivés des régions hypervanables de l'anticorps anti PA modifié selon l'invention.

Dans un premier mode de réalisation de l'invention, l'anticorps anti PA modifié selon l'invention est caractérisé en ce que ladite au moins une mutation est choisie parmi G/S (31A), R/K (66), K/R (73), D/G (28), G/E (31A), H/L (55), S/G (74),Y/T (113), S/L (117) dans la région variable de la chaîne lourde.

Dans un second mode de réalisation de l'invention, l'anticorps anti PA modifié selon l'invention est caractérisé en ce que ladite au moins une mutation est choisie parmi Q/R (68), Q/R (27), A/P (114), Q/L (68), P/S (115), H/R (24), S/E (69), S/R (58) dans la région variable de la chaîne légère.

Dans un troisième mode de réalisation de l'invention, l'anticorps anti PA modifié selon l'invention est caractérisé en ce qu'il est modifié par au moins une mutation choisie parmi G/S (31A), R/K (66), K/R (73), D/G (28), G/E (31A), H/L (55), S/G (74),Y/T (113), S/L (117) dans la région variable de la chaine lourde et au moins une mutation choisie parmi Q/R (68), Q/R (27), A/P (114), Q/L (68), P/S (115), H/R(24), S/E (69), S/R (58) dans la région variable de la chaîne légère.

Selon un mode préféré de l'invention, ledit anticorps anti PA modifié selon l'invention comprend les trois mutations suivantes dans la région variable de la chaîne lourde:
- G/S (31A)
- R/K (66)
- K/R (73).

Selon un autre mode préféré de l'invention, ledit anticorps anti PA modifié selon l'invention comprend les deux mutations suivantes dans la région variable de la chaîne lourde:
- H/L (55)
- S/G (74),
et la mutation Q/L (68) dans la région variable de la chaîne légère.

Selon un autre mode prêféré de l'invention, ledit anticorps anti PA modifié selon l'invention comprend la mutation suivante dans la région variable de la chaîne lourde:
- S/L (117)
et la mutation S/R (58) dans la région variable de la chaîne légère.

Selon un autre mode préféré de l'invention, ledit anticorps anti PA modifié selon l'invention comprend les deux mutations suivantes dans la région variable de la chaîne légère :
- H/R (24) et
- S/E (69).

Un objet de la présente invention est également de fournir un anticorps anti PA modifié présentant une affinité améliorée par rapport à l'anticorps non muté 35P483 ainsi qu'une meilleure tolérance par le système immunitaire humain. Un tel anticorps humanisé présente l'avantage de ne pas ou de moins induire de réponse immune contre lui-même, et d'avoir une plus longue demi-vie.

La présente invention a donc pour objet un anticorps anti PA modifié selon l'invention et humanisé, caractérisé en ce qu'il comprend en outre au moins une mutation dans la région variable de la chaîne lourde sélectionnée dans le groupe constitué par:
- aucun/Q (I)
- aucun/V (2)
- aucun/Q (3)
- aucun/L (4)
- aucun/Q (5)
- aucun/E (6)
- L/V (12)
- A/T (24)
- S/P (45)
- R/N (66)
- K/V (80)
- L/F (87)
- R/S (92)
- A/T (122)
- V/L (123).

La mutation aucun/Q (1) signifie qu'un acide aminé Q est ajouté en position 1 (voir la figure 1 pour la position des acides aminés).

Dans un autre mode de réalisation de l'invention, l'anticorps anti PA modifié selon l'invention et humanisé, comprend en outre au moins une mutation dans la région variable de la chaîne légère sélectionnée dans le groupe constitué par:
- aucun/A (1)
- aucun/I (2)
- aucun/Q (3)
- aucun/L (4)
- Y/S (14)
- K/R (18)
- H/R (24)
- Y/F (87)
- S/P (96)
- S/T (101)
- UV (124).

Dans un mode de réalisation préféré de l'invention, l'anticorps anti PA modifié selon l'invention et humanisé, comprend en outre au moins une mutation dans la région variable de la chaîne lourde tel que décrite ci-dessus et au moins une mutation dans la région variable de la chaîne légère telle que décrite ci-dessus,

Préférablement, l'anticorps anti PA modifié selon l'invention et humanisé comprend en outre les mutations suivantes dans la région variable de la chaîne lourde:
- aucun/Q (1)
- aucun/V (2)
- aucun/Q (3)
- aucun/L (4)
- aucun/Q (5)
- aucur/E (6)
- A/T (122)
- V/L (123),
et les mutations suivantes dans la région variable de la chaîne légère :
- aucun/A (I)
- aucun/I (2)
- aucun/Q (3)
- aucun/L (4)
- UV (124).

D'après la description ci-dessus des séquences d'acides aminés de la région variable de la chaîne lourde et de la région variable de la chaîne légère des anticorps anti PA modifiées selon l'invention, l'homme du métier est capable de synthétiser, ou de faire synthétiser, des acides nucléiques qui codent ces séquences d'acides aminés.

La présente invention a donc pour objet un acide nucléique codant un anticorps anti PA modifié selon l'invention, qui présente une affinité améliorée par rapport à l'anticorps 35PA83 non muté, et qui est ou non humanisé.

La présente invention a également pour objet un vecteur comprenant ledit acide nucléique.

Ces acides nucléiques pourront être compris dans un vecteur recombinant pour le clonage ou pour l'expression des anticorps de l'invention.

La présente invention inclut tous les vecteurs recombinants contenant des séquences codantes pour la transformation, transfection ou thérapie génique eucaryote ou procaryote. De tels vecteurs pourront être préparés selon les techniques conventionnelles de biologie moléculaire et comprendront en outre un promoteur approprié, optionnellement une séquence signal pour l'export ou la sécrétion, et des séquences régulatrices nécessaires pour la transcription de la séquence nucléotidique.

Un polypeptide de fusion peut être utile pour la purification des anticorps de la présente invention. Le domaine de fusion peut par exemple inclure une queue poly-histidine qui permet la purification sur des colonnes Ni⁺, ou une ancre membranaire de phage filamenteux qui est particulièrement utile pour le screening de banque, selon la technologie du "phage display".

Un des vecteurs approprié dans le cadre de l'invention est une molécule d'ADN recombinante adaptée pour recevoir et exprimer une première et une seconde séquence d'ADN, de façon à permettre l'expression d'anticorps hétérodimérique tel qu'un anticorps de longueur complète ou des fragments F(ab')Z ou Fab selon l'invention. Un tel vecteur fournit un système pour indépendamment cloner les deux séquences d'ADN dans deux cassettes séparées présentes dans le vecteur, de façon à former deux cistrons séparés pour l'expression d'un premier et d'un second polypeptide de l'anticorps hétérodimérique. Un tel vecteur d'expression est appelé vecteur di-cistronique.

Les anticorps modifiés de la présente invention peuvent être produits dans des cellules eucaryotes telles que des CHO ou des hybridomes humain ou murin par exemple, ainsi que dans des cellules végétales.

Un autre objet de la présente description est de fournir une composition comprenant au moins un anticorps anti PA modifié selon l'invention pour en améliorer l'affinité, et optionnellement humanisé.

La présente description a également pour objet une composition pharmaceutique comprenant au moins un anticorps anti PA modifié selon l'invention pour en améliorer l'affinité, et optionnellement humanisé.

Ladite composition pharmaceutique comprend de façon préférée un véhicule pharmaceutiquement acceptable. Un tel véhicule correspond au sens de l'invention à un matériel non toxique qui n'interfère pas avec l'efficacité de l'activité biologique des ingrédients actifs de la composition. Le terme "pharmaceutiquement acceptable" réfère à un matériel non toxique qui est compatible avec un système biologique tel qu'une cellule, une culture cellulaire, un tissu ou un organisme. Les caractéristiques du véhicule dépendront du mode d'administration.

La présente description concerne l'utilisation d'au moins un anticorps anti-pA modifié pour en améliorer l'affinité, et optionnellement humanisé, selon l'invention pour la préparation d'une composition pharmaceutique ou d'un médicament destiné au traitement ou à la prévention d'une infection par *Bacillus anthracis.*

L'anticorps anti PA modifié pour en améliorer l'affinité, et optionnellement humanisé, selon l'invention peut être marqué. Des exemples de marqueurs comprennent les enzymes, les radio-isotopes, les composés fluorescents, les métaux colloïdes, les composés chimioluminescents, et les composés bioluminiscents. Les méthodes de liaison d'un marqueur à un anticorps sont bien connues de l'homme du métier.

Une autre technique de marquage consiste à coupler l'anticorps à des haptènes de faibles poids moléculaire, ces haptènes pouvant être spécifiquement modifiés au moyen d'une seconde réaction. Des exemples d'haptènes sont la biotine, qui réagit avec l'avidine, ou le dinihophenol, le pyridoxal ou la fluorescéine, qui peuvent réagir avec des anticorps spécifiques anti-haptènes.

Un objet de la présente description est de fournir un kit pour la détection d'une toxine du charbon comprenant PA. Ce kit comprend :
- un container comprenant au moins un anticorps anti PA modifié selon l'invention pour en améliorer l'affinité, et optionnellement humanisé, et qui peur être ou non marqué,
- optionnellement, un container comprenant des solutions tampons
- et optionnellement un container comprenant des moyens de détection dudit anticorps anti PA modifié marqué, tel qu'une protéine de liaison à la biotine, par exemple l'avidine ou la streptavidine, liée à une molécule rapporteur, telle qu'un marqueur fluorescent ou enzymatique. Ce container peut aussi comprendre des moyens de détection dudit anticorps anti PA modifié non marqué, soit essentiellement des anticorps ou fragments d'anticorps.

L'anticorps anti PA modifié pour en améliorer l'affinité, et optiomellement humanisé, de l'invention peut être utilisé in vitro, par exemple dans des tests immunologiques dans lesquels ils sont utilisés en phase liquide ou liés à un véhicule de phase solide. Des exemples de véhicules bien connus sont le verre, le polystyrène, le polypropylène, le polyéthylène, le dextran, le nylon, l'amylase, la cellulose naturelle ou modifiée, le polyacrylamide, l'agarose ou la magnétite. Des exemples de tests immunologiques utilisant l'anticorps anti PA de l'invention sont des radioimmunoessais, des marquages histoimmunologiques, des ELISA, des western-blots, des essais d'immunoprécipitation, des essais d'immuno-diffusion, des essais de fixation du complément, des analyses au FACS ou encore des analyses par puces à protéines.

La présente description a pour objet de fournir une méthode de détection *in vitro* d'une toxine du charbon, comprenant PA, dans un échantillon biologique, comprenant :
- la mise en contact de l'échantillon avec au moins un anticorps anti PA modifié selon l'invention pour en améliorer l'affrnité, et optionnellement humanisé, et
- la détection de la liaison dudit anticorps comme indicateur de la présence de ladite toxine du charbon.

L'échantillon biologique peut être liquide : par exemple de la salive, de l'urine, du fluide cérébrospinal, du sérum ou du sang, ou solide ou semi-solide, par exemple des tissus ou des matières fécales ou un tissu solide tel qu'utilisé couramment en diagnostic histologique.

La présente description a également pour objet de fournir une méthode de détection *in vivo* d'une toxine du charbon comprenant PA, dans laquelle un anticorps anti PA modifié selon l'invention pour en améliorer l'affinité, optionnellement humanisé, et marqué est adminishé à un sujet. La quantité d'anticorps modifié marqué adminishée doit être suffisante pour permettre la détection de la liaison de l'anticorps à la toxine. La quantité d'anticorps modifié marqué administrée dépendra des facteurs tels que l'âge et le sexe du sujet, ainsi que du stade de la maladie. La quantité administrée peut varier entre 0.01 mg/kg et 50 mg/kg, préférablement entre 0.1 mg/kg et 20 mg/kg, et plus préférablement entre 0.1 mg/kg et 2 mg/Kg.

Pour effectuer le diagnostic *in vivo*, l'anticorps anti PA modifié de la description doit être lié à un radioisotope directement ou indirectement par l'intermédiaire de groupes fonctionnels. Des groupes fonctionnels couramment utilisés sont par exemple l'acide diéthylène-triamine-pentacétique (DTPA) et l'acide éthylène-diamine-tétraacétique (EDTA). Des exemples d'ions métalliques radioisotopes sont ¹¹¹In, ⁹⁷Ru, ⁶⁷Ga, ⁶⁸Ga, ⁷²As, ⁸⁹Zr et ²⁰¹Tl.

Les anticorps anti PA modifiés de la description peuvent aussi être marqués avec un isotope paramagnétique pour le diagnostic par imagerie de résonance magnétique (IRM) ou par résonance de spin électronique (ESR). Des radioisotopes gamma émefteurs de positrons peuvent également être utilisés, tels que ¹⁵⁷Gd, ⁵⁵Mn, ¹⁶²Dy, ⁶⁸Ga, ⁵²Cr, et ⁵⁶Fe.

Les anticorps anti PA modifiés pour en améliorer l'affinité, et optionnellement humanisés, de l'invention peuvent également être utilisés *in vitro* ou *in vivo* pour suivre l'évolution du traitement de la maladie, par exemple en déterminant l'augmentation ou la diminution du nombre de cellules ciblées par les toxines du charbon ou les changements dans la concentration de la toxine PA dans un échantillon biologique.

La présente description a pour objet une méthode de traitement d'un sujet, de préference un humain, susceptible d'être infecté par *Bacillus anthracis*, dans laquelle une quantité thérapeutiquement efficace d'un anticorps anti PA modifié selon l'invention pour en améliorer l'affinité, et optionnellement humanisé, est administré audit sujet.

Une quantité thérapeutiquement efficace correspond à une quantité suffisante pour diminuer les symptômes de la maladie et l'évolution de l'infection. Cette quantité peut varier avec l'âge, le sexe du sujet et le stade de la maladie et sera déterminée par l'homme du métier. Une quantité thérapeutiquement efficace peut varier entre 0.01 mg/kg et 50 mg/kg, préférablement entre 0.1 mg/kg et 20 mg/kg, et plus préférablement entre 0.1 m/kg et. 2 mg/kg, en une ou plusieurs administrations quotidiennes, pendant un ou plusieurs jours.

Le mode d'administration peut être par injection ou par perfusion gradueile. L'injection peut être intraveineuse, intra-péritonéale, intramusculaire, sous-cutanée ou transdermale.

Les préparations pour une administration parentérale peuvent inclure des solutions aqueuses ou non-aqueuses stériles, des suspensions ou des émulsions. Des exemples de solvents non-aqueux sont le propylène glycol, le polyéthylène glycol, des huiles végétales, telle que l'huile d'olive, ou des esters organiques injectables tels que l'éthyl oléate. Des véhicules aqueux comprennent l'eau, des solutions alcool/eau, des émulsions ou des suspensions.

La présente description a également pour objet un immunoconjugué comprenant un anticorps anti PA modifié selon l'invention pour en améliorer l'affinité, optionnellement humanisé, et lié, de façon directe ou indirecte, à un agent thérapeutique.

De tels agents thérapeutiques comprennent des agents chimiques, des radionucléides, des agents immunothérapeutiques, des cytokines, des chimiokines, des toxines ou des inhibiteurs d'enzyme. Des exemples de toxines sont la chaîne A de la diphtérie, la chaîne A de l'exotoxine, la chaîne A de la ricin, la chaîne A de l'abrine, la chaîne A de la modeccin, l'alpha-sarcin, les protéines Aleurites fordii, les protéines dianthines, les protéines Phytolaca americana, l'inhibiteur momordica charantia, la curcine, la crotine, l'inhibiteur sapaonaria officinalis, la gélonine, 1a mitogélline, la restrictocine, la phénomycine, l'énomycine et le tricothecenes. Des exemples de radionucléide sont ²¹²Bi, ¹³¹I, ¹³¹In, ⁹⁰Y, et ¹⁸⁶Re.

La présente description sera mieux comprise à l'aide du complément de description qui va suivre, qui se réfère à des exemples d'obtention d'anticorps anti PA.

Dans les exemples qui suivent, donnés à titre illustratif, il sera fait référence aux figures en annexe:
Figure 1 : schéma en collier de perles la région variable de la chaîne lourde et de la région variable de la chaîne légère de l'anticorps 35P483.
   La représentation IMGT en collier de perles est réalisée en accord avec la numérotation IMGT. Les points indiquent les différences entre les gènes humains les plus similaires à 35P483, et 35P483. Les cercles hachurés correspondent aux positions manquantes selon la numérotation IMGT.
Figure 2 : alignement de séquence entre 3 anticorps anti PA modifiés selon l'invention (6.20, V2 et J24-7) et l'anticorps parental 35PA83.

Le positionnement des CDRs suit la définition de l'IMGT (en gris clair) ou la définition de Kabat (en gris foncé, Wu and Kabat 1970). Les résidus sujets à mutation sont en gras. Tous les résidus sont numérotés selon la numérotation IMGT.

### Matériels et méthodes

### Souches E. coli

Les souches E. coli suivantes ont été utilisées :
- XLI (Stratagène, La jolla, CA):recA1, endAI, gyrA96 thi-1 hsdR17 sup E44 relA1 lac [F'proAB laclqZΔM15 Tn 10(Tetr)]
- SURE (Stratagène): el4(McrA) Δ(mcrCB-hsdSMR-mrr)171 endA1 supE44 thi-1 gyrA96 relA1 lac recB recJ sbcC umuC::Tn5 (Kanr) uvrC [F'proAB laclqZΔM15 Tn10 (Tetr)I
- HB2151, utilisées pour l'expression de Fabs solubles.

### Toxins

Les toxines du charbon (PA83, LF et EF) ont été achetées chez List laboratories.

### Construction de la bibliothèque de mutants 35P483

Une bibliothèque d'anticorps mutant dérivés du gène 35PA83 a été, générée par Massive mutagenesis@ (Biomethodes, Evry, France). Les mutations ont été introduites dans les CDRs des chaînes lourde et légère en utilisant des codons NNS. Les régions CDRs ont été définies selon Kabat et al. (Wu and Kabat 1970) et IMGT (Lefranc, Pommie et al. 2003).

La bibliothèque ADN a été utilisée pour transformer les cellules SURE par électroporation. Après ajout de milieu SB supplémenté en carbénicilline à la culture et incubation pendant 1h à 37°c, 1 ml de phage helper VCSM13 (environ 10¹² pfu) a été ajouté à la culture. Après incubation pendant 2h, 70 µg/ml de kanamycine ont été ajoutés et la culture a été mise sous agitation pendant la nuit à 37°C.

### Sélection d'anticorps par phage

Les particules de phages-Fab ont été purifiées et concentrées à partir de 50 ml de culture par précipitation au PEG, puis re-suspendues dans 3 ml de PBS-BSA 1%-azide 0,02% et filtrées sur un filtre de 0,45 µm. Le titre de cette préparation de phage était d'environ 5 10¹⁰ pfu/ml. Les phages-Fab ont été soumis à trois cycles d'infection-sélection-récupération tel que précédemment décrit (Andris-Widhopf, Rader et al. 2000). Cerrains phages ont été analysés, ou bien soumis à l'un ou bien à l'autre des processus décrits cidessous :

### Sélection par élution à très faible concentration d'antigène

La bibliothèque a été criblée en utilisant des concentrations décroissantes de PA83 biotinylé (100 nM, 10 nM, 1 nM, 0,1 nM, 0,01 nM, 0,001 nM) et des billes magnétiques recouvertes de streptavidine µMACS (Milteny Biotech). Différents mélanges de phages et d'antigène biotinylé, aux différentes concentrations, ont été incubés pendant 30 min à 37°C dans des tubes en verre. Les phages liés au PA83 biotinylé ont été capfurés en utilisant 100 µl de billes magnétiques recouvertes de streptavidine µMACS (5 min à tempérarure ambiante). Après capture des phages, les billes ont été lavées 5 fois dans du PBS et une fois dans du TPBS (PBS contenant 0.1 % de tween 20). Les phages liés ont ensuite été élués par incubation avec 100 µl de trypsine (10 µg/ml). L'éluat a été utilisé pour infecter les bactéries E. coli SURE en phase exponentielle de croissance.

### Sélection par incubation longue

Approximativement 10⁹-10¹⁰ phages par puits (50 µl) ont été criblés par liaison sur plaque (Nunc Maxisorp) recouverte avec 5 µg/ml de PA83 dans du PBS et bloquée avec 5% de MPBS. La plaque PA83 a été incubée avec les phages-Fab pendant 2h à 37°C et lavée 5 fois dans 0,1% Tween 20-PBS et 2 fois dans du PBS. Les phages ont ensuite été incubés à 4°C avec 50 µg/ml de PA83 soluble pendant des temps donnés (1 jour, 3, 13, 18, et 24 jours). Après 5 lavages, les phages liés ont été élués à 37°C pendant 15 min avec 50 µl de trypsine 10 µg/ml (Sigma) avant réinfection des bactéries E. coli SURE en phase exponentielle.

### Expression de Fab soluble, extraction périptasmique et purification

Chaque variant ADN a été transformé dans des bactéries de la souche d'*E. coli* appelée HB2151, rendues chimiquement compétentes. Les cellules ont été cultivées à 30°C, agitées à 250 rpm dans 1L de milieu SB contenant 50 µg/ml de carbénicilline et 0,1% de glucose. Lorsque la culture a atteint une A₆₀₀ de 1,5, une induction avec 1 mM d'IPTG a été effectuée pendant 18h à22°C.

Les Fabs ont été extraits avec du sulfate de polymixine B (Sigma) et purifiés sur colonne de nickel (Ni-NTA spin column, QIAGEN, Valencia, CA) selon les instructions du fabricant, puis dialysés avec du PBS 1X à 4°C pendant 3h.

### Quantification du Fab soluble

La pureté du Fab a été testée par SDS-PAGE et sa concentration a été déterminée à l'aide du logiciel Quantity One® (Biorad).

### Mesure en temps réel de la résonance plasmonique de surface (SPR)

Les constantes de cinétique de l'interaction entre PA83 et les variants 35PA83 ont été déterminées en utilisant le système Biacore X SPR (BIAcore, Uppsala, Sweden). Le PA83 a été immobilisé sur une puce sensible CM5 en utilisant une procédure de couplage des amines par injection de 30 µl de 2 µg/ml de PA83 dans 10 mM de sodium acétate pH 4,5. Pour minimiser la probabilité de reliaison, le K_{D} a été mesuré en utilisant un débit élevé (30 µl/min) et une quantité minimale d'antigène couplé (environ 500 RU, unités de résonance). Le taux de liaison de differentes concentrations de Fab allant de 5 à 400 nM dans du PBS a été déterminé à un débit de 30 µl/min. Les données de liaison ont été introduites dans un modèle langmuir 1 :1 du logiciel d'évaluation BIA. Les constantes d'association et de dissociation (kₒₙ et k_{off} respectivement) pour la liaison du Fab à PA83 ont été déterminées à 35°C.

### Analyse de séquences

Les séquences des chaînes lourde et légère des clones sélectionnés ont été déterminées par Genome Express (Meylan, France) en utilisant les amorces ompseq et newpelseq (Andris-Widhopf, Rader et al., 2000). Les séquences ont été analysées en ligne, en utilisant le système IMGT (http :/imgt. cines. fr).

### Résultats

### Construction de la bibliothèque mutante de Fab (35PA83)

Une maturation d'affinité in vitro a été réalisée pour générer des nouveaux variants présentant une meilleure affinité. Dans ce but, une bibliothèque de variants a été générée par mutation exclusive des résidus de 6 CDRs, c'est-à-dire au niveau de 73 positions. La taille de la bibliothèque est de 5,4 10⁸ transformants. 45 clones plasmidiques indépendants ont été séquencés pour déterminer la diversité (tableau 1) et le taux de mutation. Le taux de mutation expérimental est de 3 mutations par fragment (V_{H}+V_{L}), ce qui permet une sélection directe des combinaisons de mutations qui augmentent l'affinité à PA.

L'examen de la fréquence mutationnelle à chaque position CDR ciblée dans la bibliothèque non sélectionnée comparé à la bibliothèque sélectionnée (tableau 1) a montré que certaines positions (en grisé) ne sont pas tolérantes à la variation. Ainsi, les résidus se trouvant à ces positions semblent être des résidus clés pour la liaison à l'antigène, préservant l'intégrité du site de liaison à l'antigène. En particulier, les résidus (H31-H40) du CDR1 sont définis comme des résidus de contact à l'antigène.

Il semble qu'une pression de sélection substantielle ait eu lieu pendant le processus de sélection car la bibliothèque non sélectionnée présente une plus grande diversité en comparaison avec les séquences sélectionnées, en particulier dans L-CDR1 et H-CDR1.

Deux positions (en noir) sont fréquemment mutées dans la bibliothèque sélectionnée : H117 et L27. A l'exception du mutant J24-I5, les mutations du résidu sérine H117 n'ont pas affecté les propriétés de liaison à l'antigène des Fabs (tableau 2).En revanche, les mutations des résidus Glutamine L27 semblent défavorables car elles diminuent l'affinité du Fab (mutant 6.7) ou affectent l'efficacité de l'expression du Fab (données non montrées).

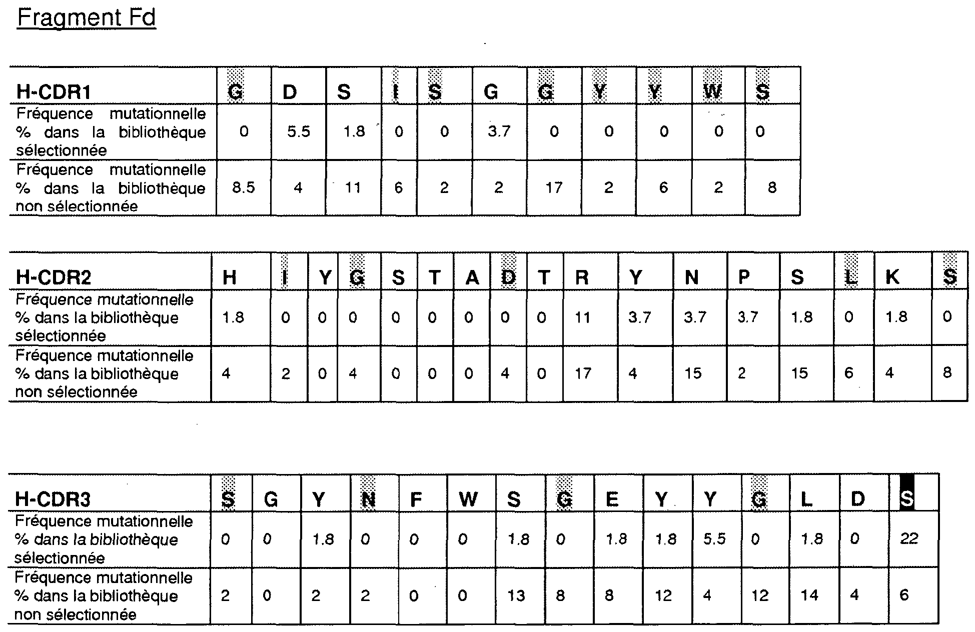

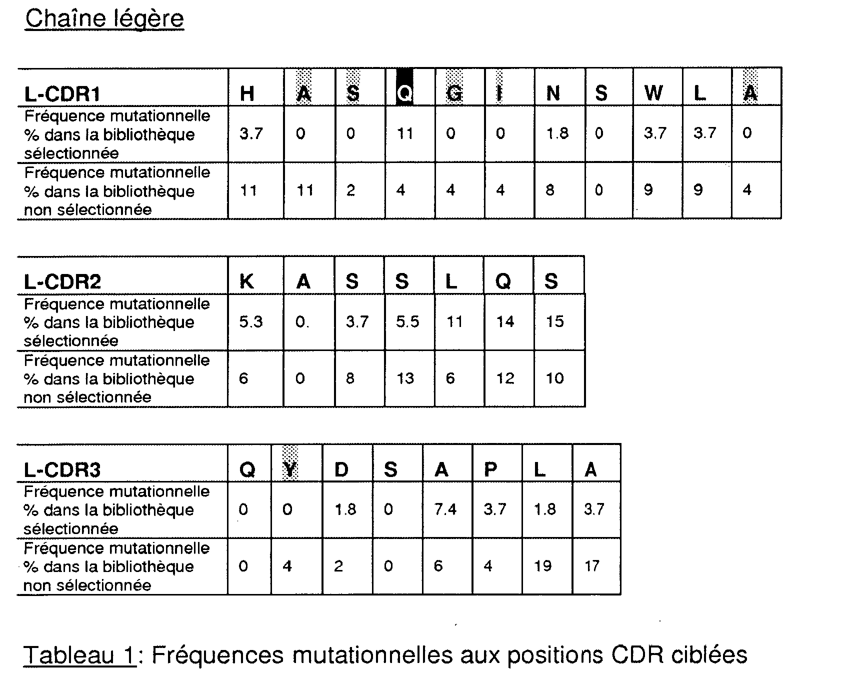

Pourcentage de séquences mutées à une position donnée (54 séquences individuelles)

**Tableau 2: Affinité et cinétiques de liaison pour le Fab 35PA83 et ses variants.**

| **Clone parental** | **Séquence H** | **Séquence L** | **K_{D} (M)** | **Kₒₙ (M⁻¹.s⁻¹)** | **K_{off} (s⁻¹)** |
|---|---|---|---|---|---|
| 35PA83 | parentale | parentale | 3,4 10⁻⁹ | 9,3 10⁴ | 3,2 10⁻⁵ |
| | | | | | |

| **Clones avec affinités nettement améliorées** | **Séquence H** | **Séquence L** | **K_{D} (M)** | **Kₒₙ (M⁻¹.s⁻¹)** | **K_{off} (s⁻¹)** |
|---|---|---|---|---|---|
| v2 | G>S (31A) | parentale | 6,6 10⁻¹⁰ | 1,22 10⁵ | 8,1 10⁻⁵ |
| | R>K (66) | | | | |
| | K>R (73) | | | | |
| 6.20 | H>L (55) | Q>L (68) | 1,8 10⁻¹⁰ | 2,86 10⁵ | 5,1 10⁻⁵ |
| | S>G (74) | | | | |
| J24-7 | parentale | H>R (24) | 7,8 10⁻¹⁰ | 4,35 10⁵ | 3,4 10⁻⁴ |
| | | S>E (69) | | | |
| J24-15 | S>L (117) | S>R (58) | 8,8 10⁻¹⁰ | 3,41 10⁵ | 3 10⁻⁴ |

| **Clones avec affinités dégradées** | **Séquence H** | **Séquence L** | **K_{D} (M)** | **Kₒₙ (M⁻¹.s⁻¹)** | **K_{off} (s⁻¹)** |
|---|---|---|---|---|---|
| v3 | parentale | L>R (67) | 2.27 10⁻⁸ | 8.98 10³ | 2 10⁻⁴ |
| 5.20 | parentale | S>R (69) | 1.22 10⁻⁸ | 2.4 10⁴ | 2.91 10⁻⁴ |
| 6.7 | parentale | Q>V (27) | 8.11 10⁻⁹ | 1.9 10⁴ | 1.5 10⁻⁴ |
| | | S>L (66) | | | |
| 6.40 | parentale | H>R (24) | 1.10⁻⁸ | 1.9 10⁴ | 2 10⁻⁴ |
| | | | | | |

| **Clones avec affinités stables ou peu améliorées** | **Séquence H** | **Séquence L** | **K_{D} (M)** | **Kₒₙ (M⁻¹.s⁻¹)** | **K_{off} (s⁻¹)** |
|---|---|---|---|---|---|
| v8 | L>F (115) | A>S (117) | 2.54 10⁻⁹ | 9.2 10⁴ | 2.3 10⁻⁴ |
| 3.5 | parentale | Q>R (27) | 1.9 10⁻⁹ | 0.8 10⁵ | 1.5 10⁻⁴ |
| | | A>P (114) | | | |
| 5.7 | S>R (29) | W>D (32) | 2.57 10⁻⁹ | 5.5 10⁴ | 1.4 10⁻⁴ |
| | S>R (117) | | | | |
| 5.15 | S>A (117) | Parentale | 3.5 10⁻⁹ | 8.85 10⁴ | 3.11 10⁴ |
| 5.47 | Y>T (112.4) | P>S (115) | 2.11 10⁻⁹ | 8.81 10⁴ | 1.86 10⁻⁴ |
| 5.25 | parentale | Q>r (68) | 2 10⁻⁹ | 4.5 10⁴ | 9 10⁻⁵ |
| 5.39 | G>E (31A) | Parentale | 1.71 10⁻⁹ | 0.63 10⁵ | 1.08 10⁻⁴ |
| | D>G (28) | | | | |
| 6.36 | Y>T (113) | P>S (115) | 2 10⁻⁹ | 0.62 10⁴ | 1.26 10⁻⁵ |
| 6.2 | S>G (74) | A>G (114) | 2 10⁻⁹ | 1.6 10⁵ | 3.2 10⁻⁴ |
| 6.46 | S>A (70) | S>R (69) | 1.82 10⁻⁹ | 6.9 10⁴ | 1.26 10⁴ |
| | E>Q (112.3) | | | | |
| | S>R (117) | | | | |
| 6.49 | P>G (69) | Parentale | 1.55 10⁻⁹ | 9.6 10⁴ | 1.5 10⁻⁴ |
| | S>T (111.2) | | | | |
| J24-3 | P>G (69) | Parentale | 1.5 10⁻⁹ | 2 10⁵ | 2.9 10⁻⁴ |
| J24-12 | parentale | A>D (114) | 3.5 10⁻⁹ | 7.8 10⁴ | 2.8 10⁻⁴ |
| J24-13 | parentale | D>E (108) | 1.5 10⁻⁹ | 1.2 10⁵ | 1.86 10⁻⁴ |
| J24-14 | D>G (28) | parentale | 3.3 10⁻⁹ | 6.14 10⁵ | 2.03 10⁻⁴ |
| | G>E (33) | | | | |
| | S>L (117) | | | | |

Les constantes d'association (Kₒₙ) et de dissociation (K_{off}) ont été déterminées par résonance plasmonique de surface (BIAcore) and K_{D} a été calculé comme égal au rapport K_{off/} Kₒₙ.

### Criblage des variants

La bibliothèque mutante a été soumise à 3 cycles (R1, R2 et R3) d'infection-sélection-récupération. Après l'étape R3, 12 clones individuels ont été analysés par séquençage de V_{H} et V_{L}. Parmi ces 8 variants, 2 ont été trouvés identiques. 7 Fabs individuels ont donc été exprimés en tant que Fabs solubles. 3 d'entre eux ont été exprimés de façon suffisante pour permettre la mesure de l'affinité par SPR.

| | | | | |
|---|---|---|---|---|
| 12 séquences H+L | 4 parentaux | 34% | | |
| | 8 variants (2 identiques) | 66% | 3 clones exprimés | 37% |
| | | | 5 clones non exprimés | 63% |

Le triple mutant V2 a montré une constante cle dissociation plus faible (K_{off} = 8,1 10⁵ s⁻¹) et une constante d'association un peu plus rapide (Kₒₙ = 1,22 10⁵ M⁻¹.s⁻¹) que 35PA83, résultant en l'augmentation de l'affinité de 5,15 fois. Ce mutant présente 3 mutations dans le domaine variable de la chaîne lourde: une mutation (G31_{A}S) dans H-CDR1 et deux mutations dans H-CDR2 (R66K, K73R).

Après le troisième cycle, les phages ont été criblés selon deux processus supplémentaires de sélection : panning dans des puits recouverts de l'antigène avec incubation longue ("sélection par incubation longue") ou bien en utilisant de l'antigène biotinylé soluble à très petite concentration ("sélection par élution à très faible concentration d'antigène soluble").

### Sélection par élution à très faible concentration d'antigène soluble

| Expérience | 1 | 2 | 3 | 4 | 5 | 6 | 7 | 8 | 9 |
|---|---|---|---|---|---|---|---|---|---|
| {PA83 biotinylé} nM | 100 | 10 | 1 | 0.1 | 0.01 | 0.001 | 0.0001 | 0.00001 | 0 |
| phages élués (x 10³) | 250 | 192 | 55 | 10 | 8.8 | 3.4 | 1 | 0.7 | 0.9 |

La bibliothèque R3 a été sélectionnée en utilisant des concentrations décroissantes de PA83 biotinylé, allant de 100 nM à 0,01 pM. 1 pM représente la plus faible concentration permettant d'éluer davantage de clones que le contrôle négatif. Des clones individuels, obtenus à partir de la condition 6, ont été analysés dans un premier temps par séquences des chaînes lourdes et légères. Environ 35% d'entre eux présentaient des séquences sauvages.

### Condition 6

| | | | | |
|---|---|---|---|---|
| 43 séquences H+L | 15 parentaux | 35% | | |
| | 28 variants | 65% | Clones exprimés | 47% |
| | | | Clones non exprimés | 63% |

Parmi ces 28 variants, 47% ont été suffisamment exprimés pour permettre la mesure de l'affinité par SPR. Etant donné la redondance des clones, 8 variants individuels ont été 10 exprimés et leurs constantes de cinétique ont été mesurées. Le fragment d'anticorps possédant la meilleure constante d'affinité (K_{D}= 1,8 10⁻¹⁰ M, soit une amélioration d'un facteur égal à 18,9) est le triple mutant 6.20. Ce variant possède deux mutations dans le CDR2 de la chaîne lourde (H55L et S74G) et une mutation dans le CDR2 de la chaîne légère (Q68L).

### Sélection par incubation longue

| | | | | | |
|---|---|---|---|---|---|
| Temps d'incubation (jour) | 3 | 13 | 18 | 24 | 25 |
| phages élués | 4000 | 5700 | 4000 | 18 | 0 |

| | | | | |
|---|---|---|---|---|
| 14 séquences H+L | 3 parentaux | 21% | | |
| | 11 variants | 78% | Clones exprimés | 72% |
| | | | Clones non exprimés | 27% |

Parmi les 18 clones élués au jour 24, 14 ont été entièrement séquencés (V_{H} et \/_{L}) et seulement 3 étaient sauvages. 6 variants ont été suffisamment exprimés pour déterminer leurs constantes de cinétique. Les doubles mutants J24-7 (K_{D}= 7,8 10⁻¹⁰ M) et J24-15 (K_{D}= 8,8 10⁻¹⁰ M) ont montré une affinité de liaison augmentée d'un facteur 4,35 et 3,96 respectivement.

### Humanisation des variants

Les anticorps injectés à l'homme sont très bien tolérés lorsqu'ils sont humains. A l'inverse, les anticorps d'origine animale peuvent être cause d'effets secondaires délétères et sont éliminés rapidement. Les régions hypervariables des anticorps sont cependant tellement mutées pendant la maturation d'affinité qu'il est difficile, d'après la seule analyse de leurs séquences, d'en définir l'origine. Puisque les Fab ne comportent pas de région constante, les régions charpentes sont les seules régions impliquées dans leur tolérance.

Pour améliorer la tolérance du Fab 35PA83, et des molécules qui en seront dérivées, ce Fab a été humanisé. Une analyse automatique a été réalisée sur le serveur IMGT (htfp://imgt.cines.fr) et a permis de localiser, dans les régions charpentes du Fab 35PA83, les résidus différents de ceux codés par les gènes germinaux humains, codant les séquences les plus proches de celles de 35P483. Des synthèses ou des mutations ponctuelles ont permis d'obtenir des séquences nucléotidiques codant des variants du Fab 35PA83 et comportant une, ou un petit nombre, de mutations augmentant l'homologie avec les séquences humaines. Les mutations qui ne causaient pas de dégradation significative de l'affinité, par rapport à 35PA83, figurent sur les tableaux 3 et 4. L'ensemble de ces mutations a été associé en un nouveau gène synthétique qui code pour un Fab dont les régions charpentes sont identiques à 97,75% à des régions charpentes codées par des gènes germinaux humains, confre 88,69% pour le Fab parental 35PA83. Toutefois, l'affinité de ce variant totalement humanisé (K_{D}= 9 10⁻⁹ M) est dégradée d'un facteur 3 environ par rapport au Fab 35PA83.

**Tableau 3 : mutations des régions charpentes de la chaîne lourde qui n'altèrent pas l'affinité de 35PA83**

| N° du résidu | 35PA83 | |
|---|---|---|
| 1 | aucun | Q |
| 2 | aucun | V |
| 3 | aucun | Q |
| 4 | aucun | L |
| 5 | aucun | Q |
| 6 | aucun | E |
| 12 | L | V |
| 24 | A | T |
| 45 | S | P |
| 66 | R | N |
| 80 | K | V |
| 87 | L | F |
| 92 | R | S |
| 122 | A | T |
| 123 | V | L |

**Tableau 4 : mutations des régions charpentes de la chaîne légère qui n'altèrent pas l'affinité de 35PA83.**

| N° du résidu | 35PA83 | |
|---|---|---|
| 1 | aucun | A |
| 2 | aucun | I |
| 3 | aucun | Q |
| 4 | aucun | L |
| 14 | Y | S |
| 18 | K | R |
| 24 | H | R |
| 87 | Y | F |
| 96 | S | P |
| 101 | S | T |
| 124 | L | V |

### Description des séquences de la demande antérieure FR 07 06744

<160> 6
<170> PatentIn version 3.3
<210> 1
   <211> 119
   <212> PRT
   <213> Macaca fascicularis
<400> 1
   Ser Gly Pro Gly Leu Leu Lys Pro Ser Glu Thr Leu Ser Leu Thr Cys
   1               5                   10                  15
   Ala Val Ser Gly Asp Ser Ile Ser Gly Gly Tyr Tyr Trp Ser Trp Ile
               20                  25                  30
   Arg Gln Ser Pro Gly Lys Gly Leu Glu Trp Ile Gly His Ile Tyr Gly
           35                  40                  45
   Ser Thr Ala Asp Thr Arg Tyr Asn Pro Ser Leu Lys Ser Arg Val Thr
       50                  55                  60
   Ile Ser Lys Asp Thr Ser Lys Asn Gln Leu Ser Leu Gln Leu Arg Ser
   65                  70                  75                  80
   Val Thr Ala Ala Asp Thr Ala Val Tyr Tyr Cys Ala Arg Ser Gly Tyr
                   85                  90                  95
   Asn Phe Trp Ser Gly Glu Tyr Tyr Gly Leu Asp Ser Trp Gly Gln Gly
               100                 105                 110
   Ala Val Val Thr Val Ser Ser
           115
<210> 2
   <211> 103
   <212> PRT
   <213> Macaca fascicularis
<400> 2
   Thr Gln Ser Pro Ser Ser Leu Ser Ala Tyr Val Gly Asp Lys Val Thr
   1               5                   10                  15
   Ile Thr Cys His Ala Ser Gln Gly Ile Asn Ser Trp Leu Ala Trp Tyr
               20                  25                  30
   Gln Gln Lys Pro Gly Lys Ala Pro Lys Leu Leu Ile Tyr Lys Ala Ser
           35                  40                  45
   Ser Leu Gln Ser Gly Val Pro Ser Arg Phe Ser Gly Ser Gly Ser Gly
       50                  55                  60
   Thr Asp Tyr Thr Leu Thr Ile Ser Ser Leu Gln Ser Glu Asp Phe Ala
   65                  70                  75                  80
   Ser Tyr Tyr Cys Leu Gln Tyr Asp Ser Ala Pro Leu Ala Phe Gly Pro
                   85                  90                  95
   Gly Thr Lys Leu Asp Ile Lys
               100
<210> 3
   <211> 223
   <212> PRT
   <213> Macaca fascicularis
<400> 3
<210> 4
   <211> 209
   <212> PRT
   <213> Macaca fascicularis
<400> 4
<210> 5
   <211> 670
   <212> DNA
   <213> Macaca fascicularis
<400> 5
   cgggcccagg actgctgaag ccttcggaaa ccctgtccct cacctgcgct gtctctggtg 60
   actccatcag cggtggttac tactggagct ggatccgcca gtccccaggg aaggggctgg 120
   agtggattgg gcatatctat ggtagtactg cggacaccag gtacaacccc tccctcaaga 180
   gtcgagtcac catttcaaaa gacacgtcca agaaccagct ctccctgcaa ctgaggtctg 240
   tgaccgccgc ggacacggcc gtgtattatt gtgcgagatc gggttacaat ttttggagtg 300
   gtgaatatta cggtttggat tcctggggcc aaggggccgt cgtcaccgtc tcctcagcct 360
   ccaccaaggg cccatcggtc ttccccctgg cgccctcctc caggagcacc tccgagagca 420
   cagcggccct gggctgcctg gtcaaggact acttccccga acccgtgacc gtgtcgtgga 480
   actcaggctc cctgaccagc ggcgtgcaca ccttcccggc tgtcctacag tcctcagggc 540
   tctactccct cagcagcgtg gtgaccgtgc cctccagcag cttgggcacc cagacctacg 600
   tctgcaacgt aaaccacaag cccagcaaca ccaaggtgga caagagagtt gagatcaaaa 660
   catgtggtgg 670
<210> 6
   <211> 634
   <212> DNA
   <213> Macaca fascicularis
<400> 6
   acccagtctc catcgtccct gtctgcatat gtgggagaca aagtcaccat cacttgccat
   60
   gccagtcagg gtattaacag ttggttagcc tggtatcagc agaaaccagg gaaagcccct
   120
   aaacttctga tctataaggc gtccagtttg caaagtgggg tcccatcaag gttcagcggc
   180
   agtggatctg ggacagatta tactctcacc atcagcagct tgcagtctga agactttgct
   240
   tcttattact gtctacaata tgacagtgcc ccattggctt tcggccccgg gaccaagctg
   300
   gatatcaaac gggctgtggc tccaccatct gtcttcatct tcccgccatc tgaagatcag
   360
   gtgacatctg gaactgtctc tgttgtgtgc ctgctgaata acttctatcc cagagaggcc
   420
   agcgtaaagt ggaaggtgga tggtgccctc aaaacgggta actcccagga gagtgtcaca
   480
   gagcaggaca gcaaggacaa cacctacagc ctgagcagca ccctgacgct gagcagcaca
   540
   gagtaccaga gtcacaaagt ctatgcctgc gaagtcaccc atcagggcct gagttcgccc
   600
   gtcacaaaga gcttcaacag gggagagtgt taat
   634

### SEQUENCE LISTING

<110> LFB Biotechnologies
<120> IgG 35PA83
<130> reference
<160> 37
<170> PatentIn version 3.3
<210> 1
   <211> 103
   <212> PRT
   <213> Macaca fascicularis
<400> 1
   Thr Gln Ser Pro Ser Ser Leu Ser Ala Tyr Val Gly Asp Lys Val Thr
   1               5                   10                  15
   Ile Thr Cys His Ala Ser Gln Gly Ile Asn Ser Trp Leu Ala Trp Tyr
               20                  25                  30
   Gln Gln Lys Pro Gly Lys Ala Pro Lys Leu Leu Ile Tyr Lys Ala Ser
           35                  40                  45
   Ser Leu Gln Ser Gly Val Pro Ser Arg Phe Ser Gly Ser Gly Ser Gly
       50                  55                  60
   Thr Asp Tyr Thr Leu Thr Ile Ser Ser Leu Gln Ser Glu Asp Phe Ala
   65                  70                  75                  80
   Ser Tyr Tyr Cys Leu Gln Tyr Asp Ser Ala Pro Leu Ala Phe Gly Pro
                   85                  90                  95
   Gly Thr Lys Leu Asp Ile Lys
               100
<210> 2
   <211> 119
   <212> PRT
   <213> Macaca fascicularis
<400> 2
   Ser Gly Pro Gly Leu Leu Lys Pro Ser Glu Thr Leu Ser Leu Thr Cys
   1               5                   10                  15
   Ala Val Ser Gly Asp Ser Ile Ser Ser Gly Tyr Tyr Trp Ser Trp Ile
               20                  25                  30
   Arg Gln Ser Pro Gly Lys Gly Leu Glu Trp Ile Gly His Ile Tyr Gly
   35 40 45
   Ser Thr Ala Asp Thr Lys Tyr Asn Pro Ser Leu Arg Ser Arg Val Thr
   50 55 60
   Ile Ser Lys Asp Thr Ser Lys Asn Gln Leu Ser Leu Gln Leu Arg Ser
   65 70 75 80
   Val Thr Ala Ala Asp Thr Ala Val Tyr Tyr Cys Ala Arg Ser Gly Tyr
   85 90 95
   Asn Phe Trp Ser Gly Glu Tyr Tyr Gly Leu Asp Ser Trp Gly Gln Gly
   100 105 110
   Ala Val Val Thr Val Ser Ser
   115
<210> 3
   <211> 107
   <212> PRT
   <213> Homo sapiens
<400> 3
<210> 4
   <211> 330
   <212> PRT
   <213> Homo sapiens
<400> 4
<210> 5
   <211> 210
   <212> PRT
   <213> chimeric construct
<400> 5
   Thr Gln Ser Pro Ser Ser Leu Ser Ala Tyr Val Gly Asp Lys Val Thr
   1               5                   10                  15
   Ile Thr Cys His Ala Ser Gln Gly Ile Asn Ser Trp Leu Ala Trp Tyr
               20                  25                  30
   Gln Gln Lys Pro Gly Lys Ala Pro Lys Leu Leu Ile Tyr Lys Ala Ser
           35                  40                  45
   Ser Leu Gln Ser Gly Val Pro Ser Arg Phe Ser Gly Ser Gly Ser Gly
       50                  55                  60
   Thr Asp Tyr Thr Leu Thr Ile Ser Ser Leu Gln Ser Glu Asp Phe Ala
   65                  70                  75                  80
   Ser Tyr Tyr Cys Leu Gln Tyr Asp Ser Ala Pro Leu Ala Phe Gly Pro
                   85                  90                  95
   Gly Thr Lys Leu Asp Ile Lys Arg Thr Val Ala Ala Pro Ser Val Phe
               100                 105                 110
   Ile Phe Pro Pro Ser Asp Glu Gln Leu Lys Ser Gly Thr Ala Ser Val
           115                 120                 125
   Val Cys Leu Leu Asn Asn Phe Tyr Pro Arg Glu Ala Lys Val Gln Trp
       130                 135                 140
   Lys Val Asp Asn Ala Leu Gln Ser Gly Asn Ser Gln Glu Ser Val Thr
   145                 150                 155                 160
   Glu Gln Asp Ser Lys Asp Ser Thr Tyr Ser Leu Ser Ser Thr Leu Thr
                   165                 170                 175
   Leu Ser Lys Ala Asp Tyr Glu Lys His Lys Val Tyr Ala Cys Glu Val
               180                 185                 190
   Thr His Gln Gly Leu Ser Ser Pro Val Thr Lys Ser Phe Asn Arg Gly
           195                 200                 205
   Glu Cys
       210
<210> 6
   <211> 449
   <212> PRT
   <213> chimeric construct
<400> 6
   Ser Gly Pro Gly Leu Leu Lys Pro Ser Glu Thr Leu Ser Leu Thr Cys
   1               5                   10                  15
   Ala Val Ser Gly Asp Ser Ile Ser Ser Gly Tyr Tyr Trp Ser Trp Ile
               20                  25                  30
   Arg Gln Ser Pro Gly Lys Gly Leu Glu Trp Ile Gly His Ile Tyr Gly
           35                  40                  45
   Ser Thr Ala Asp Thr Lys Tyr Asn Pro Ser Leu Arg Ser Arg Val Thr
       50                  55                  60
   Ile Ser Lys Asp Thr Ser Lys Asn Gln Leu Ser Leu Gln Leu Arg Ser
   65                  70                  75                  80
   Val Thr Ala Ala Asp Thr Ala Val Tyr Tyr Cys Ala Arg Ser Gly Tyr
                   85                  90                  95
   Asn Phe Trp Ser Gly Glu Tyr Tyr Gly Leu Asp Ser Trp Gly Gln Gly
               100                 105                 110
   Ala Val Val Thr Val Ser Ser Ala Ser Thr Lys Gly Pro Ser Val Phe
           115                 120                 125
   Pro Leu Ala Pro Ser Ser Lys Ser Thr Ser Gly Gly Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Gln Thr Tyr Ile Cys Asn Val Asn His Lys Pro
           195                 200                 205
   Ser Asn Thr Lys Val Asp Lys Lys Val Glu Pro Lys Ser Cys Asp Lys
       210                 215                 220
   Thr His Thr Cys Pro Pro Cys Pro Ala Pro Glu Leu Leu Gly Gly Pro
   225                 230                 235                 240
   Ser Val Phe Leu Phe Pro Pro Lys Pro Lys Asp Thr Leu Met Ile Ser
                   245                 250                 255
   Arg Thr Pro Glu Val Thr Cys Val Val Val Asp Val Ser His Glu Asp
               260                 265                 270
   Pro Glu Val Lys Phe Asn Trp Tyr Val Asp Gly Val Glu Val His Asn
           275                 280                 285
   Ala Lys Thr Lys Pro Arg Glu Glu Gln Tyr Asn Ser Thr Tyr Arg Val
       290                 295                 300
   Val Ser Val Leu Thr Val Leu His Gln Asp Trp Leu Asn Gly Lys Glu
   305                 310                 315                 320
   Tyr Lys Cys Lys Val Ser Asn Lys Ala Leu Pro Ala Pro Ile Glu Lys
                   325                 330                 335
   Thr Ile Ser Lys Ala Lys Gly Gln Pro Arg Glu Pro Gln Val Tyr Thr
               340                 345                 350
   Leu Pro Pro Ser Arg Asp Glu Leu Thr Lys Asn Gln Val Ser Leu Thr
           355                 360                 365
   Cys Leu Val Lys Gly Phe Tyr Pro Ser Asp Ile Ala Val Glu Trp Glu
       370                 375                 380
   Ser Asn Gly Gln Pro Glu Asn Asn Tyr Lys Thr Thr Pro Pro Val Leu
   385 390 395 400
   Asp Ser Asp Gly Ser Phe Phe Leu Tyr Ser Lys Leu Thr Val Asp Lys
   405 410 415
   Ser Arg Trp Gln Gln Gly Asn Val Phe Ser Cys Ser Val Met His Glu
   420 425 430
   Ala Leu His Asn His Tyr Thr Gln Lys Ser Leu Ser Leu Ser Pro Gly
   435 440 445
   Lys
<210> 7
   <211> 309
   <212> DNA
   <213> Macaca fascicularis
<400> 7
   acccagtctc catcgtccct gtctgcatat gtgggagaca aagtcaccat cacttgccat 60
   gccagtcagg gtattaacag ttggttagcc tggtatcagc agaaaccagg gaaagcccct 120
   aaacttctga tctataaggc gtccagtttg caaagtgggg tcccatcaag gttcagcggc 180
   agtggatctg ggacagatta tactctcacc atcagcagct tgcagtctga agactttgct 240
   tcttattact gtctacaata tgacagtgcc ccattggctt tcggccccgg gaccaagctg 300
   gatatcaaa 309
<210> 8
   <211> 363
   <212> DNA
   <213> Macaca fascicularis
<400> 8
   caggagtcgg gaccaggact gctgaagcct tcggaaaccc tgtccctcac ctgcgctgtc 60
   tctggtgact ccatcagcag cggttactac tggagctgga tccgccagtc cccagggaag 120
   gggctggagt ggattgggca tatctatggt agtactgcgg acaccaagta caacccctcc 180
   ctcaggagtc gagtcaccat ttcaaaagac acgtccaaga accagctctc cctgcaactg 240
   aggtctgtga ccgccgcgga cacggccgtg tattattgtg cgagatcggg ttacaatttt 300
   tggagtggtg aatattacgg tttggattcc tggggccaag gggccgtcgt caccgtctcc 360
   tca 363
<210> 9
   <211> 990
   <212> DNA
   <213> Homo sapiens
<400> 9
   gcctccacca agggcccatc ggtcttcccc ctggcaccct cctccaagag cacctctggg     60
   ggcacagcgg ccctgggctg cctggtcaag gactacttcc ccgaaccggt gacggtgtcg    120
   tggaactcag gcgccctgac cagcggcgtg cacaccttcc cggctgtcct acagtcctca    180
   ggactctact ccctcagcag cgtggtgacc gtgccctcca gcagcttggg cacccagacc    240
   tacatctgca acgtgaatca caagcccagc aacaccaagg tggacaagaa agttgagccc    300
   aaatcttgtg acaaaactca cacatgccca ccgtgcccag cacctgaact cctgggggga    360
   ccgtcagtct tcctcttccc cccaaaaccc aaggacaccc tcatgatctc ccggacccct    420
   gaggtcacat gcgtggtggt ggacgtgagc cacgaagacc ctgaggtcaa gttcaactgg    480
   tacgtggacg gcgtggaggt gcataatgcc aagacaaagc cgcgggagga gcagtacaac    540
   agcacgtacc gtgtggtcag cgtcctcacc gtcctgcacc aggactggct gaatggcaag    600
   gagtacaagt gcaaggtctc caacaaagcc ctcccagccc ccatcgagaa aaccatctcc    660
   aaagccaaag ggcagccccg agaaccacag gtgtacaccc tgcccccatc ccgggatgag    720
   ctgaccaaga accaggtcag cctgacctgc ctggtcaaag gcttctatcc cagcgacatc    780
   gccgtggagt gggagagcaa tgggcagccg gagaacaact acaagaccac gcctcccgtg    840
   ctggactccg acggctcctt cttcctctac agcaagctca ccgtggacaa gagcaggtgg    900
   cagcagggga acgtcttctc atgctccgtg atgcatgagg ctctgcacaa ccactacacg    960
   cagaagagcc tctccctgtc tccgggtaaa                                     990
<210> 10
   <211> 321
   <212> DNA
   <213> Homo sapiens
<400> 10
   cgaactgtgg ctgcaccaag tgtcttcatc ttcccgccat ctgatgagca gttgaaatct     60
   ggaactgcct ctgttgtgtg cctgctgaat aacttctatc ccagagaggc caaagtacag    120
   tggaaggtgg ataacgccct ccaatcgggt aactcccagg agagtgtcac agagcaggac    180
   agcaaggaca gcacctacag cctcagcagc accctgacgc tgagcaaagc agactacgag    240
   aaacacaaag tctacgcctg cgaagtcacc catcagggcc tgagctcgcc cgtcacaaag    300
   agcttcaaca ggggagagtg t                                              321
<210> 11
   <211> 630
   <212> DNA
   <213> chimeric construct
<400> 11
   acccagtctc catcgtccct gtctgcatat gtgggagaca aagtcaccat cacttgccat      60
   gccagtcagg gtattaacag ttggttagcc tggtatcagc agaaaccagg gaaagcccct     120
   aaacttctga tctataaggc gtccagtttg caaagtgggg tcccatcaag gttcagcggc     180
   agtggatctg ggacagatta tactctcacc atcagcagct tgcagtctga agactttgct     240
   tcttattact gtctacaata tgacagtgcc ccattggctt tcggccccgg gaccaagctg     300
   gatatcaaac gaactgtggc tgcaccaagt gtcttcatct tcccgccatc tgatgagcag     360
   ttgaaatctg gaactgcctc tgttgtgtgc ctgctgaata acttctatcc cagagaggcc     420
   aaagtacagt ggaaggtgga taacgccctc caatcgggta actcccagga gagtgtcaca     480
   gagcaggaca gcaaggacag cacctacagc ctcagcagca ccctgacgct gagcaaagca     540
   gactacgaga aacacaaagt ctacgcctgc gaagtcaccc atcagggcct gagctcgccc     600
   gtcacaaaga gcttcaacag gggagagtgt                                      630
<210> 12
   <211> 1353
   <212> DNA
   <213> chimeric construct
<400> 12
   caggagtcgg gaccaggact gctgaagcct tcggaaaccc tgtccctcac ctgcgctgtc      60
   tctggtgact ccatcagcag cggttactac tggagctgga tccgccagtc cccagggaag     120
   gggctggagt ggattgggca tatctatggt agtactgcgg acaccaagta caacccctcc     180
   ctcaggagtc gagtcaccat ttcaaaagac acgtccaaga accagctctc cctgcaactg     240
   aggtctgtga ccgccgcgga cacggccgtg tattattgtg cgagatcggg ttacaatttt     300
   tggagtggtg aatattacgg tttggattcc tggggccaag gggccgtcgt caccgtctcc     360
   tcagcctcca ccaagggccc atcggtcttc cccctggcac cctcctccaa gagcacctct     420
   gggggcacag cggccctggg ctgcctggtc aaggactact tccccgaacc ggtgacggtg     480
   tcgtggaact caggcgccct gaccagcggc gtgcacacct tcccggctgt cctacagtcc     540
   tcaggactct actccctcag cagcgtggtg accgtgccct ccagcagctt gggcacccag     600
   acctacatct gcaacgtgaa tcacaagccc agcaacacca aggtggacaa gaaagttgag     660
   cccaaatctt gtgacaaaac tcacacatgc ccaccgtgcc cagcacctga actcctgggg     720
   ggaccgtcag tcttcctctt ccccccaaaa cccaaggaca ccctcatgat ctcccggacc     780
   cctgaggtca catgcgtggt ggtggacgtg agccacgaag accctgaggt caagttcaac     840
   tggtacgtgg acggcgtgga ggtgcataat gccaagacaa agccgcggga ggagcagtac     900
   aacagcacgt accgtgtggt cagcgtcctc accgtcctgc accaggactg gctgaatggc     960
   aaggagtaca agtgcaaggt ctccaacaaa gccctcccag cccccatcga gaaaaccatc    1020
   tccaaagcca aagggcagcc ccgagaacca caggtgtaca ccctgccccc atcccgggat    1080
   gagctgacca agaaccaggt cagcctgacc tgcctggtca aaggcttcta tcccagcgac    1140
   atcgccgtgg agtgggagag caatgggcag ccggagaaca actacaagac cacgcctccc    1200
   gtgctggact ccgacggctc cttcttcctc tacagcaagc tcaccgtgga caagagcagg    1260
   tggcagcagg ggaacgtctt ctcatgctcc gtgatgcatg aggctctgca caaccactac    1320
   acgcagaaga gcctctccct gtctccgggt aaa                                  1353
<210> 13
   <211> 78
   <212> DNA
   <213> séquence artificielle : amorce
<400> 13
   atggacatga gggtccccgc tcagctcctg gggcttctgc tgctctggct cccaggtgcc      60
   agatgtgcca tccagttg                                                    78
<210> 14
   <211> 47
   <212> DNA
   <213> séquence artificielle : amorce
<400> 14
   ctcagtacta gtgccgccac catggacatg agggtccccg ctcagct 47
<210> 15
   <211> 44
   <212> DNA
   <213> séquence artificielle : amorce
<400> 15
   acctgggagc cagagcagca gaagccccag gagctgagcg ggga 44
<210> 16
   <211> 43
   <212> DNA
   <213> séquence artificielle : amorce
<400> 16
   tgctctggct cccaggtgcc agatgtgcca tccagttgac cca 43
<210> 17
   <211> 45
   <212> DNA
   <213> séquence artificielle : amorce
<400> 17
   ctcccacata tgcagacagg gacgatggag actgggtcaa ctgga 45
<210> 18
   <211> 47
   <212> DNA
   <213> séquence artificielle : amorce
<400> 18
   ctcagtacta gtgccgccac catggacatg agggtccccg ctcagct 47
<210> 19
   <211> 45
   <212> DNA
   <213> séquence artificielle : amorce
<400> 19
   ctcccacata tgcagacagg gacgatggag actgggtcaa ctgga 45
<210> 20
   <211> 25
   <212> DNA
   <213> séquence artificielle : amorce
<400> 20
   tcgtccctgt ctgcatatgt gggag 25
<210> 21
   <211> 42
   <212> DNA
   <213> séquence artificielle : amorce
<400> 21
   gatgaagaca cttggtgcag ccacagttcg tttgatatcc ag 42
<210> 22
   <211> 47
   <212> DNA
   <213> séquence artificielle : amorce
<400> 22
   ctcagtacta gtgccgccac catggacatg agggtccccg ctcagct 47
<210> 23
   <211> 42
   <212> DNA
   <213> séquence artificielle : amorce
<400> 23
   gatgaagaca cttggtgcag ccacagttcg tttgatatcc ag 42
<210> 24
   <211> 76
   <212> DNA
   <213> séquence artificielle : amorce
<400> 24
   atgaaacatc tgtggttctt ccttctcctg gtggcagctc ccagatgggt cctgtcccag 60
   gtgcagctgc aggagt 76
<210> 25
   <211> 47
   <212> DNA
   <213> séquence artificielle : amorce
<400> 25
   ctcagtgcta gcgccgccac catgaaacat ctgtggttct tccttct 47
<210> 26
   <211> 39
   <212> DNA
   <213> séquence artificielle : amorce
<400> 26
   cccatctggg agctgccacc aggagaagga agaaccaca 39
<210> 27
   <211> 42
   <212> DNA
   <213> séquence artificielle : amorce
<400> 27
   tggcagctcc cagatgggtc ctgtcccagg tgcagctgca gg 42
<210> 28
   <211> 35
   <212> DNA
   <213> séquence artificielle : amorce
<400> 28
   cagtcctggt cccgactcct gcagctgcac ctggg 35
<210> 29
   <211> 47
   <212> DNA
   <213> séquence artificielle : amorce
<400> 29
   ctcagtgcta gcgccgccac catgaaacat ctgtggttct tccttct 47
<210> 30
   <211> 35
   <212> DNA
   <213> séquence artificielle : amorce
<400> 30
   cagtcctggt cccgactcct gcagctgcac ctggg 35
<210> 31
   <211> 27
   <212> DNA
   <213> séquence artificielle : amorce
<400> 31
   cagctgcagg agtcgggacc aggactg 27
<210> 32
   <211> 38
   <212> DNA
   <213> séquence artificielle : amorce
<400> 32
   accgatgggc ccttggtgga ggctgaggag acggtgac 38
<210> 33
   <211> 47
   <212> DNA
   <213> séquence artificielle : amorce
<400> 33
   ctcagtgcta gcgccgccac catgaaacat ctgtggttct tccttct 47
<210> 34
   <211> 38
   <212> DNA
   <213> séquence artificielle : amorce
<400> 34
   accgatgggc ccttggtgga ggctgaggag acggtgac 38
<210> 35
   <211> 20
   <212> DNA
   <213> séquence artificielle : amorce
<400> 35
   gctcgataca ataaacgcca 20
<210> 36
   <211> 21
   <212> DNA
   <213> séquence artificielle : amorce
<400> 36
   tctgggatag aagttattca g 21
<210> 37
   <211> 23
   <212> DNA
   <213> chimeric construct
<400> 37
   ggaagtagtc cttgaccagg cag 23

## Revendications

1. Immunoglobuline de classe G (IgG) dirigée contre l'antigène protecteur (PA) de la toxine du charbon, comprenant :
- une région variable de chaîne légère ayant une séquence d'acides aminés représentée par la séquence SEQ ID N° 1 ou ayant une séquence d'acides aminés représentée par la séquence SEQ ID N° 1 et comprenant en outre au moins une mutation sélectionnée parmi :
- aucun/A (1) dans laquelle un acide aminé A a été ajouté en position -4 de la séquence SEQ ID N° 1 , soit immédiatement en amont du résidu N°-3 s'il existe, et à défaut immédiatement en amont du résidu N°-2, et à défaut immédiatement en amont du résidu N°-1, et à défaut immédiatement en amont du résidu N°1
- aucun/I (2) dans laquelle un acide aminé L a été ajouté en position -3 de la séquence SEQ IDN° 1 , soit immédiatement en amont du résidu N°-2 s'il existe, et à défaut immédiatement en amont du résidu N°-1, et à défaut immédiatement en amont du résidu N°1
- aucun/Q (3) dans laquelle un acide aminé Q a été ajouté en position -2 de la séquence SEQ ID N°1, soit immédiatement en amont du résidu N°-1 s'il existe, et à défaut immédiatement en amont du résidu N°1
- aucun/L (4) dans laquelle un acide aminé L a été ajouté en position -1 de la séquence SEQ ID N° 1 , soit immédiatement en amont du résidu N°1
- Y/S, dans laquelle l'acide aminé Y en position 10 de la séquence SEQ ID N° 1 est remplacé par l'acide aminé S
- K/R, dans laquelle l'acide aminé K en position 14 de la séquence SEQ ID N° 1 est remplacé par l'acide aminé R
- H/R, dans laquelle l'acide aminé H en position 20 de la séquence SEQ ID N° 1 est remplacé par l'acide amminé R
- L/V, dans laquelle l'acide aminé L en position 100 de la séquence SEQ ID N° 1 est remplacé par l'acide aminé V
- une région variable de chaîne lourde ayant une séquence d'acides aminés représentée par la séquence SEQ ID N° 2, ou ayant une séquence d'acides aminés représentée par la séquence SEQ ID N° 2 et comprenant en outre au moins une mutation sélectionnée parmi les mutations suivantes :
- aucun/Q (1) dans laquelle un acide aminé Q a été ajouté en position -6 de la séquence SEQ ID N° 2, soit immédiatement en amont du résidu N°-5 s'il existe, et à défaut immédiatement en amont du résidu N°-4, et à défaut immédiatement en amont du résidu N°-3, et à défaut immédiatement en amont du résidu N°-2, et à défaut immédiatement en amont du résidu N°-1, et à défaut immédiatement en amont du résidu N°1
- aucun/V (2) dans laquelle un acide aminé V a été ajouté en position -5 de la séquence SEQ ID N° 2, soit immédiatement en amont du résidu N°-4 s'il existe, et à défaut immédiatement en amont du résidu N°-3, et à défaut immédiatement en amont du résidu N°-2, et à défaut immédiatement en amont du résidu N°-1, et à défaut immédiatement en amont du résidu N°1
- aucun/Q (3) dans laquelle un acide aminé Q a été ajouté en position -4 de la séquence SEQ ID N° 2, soit immédiatement en amont du résidu N°-3 s'il existe, et à défaut immédiatement en amont du résidu N°-2, et à défaut immédiatement en amont du résidu N°-1, et à défaut immédiatement en amont du résidu N°1
- aucun/L (4) dans laquelle un acide aminé L a été ajouté en position -3 de la séquence SEQ ID N° 2, soit immédiatement en amont du résidu N°-2 s'il existe, et à défaut immédiatement en amont du résidu N°-1, et à défaut immédiatement en amont du résidu N°1
- aucun/Q (5) dans laquelle un acide aminé Q a été ajouté en position -2 de la séquence SEQ ID N° 2, soit immédiatement en amont du résidu N°-1 s'il existe, et à défaut immédiatement en amont du résidu N°1
- aucun/E (6) dans laquelle un acide aminé E a été ajouté en position -1 de la séquence SEQ ID N° 2, soit immédiatement en amont du résidu N°1
- L/V, dans laquelle l'acide aminé L en position 5 de la séquence SEQ ID N° 2 est remplacé par l'acide aminé V
- A/T, dans laquelle l'acide aminé A en position 17 de la séquence SEQ ID N° 2 est remplacé par l'acide aminé T
- A/T, dans laquelle l'acide aminé A en position 113 de la séquence SEQ ID N° 2 est remplacé par l'acide aminé T
- V/L, dans laquelle l'acide aminé V en position 114 de la séquence SEQ ID N° 2 est remplacé par l'acide aminé L
ladite immunoglobuline étant une IgG1 ou un IgG2.

2. Immunoglobuline de classe G (IgG) selon la revendication 1, **caractérisée en ce qu'**elle comprend :
- une région variable de chaîne légère ayant une séquence d'acides aminés représentée par la séquence SEQ ID N° 1, et
- une région variable de chaîne lourde ayant une séquence d'acides aminés représentée par la séquence SEQ ID N° 2

3. IgG selon la revendication 1, **caractérisée en ce que** la région variable de la chaîne légère (SEQ ID N°1) comprend les mutations suivantes :
- aucun/A (1) dans laquelle un acide aminé A a été ajouté en position -4 de la séquence SEQ ID N° 1, soit immédiatement en amont du résidu N°-3 s'il existe, et à défaut immédiatement en amont du résidu N°-2, et à défaut immédiatement en amont du résidu N°-1, et à défaut immédiatement en amont du résidu N°1
- aucun/I (2) dans laquelle un acide aminé I a été ajouté en position -3 de la séquence SEQ IDN° 1, soit immédiatement en amont du résidu N°-2 s'il existe, et à défaut immédiatement en amont du résidu N°-1, et à défaut immédiatement en amont du résidu N°1
- aucun/Q (3) dans laquelle un acide aminé Q a été ajouté en position -2 de la séquence SEQ ID N° 1 , soit immédiatement en amont du résidu N°-1 s'il existe, et à défaut immédiatement en amont du résidu N°1
- aucun/L (4) dans laquelle un acide aminé L a été ajouté en position -1 de la séquence SEQ ID N° 1 , soit immédiatement en amont du résidu N°1
- Y/S, dans laquelle l'acide aminé Y en position 10 de la séquence SEQ ID N° 1 est remplacé par l'acide aminé S
- K/R, dans laquelle l'acide aminé Ken position 14 de la séquence SEQ ID N° -1 est remplacé par l'acide aminé R
- H/R, dans laquelle l'acide aminé H en position 20 de la séquence SEQ ID N° 1 est remplacé par l'acide amminé R
- L/V', dans laquelle l'acide aminé L en position 100 de la séquence SEQ ID N° 1 est remplacé par l'acide aminé V.

4. IgG selon l'une des revendications 1 ou 3, **caractérisée en ce que** la région variable de chaîne lourde ayant une séquence d'acides aminés représentée par la séquence SEQ ID N°2 comprend les mutations suivantes :
- aucun/Q (1) dans laquelle un acide aminé Q a été ajouté en position -6 de la séquence SEQ ID N° 2, soit immédiatement en amont du résidu N°-5 s'il existe, et à défaut immédiatement en amont du résidu N°-4, et à défaut immédiatement en amont du résidu N°-3, et à défaut immédiatement en amont du résidu N°-2, et à défaut immédiatement en amont du résidu N °-1, et à défaut immédiatement en amont du résidu N°1
- aucun/V (2) dans laquelle un acide aminé V a été ajouté en position -5 de la séquence SEQ ID N ° 2, soit immédiatement en amont du résidu N°-4 s'il existe, et à défaut immédiatement en amont du résidu N°-3, et à défaut immédiatement en amont du résidu N°-2, et à défaut immédiatement en amont du résidu N°-1, et à défaut immédiatement en amont du résidu N°1
- aucun/Q (3) dans laquelle un acide aminé Q a été ajouté en position -4 de la séquence SEQ ID N ° 2, soit immédiatement en amont du résidu N°-3 s'il existe, et à défaut immédiatement en amont du résidu N°-2, et à défaut immédiatement en amont du résidu N°-1, et à défaut immédiatement en amont du résidu N°1
- aucun/L (4) dans laquelle un acide aminé L a été ajouté en position -3 de la séquence SEQ ID N° 2, soit immédiatement en amont du résidu N°-2 s'il existe, et à défaut immédiatement en amont du résidu N°-1, et à défaut immédiatement en amont du résidu N°1
- aucun/Q (5) dans laquelle un acide aminé Q a été ajouté en position -2 de la séquence SEQ ID N° 2, soit immédiatement en amont du résidu N°-1 s'il existe, et à défaut immédiatement en amont du résidu N°1
- aucun/E (6) dans laquelle un acide aminéE a été ajouté en position -1 de la séquence SEQ ID N° 2, soit immédiatement en amont du résidu N°1
- L/V, dans laquelle l'acide aminé L en position 5 de la séquence SEQ ID N° 2 est remplacé par l'acide aminé V
- A/T, dans laquelle l'acide aminé A en position 17 de la séquence SEQ ID N° 2 est remplacé par l'acide aminé T
- A/T, dans laquelle l'acide aminé A en position 113 de la séquence SEQ ID N° 2 est remplacé par l'acide aminé T
- V/L, dans laquelle l'acide aminé V en position 114 de la séquence SEQ ID N° 2 est remplacé par l'acide aminé L.

5. IgG selon l'une quelconque des revendications précédentes, **caractérisée en ce que** la région constante de la chaîne légère possède une séquence d'acides aminés représentée par la séquence SEQ ID N°3, et la région constante de la chaîne lourde possède une séquence d'acides aminés représentée par la séquence SEQ ID N°4.

6. IgG selon l'une quelconque des revendications précédentes, **caractérisée en ce que** la région constante de chacune de ses chaînes lourdes est de type γ1, et de préférence **en ce que** :
- la région constante de chacune de ses chaînes lourdes comprend la séquence en acides aminés codée par la séquence SEQ ID N°9 et **en ce que** la région constante de chacune de ses chaînes légères comprend la séquence en acides aminés codée par la séquence SEQ ID N°10, ou
- chacune de ses chaînes légères comprend la séquence en acides aminés codée par la séquence SEQ ID N°11 et **en ce que** chacune de ses chaînes lourdes comprend la séquence en acides aminés codée par la séquence SEQ ID N°12.

7. Acide nucléique codant un anticorps selon l'une quelconque des revendications 1 à 6.

8. Vecteur comprenant un acide nucléique selon la revendication 7.

9. Cellule hôte comprenant un vecteur selon la revendication 8.

10. Composition, de préférence composition pharmaceutique, comprenant au moins une IgG humaine selon l'une quelconque des revendications 1 à 6.

11. Utilisation d'une IgG humaine selon l'une quelconque des revendications 1 à 6 pour la préparation d'un médicament destiné au traitement ou à la prévention d'une infection par *Bacillus anthracis.*

12. Kit pour la détection d'une toxine du charbon comprenant PA, ledit kit comprenant :
- un container comprenant au moins une IgG selon l'une quelconque des revendications 1 à 6 marquée, et
- un container comprenant des moyens de détection de cette IgG marquée.

13. Méthode pour la détection in vitro d'une toxine du charbon comprenant PA dans un échantillon biologique, comprenant :
- la mise en contact de l'échantillon avec au moins une IgG selon l'une quelconque des revendications 1 à 6, et
- la détection de la liaison de ladite IgG comme indicateur de la présence de ladite toxine du charbon.

14. Immunoconjugué comprenant une IgG selon l'une quelconque des revendications 1 à 6 liée à un agent thérapeutique.

## Patentansprüche

1. Immunglobulin der Klasse G (IgG), das gegen das Schutzantigen (PA) des Anthraxtoxins gerichtet ist, umfassend:
- eine variable Region der leichten Kette mit einer Aminosäuresequenz gemäß der Sequenz SEQ ID Nr. 1 oder mit einer Aminosäuresequenz gemäß der Sequenz SEQ ID Nr. 1 sowie weiterhin umfassend mindestens eine Mutation, ausgewählt aus den folgenden:
- keine/A (1), worin eine Aminosäure A in Position -4 der Sequenz SEQ ID Nr. 1 angefügt wurde, und zwar unmittelbar stromaufwärts des Rests Nr. -3, falls vorhanden, und falls nicht vorhanden, unmittelbar stromaufwärts des Rests Nr. -2, und falls nicht vorhanden, unmittelbar stromaufwärts des Rests Nr. -1, und falls nicht vorhanden, unmittelbar stromaufwärts des Rests Nr. 1,
- keine/I (2), worin eine Aminosäure L in Position -3 der Sequenz SEQ ID Nr. 1 angefügt wurde, und zwar unmittelbar stromaufwärts des Rests Nr. -2, falls vorhanden, und falls nicht vorhanden, unmittelbar stromaufwärts des Rests Nr. -1, und falls nicht vorhanden, unmittelbar stromaufwärts des Rests Nr. 1,
- keine/Q (3), worin eine Aminosäure Q in Position - 2 der Sequenz SEQ ID Nr. 1 angefügt wurde, und zwar unmittelbar stromaufwärts des Rests Nr. -1, falls vorhanden, und falls nicht vorhanden, unmittelbar stromaufwärts des Rests Nr. 1,
- keine/L (4), worin eine Aminosäure L in Position -1 der Sequenz SEQ ID Nr. 1 angefügt wurde, und zwar unmittelbar stromaufwärts des Rests Nr. 1,
- Y/S, worin die Aminosäure Y in Position 10 der Sequenz SEQ ID Nr. 1 durch die Aminosäure S ersetzt ist,
- K/R, worin die Aminosäure K in Position 14 der Sequenz SEQ ID Nr. 1 durch die Aminosäure R ersetzt ist,
- H/R, worin die Aminosäure H in Position 20 der Sequenz SEQ ID Nr. 1 durch die Aminosäure R ersetzt ist,
- L/V, worin die Aminosäure L in Position 100 der Sequenz SEQ ID Nr. 1 durch die Aminosäure V ersetzt ist,
- eine variable Region der schweren Kette mit einer Aminosäuresequenz gemäß der Sequenz SEQ ID Nr. 2 oder mit einer Aminosäuresequenz gemäß der Sequenz SEQ ID Nr. 2 sowie weiterhin umfassend mindestens eine Mutation, ausgewählt aus den folgenden Mutationen:
- keine/Q (1), worin eine Aminosäure Q in Position -6 der Sequenz SEQ ID Nr. 2 angefügt wurde, und zwar unmittelbar stromaufwärts des Rests Nr. -5, falls vorhanden, und falls nicht vorhanden, unmittelbar stromaufwärts des Rests Nr. -4, und falls nicht vorhanden, unmittelbar stromaufwärts des Rests Nr. -3, und falls nicht vorhanden, unmittelbar stromaufwärts des Rests Nr. -2, und falls nicht vorhanden, unmittelbar stromaufwärts des Rests Nr. -1, und falls nicht vorhanden, unmittelbar stromaufwärts des Rests Nr. 1,
- keine/V (2), worin eine Aminosäure V in Position -5 der Sequenz SEQ ID Nr. 2 angefügt wurde, und zwar unmittelbar stromaufwärts des Rests Nr. -4, falls vorhanden, und falls nicht vorhanden, unmittelbar stromaufwärts des Rests Nr. -3, und falls nicht vorhanden, unmittelbar stromaufwärts des Rests Nr. -2, und falls nicht vorhanden, unmittelbar stromaufwärts des Rests Nr. -1, und falls nicht vorhanden, unmittelbar stromaufwärts des Rests Nr. 1,
- keine/Q (3), worin eine Aminosäure Q in Position -4 der Sequenz SEQ ID Nr. 2 angefügt wurde, und zwar unmittelbar stromaufwärts des Rests Nr. -3, falls vorhanden, und falls nicht vorhanden, unmittelbar stromaufwärts des Rests Nr. -2, und falls nicht vorhanden, unmittelbar stromaufwärts des Rests Nr. -1, und falls nicht vorhanden, unmittelbar stromaufwärts des Rests Nr. 1,
- keine/L (4), worin eine Aminosäure L in Position -3 der Sequenz SEQ ID Nr. 2 angefügt wurde, und zwar unmittelbar stromaufwärts des Rests Nr. -2, falls vorhanden, und falls nicht vorhanden, unmittelbar stromaufwärts des Rests Nr. -1, und falls nicht vorhanden, unmittelbar stromaufwärts des Rests Nr. 1,
- keine/Q (5), worin eine Aminosäure Q in Position -2 der Sequenz SEQ ID Nr. 2 angefügt wurde, und zwar unmittelbar stromaufwärts des Rests Nr. -1, falls vorhanden, und falls nicht vorhanden, unmittelbar stromaufwärts des Rests Nr. 1,
- keine/E (6), worin eine Aminosäure E in Position -1 der Sequenz SEQ ID Nr. 2 angefügt wurde, und zwar unmittelbar stromaufwärts des Rests Nr. 1,
- L/V, worin die Aminosäure L in Position 5 der Sequenz SEQ ID Nr. 2 durch die Aminosäure V ersetzt ist,
- A/T, worin die Aminosäure A in Position 17 der Sequenz SEQ ID Nr. 2 durch die Aminosäure T ersetzt ist,
- A/T, worin die Aminosäure A in Position 113 der Sequenz SEQ ID Nr. 2 durch die Aminosäure T ersetzt ist,
- V/L, worin die Aminosäure V in Position 114 der Sequenz SEQ ID Nr. 2 durch die Aminosäure L ersetzt ist,
wobei es sich bei dem Immunglobulin um ein IgG1 oder ein IgG2 handelt.

2. Immunglobulin der Klasse G (IgG) nach Anspruch 1, **dadurch gekennzeichnet, dass** es Folgendes umfasst:
- eine variable Region der leichten Kette mit einer Aminosäuresequenz gemäß der Sequenz SEQ ID Nr. 1, und
- eine variable Region der schweren Kette mit einer Aminosäuresequenz gemäß der Sequenz SEQ ID Nr. 2.

3. IgG nach Anspruch 1, **dadurch gekennzeichnet, dass** die variable Region der leichten Kette (SEQ ID Nr. 1) die folgenden Mutationen umfasst:
- keine/A (1), worin eine Aminosäure A in Position-4 der Sequenz SEQ ID Nr. 1 angefügt wurde, und zwar unmittelbar stromaufwärts des Rests Nr. -3, falls vorhanden, und falls nicht vorhanden, unmittelbar stromaufwärts des Rests Nr. -2, und falls nicht vorhanden, unmittelbar stromaufwärts des Rests Nr. -1, und falls nicht vorhanden, unmittelbar stromaufwärts des Rests Nr. 1,
- keine/I (2), worin eine Aminosäure L in Position -3 der Sequenz SEQ ID Nr. 1 angefügt wurde, und zwar unmittelbar stromaufwärts des Rests Nr. -2, falls vorhanden, und falls nicht vorhanden, unmittelbar stromaufwärts des Rests Nr. -1, und falls nicht vorhanden, unmittelbar stromaufwärts des Rests Nr. 1,
- keine/Q (3), worin eine Aminosäure Q in Position-2 der Sequenz SEQ ID Nr. 1 angefügt wurde, und zwar unmittelbar stromaufwärts des Rests Nr. -1, falls vorhanden, und falls nicht vorhanden, unmittelbar stromaufwärts des Rests Nr. 1,
- keine/L (4), worin eine Aminosäure L in Position -1 der Sequenz SEQ ID Nr. 1 angefügt wurde, und zwar unmittelbar stromaufwärts des Rests Nr. 1,
- Y/S, worin die Aminosäure Y in Position 10 der Sequenz SEQ ID Nr. 1 durch die Aminosäure S ersetzt ist,
- K/R, worin die Aminosäure K in Position 14 der Sequenz SEQ ID Nr. 1 durch die Aminosäure R ersetzt ist,
- H/R, worin die Aminosäure H in Position 20 der Sequenz SEQ ID Nr. 1 durch die Aminosäure R ersetzt ist,
- L/V, worin die Aminosäure L in Position 100 der Sequenz SEQ ID Nr. 1 durch die Aminosäure V ersetzt ist.

4. IgG nach einem der Ansprüche 1 oder 3, **dadurch gekennzeichnet, dass** die variable Region der schweren Kette mit einer Aminosäuresequenz gemäß der Sequenz SEQ ID Nr. 2 die folgenden Mutationen aufweist:
- keine/Q (1), worin eine Aminosäure Q in Position -6 der Sequenz SEQ ID Nr. 2 angefügt wurde, und zwar unmittelbar stromaufwärts des Rests Nr. -5, falls vorhanden, und falls nicht vorhanden, unmittelbar stromaufwärts des Rests Nr. -4, und falls nicht vorhanden, unmittelbar stromaufwärts des Rests Nr. -3, und falls nicht vorhanden, unmittelbar stromaufwärts des Rests Nr. -2, und falls nicht vorhanden, unmittelbar stromaufwärts des Rests Nr. -1, und falls nicht vorhanden, unmittelbar stromaufwärts des Rests Nr. 1,
- keine/V (2), worin eine Aminosäure V in Position -5 der Sequenz SEQ ID Nr. 2 angefügt wurde, und zwar unmittelbar stromaufwärts des Rests Nr. -4, falls vorhanden, und falls nicht vorhanden, unmittelbar stromaufwärts des Rests Nr. -3, und falls nicht vorhanden, unmittelbar stromaufwärts des Rests Nr. -2, und falls nicht vorhanden, unmittelbar stromaufwärts des Rests Nr. -1, und falls nicht vorhanden, unmittelbar stromaufwärts des Rests Nr. 1,
- keine/Q (3), worin eine Aminosäure Q in Position -4 der Sequenz SEQ ID Nr. 2 angefügt wurde, und zwar unmittelbar stromaufwärts des Rests Nr. -3, falls vorhanden, und falls nicht vorhanden, unmittelbar stromaufwärts des Rests Nr. -2, und falls nicht vorhanden, unmittelbar stromaufwärts des Rests Nr. -1, und falls nicht vorhanden, unmittelbar stromaufwärts des Rests Nr. 1,
- keine/L (4), worin eine Aminosäure L in Position -3 der Sequenz SEQ ID Nr. 2 angefügt wurde, und zwar unmittelbar stromaufwärts des Rests Nr. -2, falls vorhanden, und falls nicht vorhanden, unmittelbar stromaufwärts des Rests Nr. -1, und falls nicht vorhanden, unmittelbar stromaufwärts des Rests Nr. 1,
- keine/Q (5), worin eine Aminosäure Q in Position -2 der Sequenz SEQ ID Nr. 2 angefügt wurde, und zwar unmittelbar stromaufwärts des Rests Nr. -1, falls vorhanden, und falls nicht vorhanden, unmittelbar stromaufwärts des Rests Nr. 1,
- keine/E (6), worin eine Aminosäure E in Position -1 der Sequenz SEQ ID Nr. 2 angefügt wurde, und zwar unmittelbar stromaufwärts des Rests Nr. 1,
- L/V, worin die Aminosäure L in Position 5 der Sequenz SEQ ID Nr. 2 durch die Aminosäure V ersetzt ist,
- A/T, worin die Aminosäure A in Position 17 der Sequenz SEQ ID Nr. 2 durch die Aminosäure T ersetzt ist,
- A/T, worin die Aminosäure A in Position 113 der Sequenz SEQ ID Nr. 2 durch die Aminosäure T ersetzt ist,
- V/L, worin die Aminosäure V in Position 114 der Sequenz SEQ ID Nr. 2 durch die Aminosäure L ersetzt ist.

5. IgG nach einem der vorhergehenden Ansprüche, **dadurch gekennzeichnet, dass** die konstante Region der leichten Kette eine Aminosäuresequenz gemäß der Sequenz SEQ ID Nr. 3 aufweist und die konstante Region der schweren Kette eine Aminosäuresequenz gemäß der Sequenz SEQ ID Nr. 4 aufweist.

6. IgG nach einem der vorhergehenden Ansprüche, **dadurch gekennzeichnet, dass** die konstante Region von jeder seiner schweren Ketten den Typ γ1 aufweist, und vorzugsweise dadurch, dass:
- die konstante Region von jeder seiner schweren Ketten die Aminosäuresequenz, die von der Sequenz SEQ ID Nr. 9 codiert wird, umfasst, und dadurch, dass die konstante Region von jeder seiner leichten Ketten die Aminosäuresequenz, die von der Sequenz SEQ ID Nr. 10 codiert wird, umfasst, oder
- jede seiner leichten Ketten die Aminosäuresequenz, die von der Sequenz SEQ ID Nr. 11 codiert wird, umfasst, und dadurch, dass jede seiner schweren Ketten die Aminosäuresequenz, die von der Sequenz SEQ ID Nr. 12 codiert wird, umfasst.

7. Nukleinsäure, die für einen Antikörper nach einem der Ansprüche 1 bis 6 codiert.

8. Vektor, umfassend eine Nukleinsäure nach Anspruch 7.

9. Wirtszelle, umfassend einen Vektor nach Anspruch 8.

10. Zusammensetzung, vorzugsweise pharmazeutische Zusammensetzung, umfassend mindestens ein menschliches IgG nach einem der Ansprüche 1 bis 6.

11. Verwendung eines menschlichen IgG nach einem der Ansprüche 1 bis 6 für die Herstellung eines Arzneimittels zur Behandlung oder Vorbeugung einer *Bacillus*-*anthracis*-Infektion.

12. Kit zum Nachweisen eines Anthraxtoxins umfassend PA, wobei das Kit Folgendes umfasst:
- ein Behältnis, das mindestens ein markiertes IgG nach einem der Ansprüche 1 bis 6 umfasst, und
- ein Behältnis, das Mittel zum Nachweisen dieses markierten IgG umfasst.

13. Verfahren für den in-vitro-Nachweis eines Anthraxtoxins umfassend PA in einer biologischen Probe, umfassend die folgenden Schritte:
- Inkontaktbringen der Probe mit mindestens einem IgG nach einem der Ansprüche 1 bis 6, und
- Nachweisen der Bindung des IgG als Indikator für das Vorliegen des Anthraxtoxins.

14. Immunkonjugat, umfassend ein an ein Therapeutikum gebundenes IgG nach einem der Ansprüche 1 bis 6.

## Claims

1. A G-class immunoglobulin (IgG) directed against the protective antigen (PA) of the anthrax toxin, comprising:
- a light chain variable region having an amino acid sequence represented by the sequence SEQ ID N° 1 or having an amino acid sequence represented by the sequence SEQ ID N° 1 and further comprising at least one mutation selected from:
- none/A (1) wherein an amino acid A was added at position -4 of the sequence SEQ ID N° 1, either immediately upstream of residue N° -3 if present, or if absent, immediately upstream of residue N° -2, or if absent, immediately upstream of residue N° -1, or if absent, immediately upstream of residue N° 1
- none/l (2) wherein an amino acid 1 was added at position -3 of the sequence SEQ ID N° 1, either immediately upstream of residue N° -2 if present, or if absent, immediately upstream of residue N° -1, or if absent, immediately upstream of residue N° 1
- none/Q (3) wherein an amino acid Q was added at position -2 of the sequence SEQ ID N° 1, either immediately upstream of residue N° -1 if present, or if absent, immediately upstream of residue N° 1,
- none/L (4) wherein an amino acid L was added at position -1 of the sequence SEQ ID N° 1, either immediately upstream of residue N° 1
- Y/S, wherein the amino acid Y at position 10 of the sequence SEQ ID N° 1 is replaced with amino acid S
- K/R, wherein the amino acid K at position 14 of the sequence SEQ ID N° 1 is replaced with amino acid R
- H/R, wherein the amino acid H at position 20 of the sequence SEQ ID N° 1 is replaced with amino acid R
- L/V, wherein the amino acid L at position 100 of the sequence SEQ ID N° 1 is replaced with amino acid V
- a heavy chain variable region having an amino acid sequence represented by the sequence SEQ ID N° 2, or having an amino acid sequence represented by the sequence SEQ ID N° 2 and further comprising at least one mutation selected from the following mutations:
- none/Q (1) wherein an amino acid Q was added at position -6 of the sequence SEQ ID N° 2, either immediately upstream of residue N° -5 if present, or if absent, immediately upstream of residue N° -4, or if absent, immediately upstream of residue N° -3, or if absent, immediately upstream of residue N° -2, or if absent, immediately upstream of residue N° -1, or if absent, immediately upstream of residue N° I
- none/V (2) wherein an amino acid V was added at position -5 of the sequence SEQ ID N° 2, either immediately upstream of residue N° -4 if present, or if absent, immediately upstream of residue N° -3, or if absent, immediately upstream of residue N° -2, or if absent, immediately upstream of residue N° -1, or if absent, immediately upstream of residue N° 1
- none/Q (3) wherein an amino acid Q was added at position -4 of the sequence SEQ ID N° 2, either immediately upstream of residue N° -3 if present, or if absent, immediately upstream of residue N° -2, or if absent, immediately upstream of residue N° -1, or if absent, immediately upstream of residue N° 1
- none/L (4) wherein an amino acid L was added at position -3 of the sequence SEQ ID N° 2, either immediately upstream of residue N° -2 if present, or if absent, immediately upstream of residue N° -1, or if absent, immediately upstream of residue N° 1
- none/Q (5) wherein an amino acid Q was added at position -2 of the sequence SEQ ID N° 2, either immediately upstream of residue N° -1 if present, or if absent, immediately upstream of residue N° 1
- none/E (6) wherein an amino acid E was added at position -1 of the sequence SEQ ID N° 2, either immediately upstream of residue N° 1
- L/V, wherein the amino acid L at position 5 of the sequence SEQ ID N° 2 is replaced with amino acid V
- A/T, wherein the amino acid A at position 17 of the sequence SEQ ID N° 2 is replaced with amino acid T
- A/T, wherein the amino acid A at position 113 of the sequence SEQ ID N° 2 is replaced with amino acid T
- V/L, wherein the amino acid V at position 114 of the sequence SEQ ID N° 2 is replaced with amino acid L
wherein the immunoglobulin is an IgG1 or an IgG2.

2. The G-class immunoglobulin (IgG) according to claim 1, **characterized in that** it comprises:
- a light chain variable region having an amino acid sequence represented by the sequence SEQ ID N° 1, and
- a heavy chain variable region having an amino acid sequence represented by the sequence SEQ ID N° 2.

3. The IgG according to claim 1, **characterized in that** the light chain variable region (SEQ ID N° 1) comprises the following mutations:
- none/A (1) wherein an amino acid A was added at position -4 of the sequence SEQ ID N° 1, either immediately upstream of residue N° -3 if present, or if absent, immediately upstream of residue N° -2, or if absent, immediately upstream of residue N° -1, or if absent, immediately upstream of residue N° 1
- none/l (2) wherein an amino acid 1 was added at position -3 of the sequence SEQ ID N° 1, either immediately upstream of residue N° -2 if present, or if absent, immediately upstream of residue N° -1, or if absent, immediately upstream of residue N° 1
- none/Q (3) wherein an amino acid Q was added at position -2 of the sequence SEQ ID N° 1, either immediately upstream of residue N° -1 if present, or if absent, immediately upstream of residue N° 1,
- none/L (4) wherein an amino acid L was added at position -1 of the sequence SEQ ID N° 1, either immediately upstream of residue N° 1
- Y/S, wherein the amino acid Y at position 10 of the sequence SEQ ID N° 1 is replaced with amino acid S
- K/R, wherein the amino acid K at position 14 of the sequence SEQ ID N° 1 is replaced with amino acid R
- H/R, wherein the amino acid H at position 20 of the sequence SEQ ID N° 1 is replaced with amino acid R
- L/V, wherein the amino acid L at position 100 of the sequence SEQ ID N° 1 is replaced with amino acid V.

4. The IgG according to any one of claims 1 or 3, **characterized in that** the heavy chain variable region having an amino acid sequence represented by the sequence SEQ ID N° 2 comprises the following mutations:
- none/Q (1) wherein an amino acid Q was added at position -6 of the sequence SEQ ID N° 2, either immediately upstream of residue N° -5 if present, or if absent, immediately upstream of residue N° -4, or if absent, immediately upstream of the residue N° -3, or if absent, immediately upstream of residue N° -2, or if absent, immediately upstream of residue N° -1, or if absent, immediately upstream of residue N° 1
- none/V (2) wherein an amino acid V was added at position -5 of the sequence SEQ ID N° 2, either immediately upstream of residue N° -4 if present, or if absent, immediately upstream of residue N° -3, or if absent, immediately upstream of residue N° -2, or if absent, immediately upstream of the residue N° -1, or if absent, immediately upstream of residue N° 1
- none/Q (3) wherein an amino acid Q was added at position -4 of the sequence SEQ ID N° 2, either immediately upstream of residue N° -3 if present, or if absent, immediately upstream of residue N° -2, or if absent, immediately upstream of residue N° -1, or if absent, immediately upstream of residue N° 1
- none/L (4) wherein an amino acid L was added at position -3 of the sequence SEQ ID N° 2, either immediately upstream of residue N° -2 if present, or if absent, immediately upstream of residue N° -1, or if absent, immediately upstream of residue N° 1
- none/Q (5) wherein an amino acid Q was added at position -2 of the sequence SEQ ID N° 2, either immediately upstream of residue N° -1 if present, or if absent, immediately upstream of residue N° 1
- none/E (6) wherein an amino acid E was added at position -1 of the sequence SEQ ID N° 2, either immediately upstream of residue N° 1
- L/V, wherein the amino acid L at position 5 of the sequence SEQ ID N° 2 is replaced with amino acid V
- A/T, wherein the amino acid A at position 17 of the sequence SEQ ID N° 2 is replaced with amino acid T
- A/T, wherein the amino acid A at position 113 of the sequence SEQ ID N° 2 is replaced with amino acid T
- V/L, wherein the amino acid V at position 114 of the sequence SEQ ID N° 2 is replaced with amino acid L.

5. The IgG according to any one of the preceding claims, **characterized in that** the light chain constant region has an amino acid sequence represented by the sequence SEQ ID N° 3, and the heavy chain constant region has an amino acid sequence represented by the sequence SEQ ID N° 4.

6. The IgG according to any one of the preceding claims, **characterized in that** the constant region of each of its heavy chains is of a γ1 type, and preferably, **in that**:
- the constant region of each of its heavy chains comprises the amino acid sequence encoded by the sequence SEQ ID N° 9, and **in that** the constant region of each of its light chains comprises the amino acid sequence encoded by the sequence SEQ ID N° 10, or
- each of its light chains comprises the amino acid sequence encoded by the sequence SEQ ID N° 11, and **in that** each of its heavy chains comprises the amino acid sequence encoded by the sequence SEQ ID N° 12.

7. A nucleic acid encoding an antibody according to any one of claims 1 to 6.

8. A vector comprising a nucleic acid according to claim 7.

9. A host cell comprising a vector according to claim 8.

10. A composition, preferably a pharmaceutical composition, comprising at least one human IgG according to any one of claims 1 to 6.

11. The use of a human IgG according to any one of claims 1 to 6 for the preparation of a drug for treating or preventing an infection with *Bacillus anthracis.*

12. A kit for detecting an anthrax toxin comprising PA, said kit comprising:
- a container comprising at least one labeled IgG according to any one of claims 1 to 6, and
- a container comprising means for detecting this labeled IgG.

13. A method for detecting in vitro an anthrax toxin comprising PA in a biological sample, comprising:
- contacting the sample with at least one IgG according to any one of claims 1 to 6, and
- detecting the binding of said IgG as an indicator of the presence of said anthrax toxin.

14. An immunoconjugate comprising an IgG according to any one of claims 1 to 6 bound to a therapeutic agent.
